# FASCICULE DE BREVET EUROPEEN

(11) **EP 1 615 925 B1**
(45) Date de publication et mention de la délivrance du brevet: **27.05.2009**
(21) Numéro de dépôt: 04742386.8
(22) Date de dépôt: 29.03.2004
(51) Int. Cl.: C07D 471/04, A61K 31/4188, A61K 31/4353

(54) **DERIVES D'IMIDAZO-PYRIDINE COMME AGONISTES DU RECEPTEUR DE LA MELANCORTINE**
IMIDAZOPYRIDINDERIVATE ALS MELANOCORTINREZEPTORAGONISTEN
IMIDAZOPYRIDINE DERIVATIVES AS MELANOCORTIN RECEPTOR AGONISTS

(30) Priorité: 31.03.2003 FR 0303924
(43) Date de publication de la demande: 18.01.2006
(73) Titulaire: IPSEN PHARMA, 92100 Boulogne-Billancourt (FR)
(72) Inventeur: POITOUT, Lydie, F-92160 Antony (FR); BRAULT, Valérie, F-78730 Saint-Arnoult-en-Yvelines (FR); SACKUR, Carole, F-75004 Paris (FR); ROUBERT, Pierre, F-75014 Paris (FR); PLAS, Pascale, F-92320 Châtillon (FR)
(74) Mandataire: Bourgouin, André
(86) Numéro de dépôt international: PCT/FR2004/000785
(87) Numéro de publication internationale: WO 2004/089951

(56) Documents cités:
- WO-A-01/91752
- WO-A-02/060879
- US-B1- 6 350 760

## Description

La présente demande a pour objet de nouveaux dérivés d'imidazo-pyridine. Ces produits ont une bonne affinité pour certains sous-types de récepteurs des mélanocortines, en particulier des récepteurs MC4. Ils sont particulièrement intéressants pour traiter les états pathologiques et les maladies dans lesquels un ou plusieurs récepteurs des mélanocortines sont impliqués. L'invention concerne également des compositions pharmaceutiques contenant lesdits produits et leur utilisation pour la préparation d'un médicament.

Les mélanocortines représentent un groupe de peptides qui dérivent d'un même précurseur, la proopiomélanocortine (POMC), et qui sont structurellement proches : l'hormone adrénocorticotrope (ACTH), l'hormone stimulante des mélanocytes α (α-MSH), la β-MSH et la γ-MSH (Eipper B.A. et Mains R.E., Endocr. Rev. 1980, 1, 1-27). Les mélanocortines exercent de nombreuses fonctions physiologiques. Elles stimulent la synthèse des stéroïdes par la cortico-surrénale et la synthèse d'eumélanine par les mélanocytes. Elles régulent la prise de nourriture, le métabolisme énergétique, la fonction sexuelle, la régénération neuronale, la pression sanguine et la fréquence cardiaque, ainsi que la perception de la douleur, l'apprentissage, l'attention et la mémoire. Les mélanocortines possèdent également des propriétés anti-inflammatoires et anti-pyrétiques et contrôlent la sécrétion de plusieurs glandes endocrines ou exocrines telles les glandes sébacées, lacrymales, mammaires, la prostate et le pancréas (Wikberg J.E. et al. Pharmacol. Res. 2000,42,393-420; Abdel-Malek Z.A., Cell. Mol. Life. Sci. 2001, 58, 434-441).

Les effets des mélanocortines sont médiés par une famille de récepteurs membranaires spécifiques à sept domaines transmembranaires et couplés aux protéines G. Cinq sous-types de récepteurs, nommés MCl à MC5, ont été clonés et caractérisés à ce jour. Ces récepteurs diffèrent par leur distribution tissulaire et par l'affinité des différentes mélanocortines, les récepteurs MC2 ne reconnaissant que l'ACTH. La stimulation des récepteurs des mélanocortines active l'adénylate cyclase avec production d'AMP cyclique. Si les rôles fonctionnels spécifiques de chacun des récepteurs ne sont pas totalement élucidés, le traitement de désordres pathologiques ou de maladies peut être associé à une affinité pour certains sous-types de récepteurs. Ainsi l'activation des récepteurs MC1 a été associée au traitement des inflammations, alors que leur blocage a été associé au traitement de cancers cutanés. Le traitement des troubles de la nutrition a été associé aux récepteurs MC3 et MC4, le traitement de l'obésité par les agonistes et le traitement de la cachexie et de l'anorexie par les antagonistes. D'autres indications associées à l'activation des récepteurs MC3 et MC4 sont les troubles de l'activité sexuelle, les douleurs neuropathiques, l'anxiété, la dépression et les toxicomanies. L'activation des récepteurs MC5 a été associée au traitement de l'acné et des dermatoses.

Des efforts de recherche sont donc dédiés à la découverte de composés non peptidiques de faibles poids moléculaires, bio-disponibles par voie orale, puissants agonistes ou antagonistes des récepteurs de mélanocortine.

Les déposants ont trouvé que les nouveaux composés de formule générale (I) décrits ci-après possèdent une bonne affinité pour les récepteurs des mélanocortines. Ils agissent préférentiellement sur les récepteurs MC4. Lesdits composés, agonistes ou antagonistes des récepteurs des mélanocortines, peuvent être utilisés pour traiter les états pathologiques ou les maladies métaboliques, du système nerveux ou dermatologiques, dans lesquels un ou plusieurs récepteurs des mélanocortines sont impliqués tels que les exemples suivants : les états inflammatoires, les troubles de l'homéostasie énergétique, de la prise de nourriture, les désordres pondéraux (l'obésité, la cachexie, l'anorexie), les désordres de l'activité sexuelle (les troubles de l'érection), la douleur et plus particulièrement la douleur neuropathique. On peut également citer les troubles mentaux (l'anxiété, la dépression), les toxicomanies, les maladies de la peau (l'acné, les dermatoses, les cancers cutanés, les mélanomes). Ces composés peuvent également être utilisés pour stimuler la régénération nerveuse.

L'invention a donc pour objet un composé de formule générale (I) 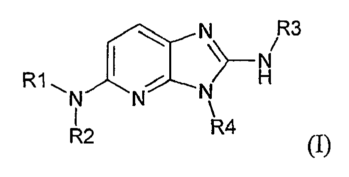 sous forme racémique, d'énantiomère ou toutes combinaisons de ces formes et dans laquelle :
R₁ et R₂ représentent, indépendamment, l'atome d'hydrogène ; un radical (C₁-C₈)alkyle éventuellement substitué par hydroxy ; (C₂-C₆)alkényle ; un bicycloalkyle ; ou un radical de formule -(CH₂)ₙ-X₁ ou -X-(CH₂)_{n'}-X'₁ ;
X représente -C(O)- ou -C(S)-NH- ;
X₁ représente un radical (C₁-C₆)alkoxy, (C₃-C₇)cycloalkyle, adamantyle, hétérocycloalkyle, aryle ou hétéroaryle,
   les radicaux (C₃-C₇)cycoalkyle, hétérocycloalkyle, aryle et hétéroaryle étant éventuellement substitués par un ou plusieurs substituants identiques ou différents choisis parmi : -(CH₂)ₙ₁-V₁-Y₁, halo , nitro et cyano ;
   V₁ représente -O-, -S- ou une liaison covalente ;
   Y₁ représente un radical (C₁-C₆)alkyle éventuellement substitué par un ou plusieurs radicaux halo identiques ou différents, ou aryle ;
   n et n' représentent un entier de 0 à 6 et n₁ un entier de 0 à 2 (étant entendu que lorsque n est égal à 0, alors X₁ ne représente pas le radical alkoxy) ;
X'₁ représente l'atome d'hydrogène, un radical (C₁-C₆)alkyle éventuellement substitué par un ou plusieurs radicaux halo identiques ou différents, (C₃-C₇)cycloalkyle ; ou aryle éventuellement substitué par un ou plusieurs substituants identiques ou différents choisi parmi : halo, nitro, cyano, (C₁-C₆)alkyl-carbonyle, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, -(CH₂)_{n"}-A, -C(O)-NV₁'Y₁', et hétérocycloalkyle ; ou bien R₁ et R₂ forment ensemble un radical de formule : 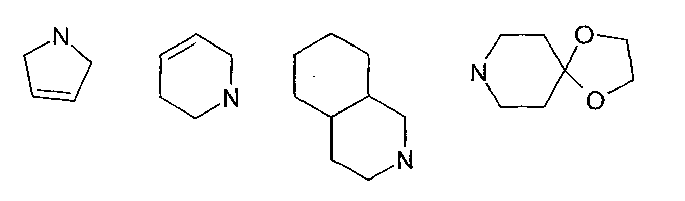 V₁' et Y₁' représentent, indépendamment, l'atome d'hydrogène ou un (C₁-C₆)alkyle ;
A représente un radical aryle éventuellement substitué par un ou plusieurs substituants identiques ou différents choisi parmi : halo, nitro, cyano, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
n" représente un entier de 0 à 2 ;
R₃ représente -Z₃, -C(R_{Z3})(R'_{Z3})-Z₃, -C(R_{Z3})(R'_{Z3})-(CH₂)ₚ-Z₃ ou -C(O)-Z'₃ ;
R_{Z3} et R'_{Z3} représentent, indépendamment, l'atome d'hydrogène ou un radical (C₁-C₆)alkyle ;
Z₃ représente Z₃ₐ, Z_{3b}, Z_{3c}, Z_{3d}, ou Z₃ₑ ;
Z₃ₐ représente un radical (C₁-C₆)alkyle ou (C₂-C₆)alkényle ;
Z_{3b} représente un radical (C₁-C₆)alkoxy, (C₁-C₆)alkylthio, (C₁-C₆)alkylamino ou di((C₁-C₆)alkyl)amino ;
Z_{3c} représente un radical aryle ou hétéroaryle ;
les radicaux aryle et hétéroaryle étant éventuellement substitués par un ou plusieurs substituants identiques ou différents choisis parmi : halo, cyano, nitro, azido, oxy ou -(CH₂)_{p'}-V₃-Y₃ ;
V₃ représente -O-, -S-, -C(O)-, -C(O)-O-, -O(CO)-, -SO₂-, -SO₂NH-, -NR'₃-SO₂-, -NR'₃-, -NR'₃-C(O)-, -C(O)-NR'₃-, -NH-C(O)-NR'₃- ou une liaison covalente ;
Y₃ représente l'atome d'hydrogène ou un radical (C₁-C₆)alkyle éventuellement substitué par un ou plusieurs radicaux halo identiques ou différents ; un radical aryle éventuellement substitué par un ou plusieurs substituants identiques ou différents choisis parmi : halo, nitro, (C₁-C₆)alkyle et (C₁-C₆)alkoxy ; ou un radical aryl-(C₁-C₆)alkyle éventuellement substitué par un ou plusieurs substituants identiques ou différents choisis parmi : halo, nitro, (C₁-C₆)alkyle et (C₁-C₆)alkoxy ;
Z_{3d} représente un radical (C₁-C₆)alkoxy-carbonyle, amino-carbonyle, (C₁-C₆)alkylamino-carbonyle, di((C₁-C₆)alkyl)amino-carbonyle ;
Z₃ₑ représente un radical (C₁-C₆)alkyl-C(O)-NH-, (C₃-C₇)cycloalkyle, hétérocycloalkyle ou un radical de formule 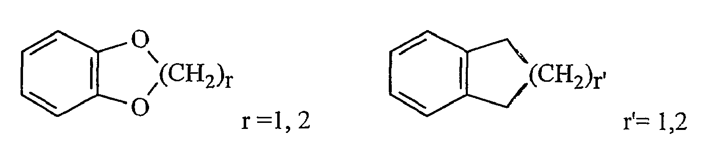 les radicaux (C₃-C₇) cycloalkyle et hétérocycloalkyle étant éventuellement substitués par un ou plusieurs radicaux oxy ou (C₁-C₆)alkyle identiques ou différents,
Z'₃ représente un radical aryle éventuellement substitué par un ou plusieurs substituants identiques ou différents choisis parmi : halo, nitro et -(CH₂)ₚ"-V'₃-Y'₃ ;
V'₃ représente -O-, -C(O)-, -C(O)-O-, -C(O)-NR'₃-, -NR'₃-C(O)-, -NH-C(O)-NR'₃ ou une liaison covalente ;
Y'₃ représente l'atome d'hydrogène ou un radical (C₁-C₆)alkyle éventuellement substitué par un ou plusieurs radicaux halo identiques ou différents ;
R'₃ représente l'atome d'hydrogène, un radical (C₁-C₆)alkyle ou (C₁-C₆)alkoxy ;
p, p' et p" représentent, indépendamment, un entier de 0 à 6 ;
R₄ représente un radical de formule-(CH₂)ₛ-R'₄
R'₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle ; aryle éventuellement substitué par un ou plusieurs radicaux identiques ou différents choisis parmi : -(CH₂)_{s"}-V₄-Y₄, , halo et nitro ;
   V₄ représente -O-, -S-, -NH-C(O)-, -NV₄'- ou une liaison covalente ;
   Y₄ représente un atome d'hydrogène ou un radical (C₁-C₆)alkyle éventuellement substitué par un ou plusieurs radicaux halo identiques ou différents ;
   V₄' représente un atome d'hydrogène ou un (C₁-C₆)alkyle ;
   s" représente un entier de 0 à 4 ;
   ou bien Z₄ représente un radical de formule 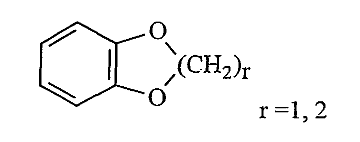 s et s' représentent, indépendamment, un entier de 0 à 6 ; ou un sel pharmaceutiquement acceptable de ce dernier.

Dans les définitions indiquées ci-dessus, l'expression halo représente le radical fluoro, chloro, bromo ou iodo, de préférence chloro, fluoro ou bromo. L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, neopentyle, 2,2-diméthyl-propyle, hexyle, isohexyle ou 1,2,2-triméthyl-propyle. Le terme (C₁-C₈)alkyle désigne un radical alkyle ayant de 1 à 8 atomes de carbone, linéaire ou ramifié, tels les radicaux contenant de 1 à 6 atomes de carbone tels que définis ci-dessus mais également heptyle, octyle, 1,1,2,2-tétraméthylpropyle, 1,1,3,3-tétraméthyl-butyle. Par l'expression alkyle substitué par hydroxy, il faut comprendre toute chaîne alkyle linéaire ou ramifiée, contenant un radical hydroxy positionnée le long de la chaîne ; ainsi pour une chaîne contenant 3 atomes de carbone et un radical hydroxy, on peut donner comme exemple HO-(CH₂)₃-, CH₃-CH(OH)-CH₂- et CH₃-CH₂-CH(OH)-.

Par alkényle, lorsqu'il n'est pas donné plus de précision, on entend un radical alkyle linéaire ou ramifié comptant de 1 à 6 atomes de carbone et présentant au moins une insaturation (double liaison), comme par exemple vinyle, allyle, propényle, butènyle ou pentènyle.

Le terme alkoxy désigne les radicaux dans lesquels le radical alkyle est tel que défini ci-dessus comme par exemple les radicaux méthoxy, éthoxy, propyloxy ou isopropyloxy mais également butoxy linéaire, secondaire ou tertiaire, pentyloxy. Le terme alkoxy-carbonyle désigne de préférence les radicaux dans lesquels le radical alkoxy est tel que défini ci-dessus comme par exemple méthoxycarbonyle, éthoxycarbonyle.

Le terme (C₃-C₇)cycloalkyle désigne un système monocyclique carboné saturé comprenant de 3 à 7 atomes de carbone, et de préférence les cycles cyclopropyle, cyclobutyle, cyclopentyle, cyclohexyle ou cycloheptyle. L'expression hétérocycloalkyle désigne un système saturé monocyclique ou bicyclique condensé contenant de. 2 à 9 atomes de carbone et au moins un hétéroatome. Ce radical peut contenir plusieurs hétéroatomes identiques ou différents. De préférence, les hétéroatomes sont choisis parmi l'oxygène, le soufre ou l'azote. Comme exemple d'hétérocycloalkyle, on peut citer les cycles contenant au moins un atome d'azote tels que pyrrolidine, imidazolidine, pyrrazolidine, isothiazolidine, thiazolidine, isoxazolidine, oxazolidine, pipéridine, pipérazine, azépane (azacycloheptane), azacyclooctane, diazépane, morpholine, décahydroisoquinoline (ou décahydroquinoline) mais également les cycles ne contenant aucun atome d'azote tels que tétrahydrofurane ou tétrahydrothiophène. Comme illustration de cycloalkyle ou hétérocycloalkyle substitué par oxy, on peut citer par exemple pyrrolidinone et imidazolidinone.

Le terme bicycloalkyle désigne un système bicyclique hydrocarboné saturé non condensé contenant de 5 à 9 atomes de carbone, tel que bicyclo-heptane comme par exemple bicylo[2,2,1]heptane, ou bicyclo-octane comme par exemple bicyclo[2,2,2]octane ou bicyclo[3,2,1]octane. Le terme hétérobicycloalkyle désigne un système bicyclique hydrocarboné saturé non condensé contenant de 5 à 8 atomes de carbone et au moins un hétéroatome choisi parmi l'azote, l'oxygène et le soufre. Comme exemple d'hétérobicycloalkyle, on peut citer les aza-bicycloheptane et azabicyclooctane tels que 7-aza-bicyclo[2,2,1]heptane, 2-aza-bicyclo[2,2,2]octane ou 6-aza-bicyclo[3,2,1]octane.

L'expression aryle représente un radical aromatique, constitué d'un cycle ou de cycles condensés, comme par exemple le radical phényle, naphtyle, fluorényle ou anthryle. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux contenant au moins un atome d'azote tels que pyrrolyle, imidazolyle, pyrazolyle, isothiazolyle, thiazolyle, isoxazolyle, oxazolyle, triazolyle, thiadiazolyle, pyridyle, pyrazinyle, pyrimidyle, quinolyle, isoquinolyle, quinoxalinyle, indolyle, dihydroindolyle, benzoxadiazoyle, carbazolyle, phénoxazinyle mais également les radicaux ne contenant pas d'atome d'azote tels que thiényle, benzothiényle, furyle, benzofuryle, dibenzofuryle, dihydrobenzofuryle, dibenzothiènyle, thioxanthènyle, ou pyranyle. Le terme aralkyle (arylalkyle) désigne de préférence les radicaux dans lesquels les radicaux aryle et alkyle sont tels que définis ci-dessus comme par exemple benzyle ou phenéthyle. Comme illustration de radical aryle ou hétéroaryle substitué par oxy, on peut citer par exemple fluorènone, l'acridone, xanthènone, benzothiényle-dione, anthraquinone, thioxanthène, benzocoumarine.

Dans la présente demande également, le radical (CH₂)ᵢ (i entier pouvant représenter n, n', n", n₁, p, p', p", s, s' et s" tels que définis ci-dessus), représente une chaîne hydrocarbonée, linéaire ou ramifiée, de i atomes de carbone. Ainsi le radical -(CH₂)₃- peut représenter -CH₂-CH₂-CH₂- mais également -CH(CH₃)-CH₂-, -CH₂-CH(CH₃)- ou -C(CH₃)₂-.

Selon la présente demande également, lorsqu'un radical a pour formule -B-D-E avec D représentant par exemple -C(O)-NH-, cela signifie que l'atome de carbone de -C(O)-NH- est lié à B et l'atome d'azote à E.

De préférence, l'invention concerne un composé de formule I telle que définie ci-dessus, **caractérisé en ce que**
R₁ et R₂ représentent, indépendamment, l'atome d'hydrogène, un radical (C₁-C₈)alkyle, un bicycloalkyle ou un radical de formule -(CH₂)ₙ-X₁ ou -X-(CH₂)ₙ'-X'₁ ;
X représente -C(O)- ou -C(S)-NH- ;
X₁ représente un radical (C₁-C₆)alkoxy, (C₃-C₇)cycloalkyle éventuellement substitué par un (C₁-C₆)alkyle, ou hétéroaryle ;
X'₁ représente l'atome d'hydrogène, un radical (C₁-C₆)alkyle éventuellement substitué par un ou plusieurs radicaux halo identiques ou différents, (C₃-C₇)cycloalkyle ou aryle éventuellement substitué par un (C₁-C₆)alkyl-carbonyle ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : (C₁-C₆)alkyle, (C₁-C₆)alkoxy-carbonyle et -(CH2)_{n"}-A ;
A représente un radical aryle éventuellement substitué par un ou plusieurs substituants identiques ou différents choisi parmi : halo et (C₁-C₆)alkyle ;
n" représente un entier de 0 à 1 ;
R₄ représente un radical de formule-(CH₂)ₛ-R'₄
R'₄ représente un hétérocycloalkyle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle ou aryle éventuellement substitué par un ou plusieurs radicaux identiques ou différents choisis parmi : -(CH₂)_{s"}-V₄-Y₄ ;
   V₄ représente -O- ;
   Y₄ représente un radical (C₁-C₆)alkyle éventuellement substitué par un ou plusieurs radicaux halo identiques ou différents ;
   s" représente un entier de 0 à 4 ;
s et s' représentent, indépendamment, un entier de 1 à 4 ; ou un sel ; ou un sel pharmaceutiquement acceptable de ce dernier,
et plus particulièrement le composé I comporte au moins une des caractéristiques suivantes :
- le radical cycloalkyle est choisi parmi cyclopropyle, cyclobutyle et cyclohexyle ;
- le radical bicycloalkyle est le bicylo[2,2,1]heptane ;
- l'hétérobicycloalkyle est le 7-aza-bicyclo[2,2,1]heptane ;
- le radical aryle est le radical phényle ;
- le radical hétéroaryle est le radical furyle ;
- l'hétérocycloalkyle est choisi parmi pipéridine, morpholine et pipérazine ; ou un sel pharmaceutiquement acceptable de ce dernier.

De manière très préférentielle également, l'invention concerne un composé de formule I telle que définie ci-dessus, **caractérisé en ce que**
R₁ et R₂ représentent, indépendamment, l'atome d'hydrogène, un radical (C₁-C₈)alkyle ou un radical de formule -(CH₂)ₙ-X₁ ou -X-(CH₂)_{n'}-X'₁ ;
   X représente -C(O)- ;
   X₁ représente un radical (C₃-C₇)cycloalkyle ;
   X'₁ représente l'atome d'hydrogène ou un radical (C₃-C₇)cycloalkyle ;
   n représente 0 ou 1 ; n'représente un entier de 0 à 5 ;
   ou bien R₁ et R₂ forment ensemble, avec l'atome d'azote auquel ils sont rattachés, un hétérocycloalkyle éventuellement substitué par un ou plusieurs substituants (C₁-C₆)alkyle identiques ou différents ;
et plus particulièrement le radical (C₃-C₇)cycloalkyle que représentent X₁ et X'₁ est choisi parmi cyclopropyle, cyclobutyle et cyclohexyle ; et l'hétérocycloalkyle que forment ensemble R₁ et R₂, est le cycle pipéridine ; ou un sel pharmaceutiquement acceptable de ce dernier.

De manière très préférentielle également, l'invention concerne un composé de formule I telle que définie ci-dessus, **caractérisé en ce que**
R₄ représente un radical de formule-(CH₂)ₛ-R'₄
R'₄ représente un hétérocycloalkyle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène ou (C₁-C₈)alkyle ;
s et s'représentent, indépendamment, un entier de 2 à 4 ;
et plus particulièrement l'hétérocycloalkyle que représente R'₄ est choisi parmi : pipéridine et morpholine ; ou un sel pharmaceutiquement acceptable de ce dernier.

De préférence également, l'invention concerne un composé de formule I telle que définie ci-dessus, **caractérisé en ce que** R₃ représente -C(O)-Z'₃
Z'₃ représente un radical aryle éventuellement substitué par un ou plusieurs substituants identiques ou différents choisis parmi halo et -(CH₂)_{p"}-V'₃-Y'₃ ;
V'₃ représente -O- ou une liaison covalente ;
Y'₃ représente l'atome d'hydrogène ou un radical (C₁-C₆)alkyle éventuellement substitué par un ou plusieurs radicaux halo identiques ou différents ;
p" représente un entier de 0 à 2 ; ou un sel pharmaceutiquement acceptable de ce dernier.

De préférence également, l'invention concerne un composé de formule I telle que définie ci-dessus, **caractérisé en ce que** R₃ représente -Z₃, -C(R_{Z3})(R'_{Z3})-Z₃ ou -C(R_{Z3})(R'_{Z3})-(CH₂)ₚ-Z₃ ; ou un sel pharmaceutiquement acceptable de ce dernier.

De manière très préférentielle également, l'invention concerne un composé de formule I telle que définie ci-dessus, **caractérisé en ce que** R₃ représente -Z₃ et Z₃ représente Z_{3b}, Z_{3c} ou Z₃ₑ ; et de préférence Z₃ représente Z_{3c} et Z_{3c} représente un radical aryle,
et plus particulièrement Z_{3c} représente un radical phényle étant éventuellement substitué par un ou plusieurs substituants identiques ou différents choisis parmi : halo, nitro ou -(CH₂)_{p'}-V₃-Y₃ ;
V₃ représente -O-, -S-, -C(O)-, -C(O)-O-, -SO₂NH-, -NR'₃-C(O)-, -C(O)-NR'₃- ou une liaison covalente ;
R'₃ représente l'atome d'hydrogène ;
Y₃ représente l'atome d'hydrogène ou un radical (C₁-C₆)alkyle éventuellement substitué par un ou plusieurs radicaux halo identiques ou différents ;
et plus particulièrement également Z_{3c} représente un radical phényle substitué par un ou plusieurs substituants identiques ou différents de formule -(CH₂)ₚ'-V₃-Y₃ ;
V₃ représente -C(O)-, -C(O)-O- ou -C(O)-NR'₃- ;
R'₃ représente l'atome d'hydrogène ;
Y₃ représente l'atome d'hydrogène ou un radical (C₁-C₆)alkyle ; ou un sel pharmaceutiquement acceptable de ce dernier.

De manière très préférentielle également, l'invention concerne des composés de formule I telle que définie ci-dessus, **caractérisés en ce que** R₃ représente -C(R_{Z3})(R'_{Z3})-Z₃ et Z₃ représente Z_{3d} ou Z₃ₑ ; ou un sel pharmaceutiquement acceptable de ce dernier.

De manière très préférentielle également, l'invention concerne des composés de formule I telle que définie ci-dessus, **caractérisés en ce que** R₃ représente -C(R_{Z3})(R'_{Z3})-(CH₂)ₚ-Z₃ et Z₃ représente Z_{3c}, Z_{3d} ou Z₃ₑ,
et plus particulièrement Z₃ représente Z_{3d} ou Z₃ₑ
Z_{3d} représente un radical (C₁-C₆)alkoxy-carbonyle ou amino-carbonyle ;
Z₃ₑ représente un radical (C₁-C₆)alkyl-C(O)-NH-, hétérocycloalkyle étant éventuellement substitué par un radical oxy, ou un radical de formule 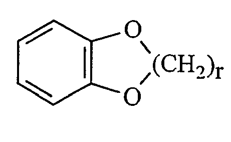 r =1, 2 ;
ou un sel pharmaceutiquement acceptable de ce dernier.

Dans la présente demande, le symbole -> * correspond au point de rattachement du radical. Lorsque le site de rattachement n'est pas précisé sur le radical, cela signifie que le rattachement s'effectue sur un des sites disponibles de ce radical pour un tel rattachement.

Suivant les définitions des groupes variables R₁, R₂, R₃ et R₄, les composés selon l'invention peuvent être préparés en phase liquide selon les différentes procédures A à E décrites ci-dessous.

### A. Préparation selon le schéma réactionnel A :

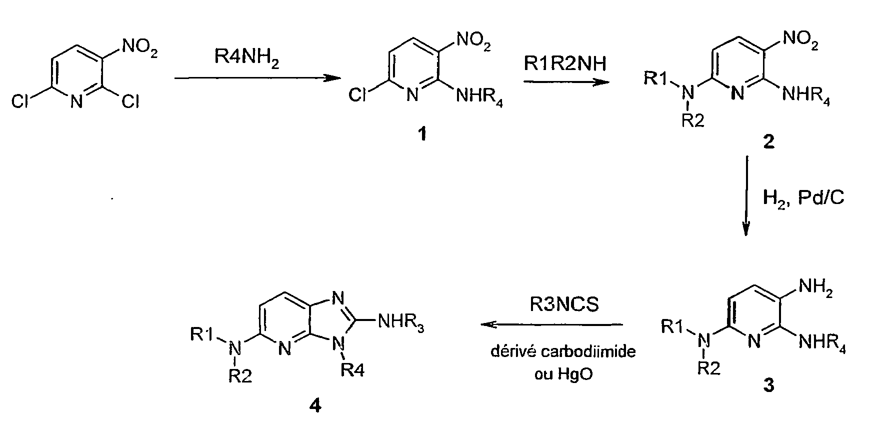

Comme décrit dans le schéma A, le 2,6-dichloro-3-nitropyridine peut être traité par une amine primaire en présence d'une base organique telle qu'une amine tertiaire ou une base inorganique tel que le carbonate de potassium ou césium, dans un solvant aprotique apolaire tel que le toluène à une température voisine de 20° C pendant 3-18 heures pour donner le composé (1). Le dérivé chloré (1) peut réagir avec une amine primaire ou secondaire, en présence d'une base organique telle qu'une amine tertiaire ou une base inorganique tel que le carbonate de potassium ou césium, dans un solvant aprotique polaire tel que l'acétonitrile, le diméthylformamide ou l'HMPA à une température de 20-70° C pendant 2-18 heures pour conduire au composé (2). La fonction nitro du composé (2) est réduite par hydrogénation catalytique en présence de palladium sur charbon 10 % dans un solvant inerte tel que le méthanol, éthanol, acétate d'éthyle ou un mélange de ces solvants, à une température de 18-25° C, pendant 2 à 8 heures pour conduire à la dianiline (3). Le dérivé (3) est ensuite traité par un isothiocyanate en présence d'un agent de couplage supporté sur une résine ou non tel que le diisopropylcarbodiimide ou dicyclohexylcarbodiimide ou la résine *N*-cyclohexylcarbodiimide *N*-méthyl polystyrène dans un solvant inerte tel que le tétrahydrofuranne, chlorure de méthylène, ou chloroforme à une température de 20-70° C pendant 2 à 72 heures pour conduire au dérivé (4). Alternativement, le dérivé (3) peut être traité par un isothiocyanate dans un solvant inerte tel que le tétrahydrofuranne, chlorure de méthylène, chloroforme ou éthanol à une température de 20-80° C pendant 1-16 heures puis la thiourée résultante peut être traitée par de l'oxyde de mercure (II) jaune en présence d'une quantité catalytique de soufre dans un solvant polaire tel que le méthanol ou l'éthanol pendant 2 à 24 heures à une température de 20-80° C pour conduire à (4).

### Exemple A1 : chlorhydrate de 4-{[3-(3-aminopropyl)-5-(diisobutylamino)-3H-imidazo[4,5-b] pyridin-2-yl]amino}-N-methylbenzamide

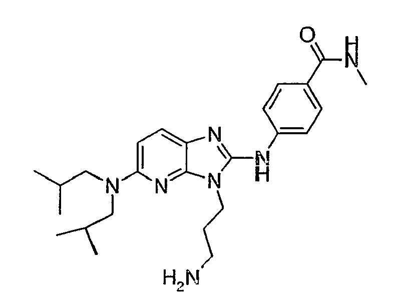

### Etape 1 : tert-butyl 3-[(6-chloro-3-nitropyridin-2-yl)amino]propylcarbamate

A la 2,6-dichloro-3-nitropyridine (8 g, 1 éq) en solution dans le toluène (150 ml) sont successivement additionnés le carbonate de potassium (5,4 g, 1,2 éq) et le *tert*-butyl-*N*(2-aminopropyl)carbamate (6,8 g, 1 éq). Après 6 heures d'agitation à une température voisine de 20° C, le mélange est concentré sous pression réduite à 40° C puis additionné d'eau (80 ml) et de dichlorométhane (200 ml). Après décantation et extractions, les phases organiques combinées sont lavées avec de la saumure, séchées sur Na₂SO₄ puis concentrées sous pression réduite à 40° C. La purification du solide obtenu, par chromatographie éclair sur gel de silice (éluant : heptane/acétate d'éthyle 7 :3), donne le composé attendu sous forme d'un solide jaune (11,4 g ; 92 % rendement).

SM/CL : MM calculée = 330,7 ; m/z = 331,1 (MH+)

RMN (¹H, 400 MHz, DMSO-*d*₆) : *δ* 1,36 (s, 9H), 1,68 (m, 2H), 2,99 (dd, 2H), 3,51 (dd, 2H), 6,76 (d, 1H), 6,86 (t, 1H), 8,41 (d, 1H), 8,75 (t, 1H).

### Etape 2 : tert-butyl 3-{[6-(diisobutylamino)-3-nitropyridin-2-yl]amino}propyl carbamate

A une solution de *tert*-butyl 3-[(6-chloro-3-nitropyridin-2-yl)amino]propylcarbamate (2 g, 1 éq) dans l'acétonitrile (100 ml) sont successivement additionnés le carbonate de potassium (1,31 g, 1,5 éq) et la diisobutylamine (981 mg, 1,2 éq). Le mélange est chauffé a reflux pendant 5 heures puis refroidi à température ambiante et concentré sous pression réduite à 40° C. Le résidu est repris dans le dichlorométhane (200 ml) et l'eau (90 ml). Après décantation et extractions, les phases organiques combinées sont lavées avec de la saumure, séchées sur Na₂SO₄ puis concentrées sous pression réduite à 40° C. La purification du résidu obtenu, par chromatographie éclair sur gel de silice (éluant : heptane/acétate d'éthyle 7 :3 à 1 :1), donne le composé attendu sous forme d'une huile jaune (2,46 g; 95 % rendement).

SM/CL : MM calculée = 437,6 ; m/z = 438,3 (MH+)

RMN (¹H, 400 MHz, DMSO-*d*₆) : δ 0,86 (m, 12H), 1,36 (s, 9H), 1,68 (m, 2H), 1,99 (m, 1H), 2,15 (m, 1H), 2,98 (dd, 2H), 3,35 (m, 2H), 3,48 (m, 4H), 6,20 (d, 1H), 6,85 (t, 1H), 8,01 (d, 1H), 8,85 (t, 1H).

### Etape 3 : tert-butyl 3-[5-(diisobutylamino)-2-({4-[(méthylamino)carbonyl]phényl} amino)-3H-imidazo[4,5-b]pyridin-3-yl]propylcarbamate

Dans un tube à hémolyse placé dans un autoclave sont additionnés le *tert*-butyl 3-{[6-(diisobutylamino)-3-nitropyridin-2-yl]amino}propylcarbamate (63 mg) en solution dans un mélange d'acétate d'éthyle/ éthanol 3 :1 (1,5 ml), et le palladium sur charbon 10 % (7 mg). Après 3 heures d'agitation sous atmosphère d'hydrogène (3 bars) à une température d'environ 20° C, le mélange est filtré sur célite dans un tube à hémolyse contenant une solution de 4-isothiocyanato-*N*-méthylbenzamide (43 mg, 1,2 éq) dans le tétrahydrofuranne (1 ml). Au filtrat ainsi obtenu est additionnée la résine *N*-cyclohexylcarbodiimide-*N*-méthyl-polystyrène (acquise auprès de Novabiochem ; charge 1,9 mmol/g ; 237 mg, 3 éq). Le mélange est chauffé à reflux pendant 18 heures, refroidi à température ambiante puis filtré sur fritté et le filtrat est concentré sous pression réduite à 40° C. La purification du résidu par chromatographie éclair sur gel de silice (éluant : heptane/acétate d'éthyle 1 :1 à acétate d'éthyle 100 %) donne le composé attendu (53 mg ; 65 % rendement).

SM/CL : MM calculée = 551,7 ; m/z = 552,3 (MH+)

RMN (¹H, 400 MHz, DMSO-*d*₆): *δ* 0,88 (d, 12H), 1,36 (s, 9H), 1,83 (m, 2H), 2,09 (m, 2H), 2,76 (d, 3H), 2,98 (m, 2H), 3,35 (m, 4H), 4,15 (t, 2H), 6,34 (d, 1H), 6,80 (t, 1H), 7,51 (d, 1H), 7,78 (AB, 2H), 7,85 (AB, 2H), 8,20 (m, 1H), 8,97 (s, 1H).

### Etape 4 : chlorhydrate de 4-{[3-(3-aminopropyl)-5-(diisobutylamino)-3H-imidazo[4,5-b] pyridin-2-yl]amino}-N-méthylbenzamide

A une solution de *tert*-butyl 3-[5-(diisobutylamino)-2-({4-[(méthylamino)carbonyl] phényl}amino)-3*H*-imidazo[4,5-*b*]pyridin-3-yl] propylcarbamate (51 mg) dans l'acétate d'éthyle (0,5 ml) est additionnée une solution d'acide chlorhydrique dans l'ether (1N, 2 ml). Après 2 heures d'agitation à une température voisine de 20° C, le mélange est filtré et le solide obtenu est lavé à l'éther éthylique et séché (50 mg ; 95 % rendement).

SM/CL : MM calculée = 451,6 ; m/z = 452,3 (MH+)

RMN (¹H, 400 MHz, DMSO-*d*₆) : *δ*0,88 (d, 12H), 2,10-2,19 (m, 4H), 2,79 (d, 3H), 2,95 (m, 2H), 3,35 (m, 4H), 4,40 (t, 2H), 6,55 (d, 1H), 7,53 (d, 1H), 7,64 (m, 2H), 7,94 (m, 5H), 8,45 (m, 2H).

### Exemple A2 : chlorhydrate de 1-(4-{[5-(dibutylamino)-3-(3-pipéridin-1-ylpropyl)-3H-imidazo[4,5-b]pyridin-2-yl]amino}phényl)éthanone

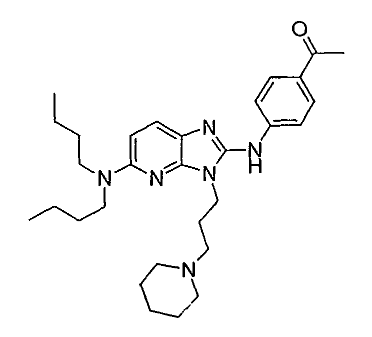

### Etape 1 : 6-chloro-3-nitro-N-(3-pipéridin-1-ylpropyl)pyridin-2-amine

A la 2,6-dichloro-3-nitropyridine (500 mg, 1 éq) en solution dans le toluène (10 ml) sont successivement additionnés le carbonate de potassium (540 mg, 1,5 éq) et la 3-pipéridino-propylamine (420 mg, 1 éq). Après 2 heures d'agitation à une température voisine de 20° C, le mélange est concentré sous pression réduite à 40° C puis additionné d'eau (20 ml) et de dichlorométhane (70 ml). Après décantation et extractions, les phases organiques combinées sont lavées avec de la saumure, séchées sur Na₂SO₄ puis concentrées sous pression réduite à 40° C. La purification du solide obtenu, par chromatographie éclair sur gel de silice (éluant : heptane/acétate d'éthyle 3 :7 à acétate d'éthyle 100 %), donne le composé attendu sous forme d'un solide jaune (473 mg ; 61 % rendement).

SM/CL : MM calculée = 298,8 ; m/z = 299,1 (MH+)

RMN (¹H, 400 MHz, DMSO-*d₆*) : *δ* 1,37 (m, 2H), 1,49 (m, 4H), 1,74 (m, 2H), 2,34 (m, 6H), 3,55 (m, 2H), 6,75 (d, 1H), 8,40 (d, 1H), 8,96 (t, 1H).

### Etape 2 : N⁶,N⁶-dibutyl-3-nitro-N²-(3-pipéridin-1-ylpropyl)pyridine-2,6-diamine

A une solution de 6-chloro-3-nitro-*N*-(3-pipéridin-1-ylpropyl)pyridin-2-amine (59 mg, 1 éq) dans l'acétonitrile (3 ml) sont successivement additionnés le carbonate de potassium (54 mg, 2 éq) et une solution de dibutylamine (30 mg, 1,2 éq) dans l'acétonitrile (2 ml). Le mélange est chauffé a reflux pendant 15 heures puis refroidi à température ambiante et concentré sous pression réduite à 40° C. Le résidu est repris dans le dichlorométhane (200 ml) et l'eau (90 ml). Après décantation et extractions, les phases organiques combinées sont lavées avec de la saumure, séchées sur Na₂SO₄ puis concentrées sous pression réduite à 40° C. La purification du résidu obtenu, par chromatographie éclair sur gel de silice (éluant : heptane/acétate d'éthyle 1 :1 à acétate d'éthyle 100 %), donne le composé attendu (73 mg ; 95 % rendement).

SM/CL : MM calculée = 391,6 ; m/z = 392,2 (MH+)

### Etape 3 : chlorhydrate de 1-(4-{[5-(dibutylamino)-3-(3-pipéridin-1-ylpropyl)-3H-imidazo [4,5-b]pyridin-2-yl]amino}phényl)éthanone

Dans un tube à hémolyse placé dans un autoclave sont additionnés le *N*⁶,*N*⁶-dibutyl-3-nitro-*N*²-(3-pipéridin-1-ylpropyl)pyridine-2,6-diamine (70 mg) en solution dans un mélange d'acétate d'éthyle/ méthanol, 3 :1 (2 ml), et le palladium sur charbon 10 % (7 mg). Après 3 heures d'agitation sous atmosphère d'hydrogène (3 bars) à une température d'environ 20° C, le mélange est filtré sur célite dans un tube à hémolyse contenant une solution de 4-isothiocyanato-*N*-méthylbenzamide (43 mg, 1,2 éq) dans le tétrahydrofuranne (1 ml). Au filtrat ainsi obtenu est additionnée la résine *N*-cyclohexylcarbodiimide-*N*-méthyl-polystyrène (acquise auprès de Novabiochem ; charge 1,9 mmol/g ; 284 mg, 3 éq). Le mélange est chauffé à reflux pendant 18 heures, refroidi à température ambiante puis filtré sur fritté et le filtrat est concentré sous pression réduite à 40° C. La purification du résidu par chromatographie éclair sur gel de silice (éluant : dichlorométhane 100 % à dichlorométhane / méthanol 9 :1) donne le composé attendu sous forme de base. Le sel de chlorhydrate correspondant est formé par addition d'une solution d'acide chlorhydrique 1N dans l'éther éthylique. Le précipité obtenu est filtré et séché pour donner le composé attendu (72 mg).

SM/CL : MM calculée = 504,7 ; m/z = 505,2 (MH+)

RMN (¹H, 400 MHz, DMSO-*d*₆) : *δ* 0,92 (t, 6H), 1,34 (m, 5H), 1,55 (m, 4H), 1,77 (m, 5H), 2,35 (m, 2H), 2,59 (s, 3H), 2,88 (m, 2H), 3,18 (m, 2H), 3,38 (m, 2H), 3,49 (m, 4H), 4,48 (t, 2H), 6,60 (m, 1H), 7,59 (d, 1H), 7,70 (d, 2H), 8,06 (m, 2H), 10,62 (s, 1H), 11,71 (s, 1H).

### Préparation d'isothiocyanates non commerciaux :

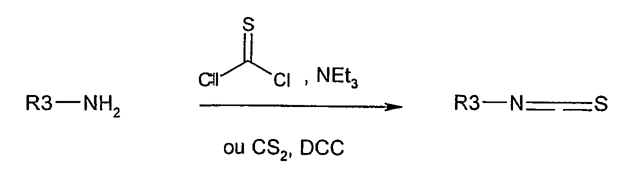

Une amine primaire peut être convertie en isothiocyanate, par traitement avec du thiophosgène en présence d'une base tertiaire telle que la triéthylamine, dans un solvant aprotique tel que le dichlorométhane ou le tétrahydrofuranne, à une température de 0-20° C pendant 0,3 à 2 heures, ou alternativement par traitement avec du disulfure de carbone et du cyclohexylcarbodiimide supporté sur une résine ou non dans un solvant aprotique tel que le dichlorométhane ou le tétrahydrofuranne, à une température de 0-70°C pendant 0,3 à 15 heures.

### Préparation du 4-isothiocyanato-N-méthylbenzamide :

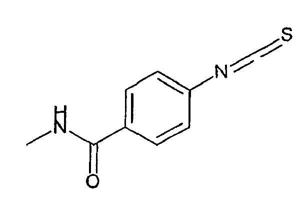

A une solution refroidie à 0° C, de 4-amino-*N*-méthylbenzamide (2 g, 1 éq) et de triéthylamine (5,6 ml, 3 éq) dans le tétrahydofuranne (260 ml) est additionné goutte à goutte le thiophosgène (1,13 ml, 1,1 éq). Le mélange est agité 30 min à 0° C puis le bain froid est retiré et l'agitation est poursuivie 30 min supplémentaires. Le mélange est additionné d'eau (100 ml) et d'éther diéthylique (250 ml). Après décantation et extractions, les phases organiques sont réunies, lavées avec de la saumure, séchées sur Na₂SO₄ puis concentrées sous pression réduite à 40° C. Le solide obtenu est recristallisé dans un mélange dichlorométhane /éther de pétrole (2,2 g ; 86 % rendement).

RMN (¹H, 400 MHz, DMSO-*d*₆) : δ 2,77 (d, 3H), 7,51 (AB, 2H), 7,88 (AB, 2H), 8,52 (m, 1H).

Selon la même procédure que celle décrite pour le *N*-(4-isothiocyanatophényl)acétamide, les isothiocyanates suivants peuvent être préparés :

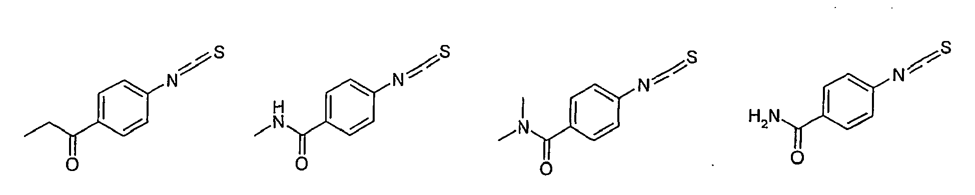

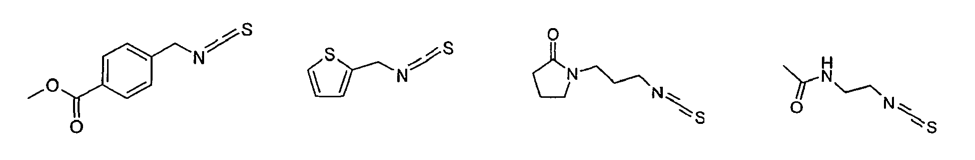

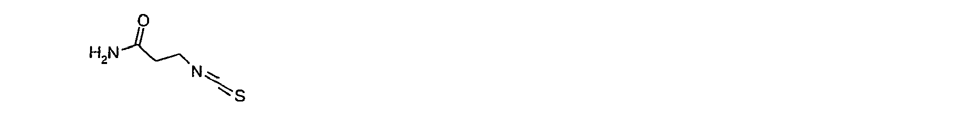

### Préparation de N-(4-isothiocyanatophényl)-N'-methoxyurée :

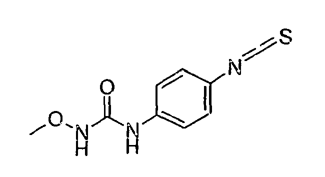

A une solution refroidie à 0° C de *tert*-butyl 4-aminophénylcarbamate (1,04 g) dans le dichlorométhane anhydre (100 ml) est additionné le carbonyldiimidazole (CDI) (1,62 g, 2 éq). Le mélange est ramené à une température de 20° C et est agité à cette température pendant 15 heures. Au milieu réactionnel refroidi à 0° C sont successivement additionnés la triéthylamine (7 ml, 10 éq) suivie du chlorhydrate de *O*-méthylhydroxylamine (4,2 g, 10 éq). Après 3 heures d'agitation à une température voisine de 20° C, le mélange est additionné d'eau saturée en hydrogénocarbonate de sodium et de chloroforme. Après décantation et extractions, les phases organiques réunies sont lavées avec de la saumure, séchées sur Na₂SO₄ puis concentrées sous pression réduite à 40° C pour conduire au *tert*-butyl 4-{[(méthoxyamino)carbonyl]amino}phénylcarbamate (1,33 g). Une suspension de ce dérivé dans l'acétate d'éthyle est traversée par un flux d'acide chlorhydrique gazeux jusqu'à ce que la réaction soit totale. Le précipité obtenu est filtré puis lavé à l'éther diéthylique et séché pour conduire au chlorhydrate de N-(4-aminophényl)-*N*'-méthoxyurée (1 g).

A une solution refroidie à 0° C, de chlorhydrate de *N*-(4-aminophényl)-*N*'-méthoxyurée (1 g) et de triéthylamine (3,2 ml, 5 éq) dans le tétrahydofuranne (90 ml) est additionné goutte à goutte le thiophosgène (0,38 ml, 1,1 éq). Le mélange est agité 15 min à 0° C puis est additionné d'eau et d'éther diéthylique. Après décantation et extractions, les phases organiques sont réunies, lavées avec de la saumure, séchées sur Na₂SO₄ puis concentrées sous pression réduite à 40° C. La purification par chromatographie éclair sur gel de silice (éluant : heptane / acétate d'éthyle 7 :3 à 3 :7) donne le composé attendu (630 mg ; 62 % rendement).

RMN (¹H, 400 MHz, DMSO-*d*₆) : δ 3,61 (s, 3H), 7,34 (AB, 2H), 7,67 (AB, 2H), 9,11 (s, 1H), 9,65 (s, 1H).

### Préparation d'acyl-isothiocyanates non commerciaux :

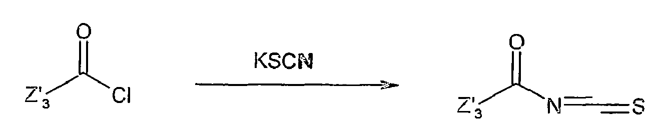

Les acyl-isothiocyanates peuvent être préparés à partir des chlorures d'acides correspondants par traitement avec du thiocyanate de potassium dans un solvant aprotique tel que l'acétonitrile à une température de 0-60° C pendant 0,2-5 heures.

### Méthyl-4-isothiocyanatocarbonylbenzoate :

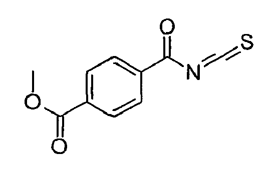

A une solution de méthyl-4-chlorocarbonylbenzoate (2 g) dans l'acétonitrile (30 ml) est additionné le thiocyanate de potassium (1,08 g, 1,1 éq). Après 1 heure d'agitation à environ 20° C, le mélange est filtré et le filtrat est concentré sous pression réduite à 40° C. Le solide obtenu est purifié par chromatographie éclair sur gel de silice (éluant : heptane / acétate d'éthyle 1 :1) pour donner le composé attendu (2,1 g ; 95 % rendement).

RMN (¹H, 400 MHz, DMSO-*d*₆) : δ 3,88 (s, 3H), 8,0 (m, 4H).

Selon la même procédure que celle décrite pour le méthyl-4-isothiocyanatocarbonylbenzoate, les isothiocyanates suivants peuvent être préparés :

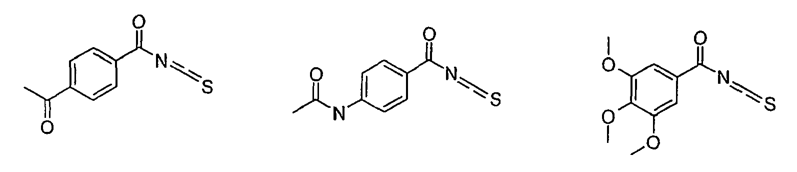

Selon le schéma réactionnel A et de façon analogue à la procédure décrite pour la synthèse du chlorhydrate de 4-{[3-(3-aminopropyl)-5-(diisobutylamino)-3*H-*imidazo[4,5-*b*]pyridin-2-yl]amino}-*N*-méthylbenzamide ou du chlorhydrate de 1-(4-{[5-(dibutylamino)-3-(3-pipéridin-1-ylpropyl)-3*H*-imidazo[4,5-*b*] pyridin-2-yl]amino} phényl)éthanone, les composés suivants peuvent être préparés : 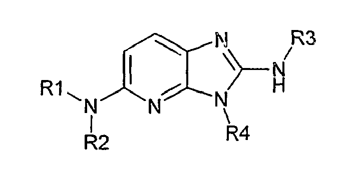 dans lesquels R₁R₂N représente l'un des radicaux ci-après : 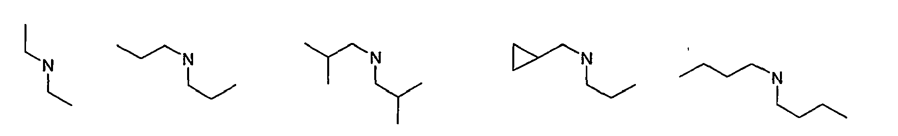 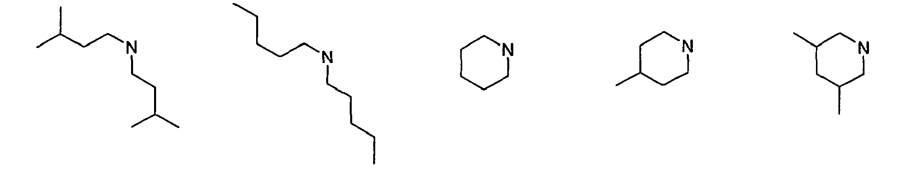 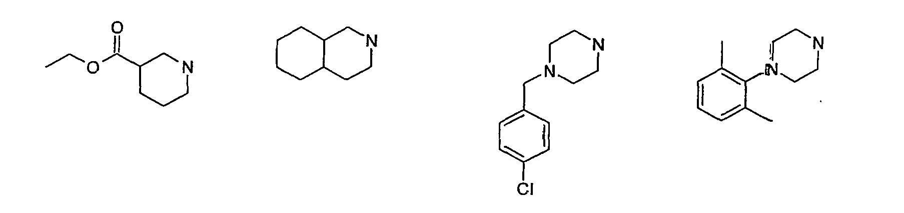 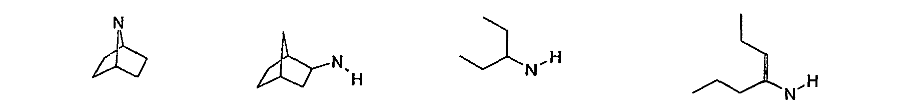 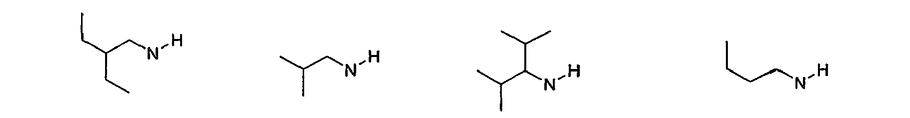 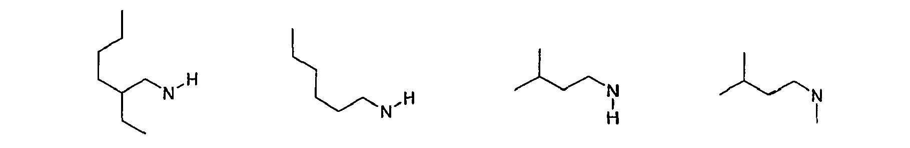 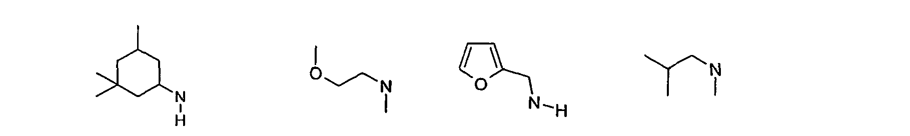 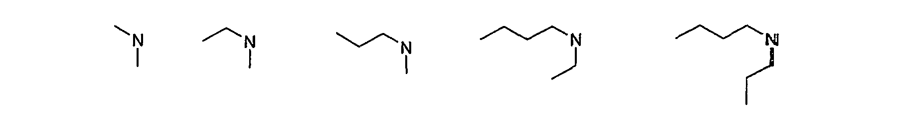 R₃ représente l'un des radicaux ci-après : 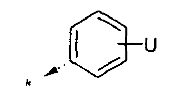 1 ou plusieurs substitutions choisies parmi :
**U** = H, F, Cl, Br, I, NO₂, OMe, SMe, Me, Et, iPr, tBu, CF₃, OCF₃, C(O)OMe, C(O)OEt, C(O)Me, C(O)Et, NHC(O)Me, C(O)NHMe, C(O)NH₂, S(O)₂NH₂, NHC(O)NHMe, NHC(O)NHOMe 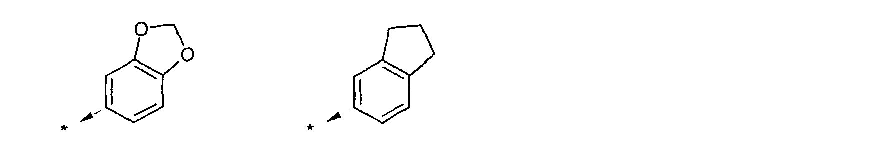 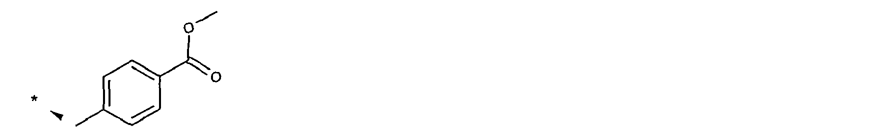 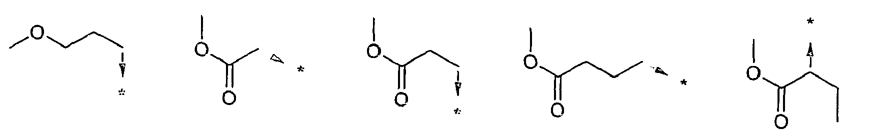 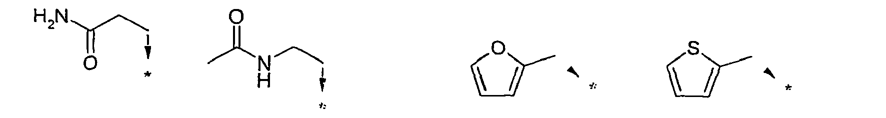 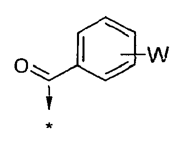 W = H, F, Cl, Br, NO₂, Me, OMe, OEt, CF₃, OCF₃, tBu, C(O)Me, C(O)OMe, C(O)NHMe
et R₄ représente l'un des radicaux ci-après :

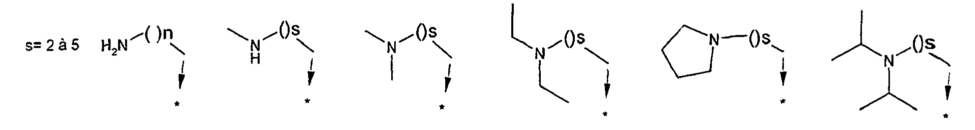

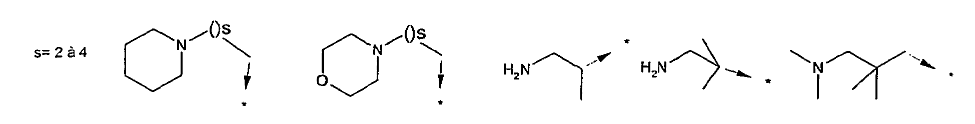

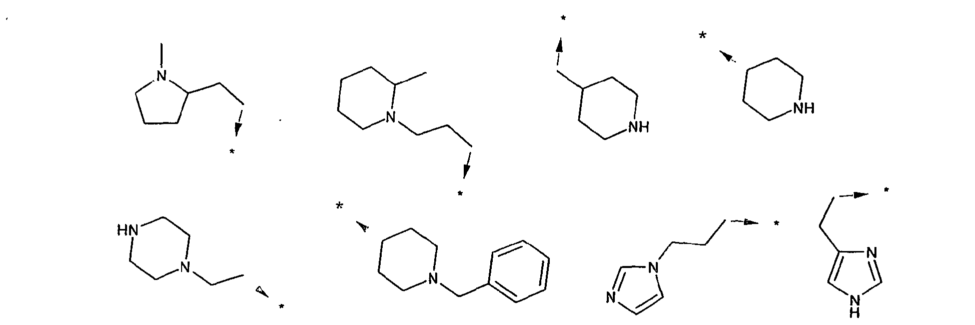

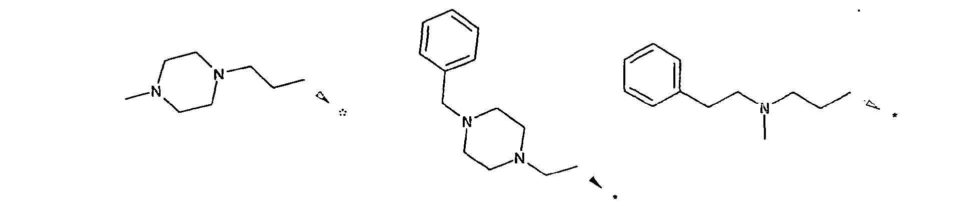

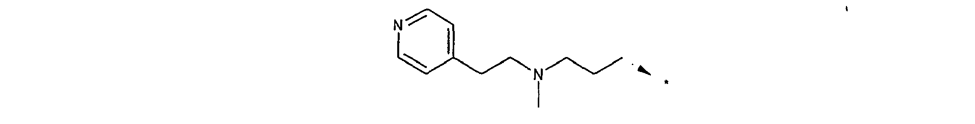

### B. Préparation selon le schéma réactionnel B :

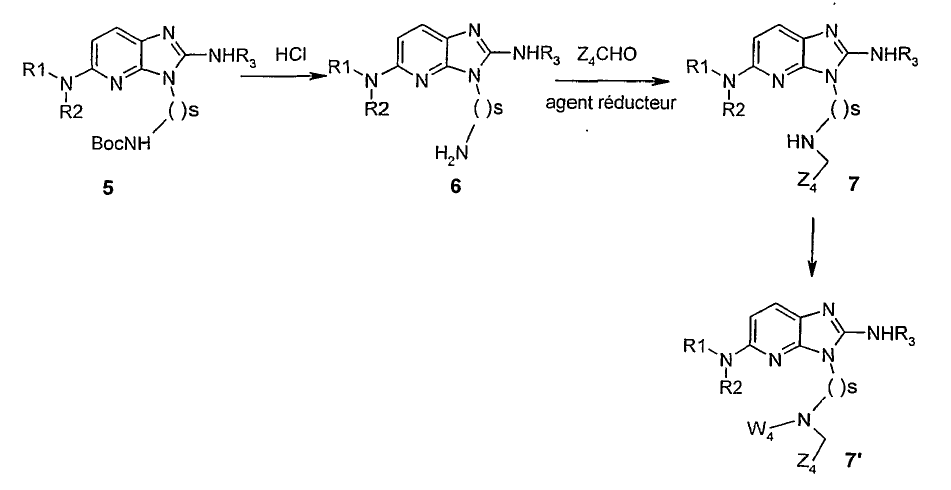

Comme décrit dans le schéma B, le dérivé (5), préparé selon le schéma réactionnel A peut être traité par un acide organique ou inorganique comme l'acide trifluoroacétique ou l'acide chlorhydrique (aqueux ou sous forme gazeuse) dans un solvant aprotique tel que le dichlorométhane, l'éther diéthylique ou l'acétate d'éthyle à une température de 0-20° C pendant 0,5 à 5 heures, pour conduire à l'amine (6). L'amine (6) peut réagir avec un aldéhyde dans un solvant protique ou aprotique, tel que le dichlorométhane, le tétrahydrofuranne ou le méthanol, pendant 1 à 15 heures à une température de 0-50°C. L'imine résultante est ensuite réduite *in situ* par un agent réducteur supporté sur une résine ou non, de préférence le triacétoxyborohydrure de sodium, le cyanoborohydrure de sodium ou borohydrure supporté sur une résine, avec ou sans la présence d'un acide tel que l'acide acétique, à une température de 20 à 50° C pendant une durée de 0,2 à 5 heures, pour conduire au composé (7). L'amine secondaire (7) peut éventuellement subir une seconde amination réductrice dans les mêmes conditions opératoires que celles décrites précédemment pour conduire à l'amine tertiaire (7').

### Exemple B1 : 3-{2-[(4-méthoxybenzyl)amino]éthyl}-N⁵,N⁵-bis(3-méthylbutyl)-N²-(3,4,5-triméthoxyphényl)-3H-imidazo[4,5-b]pyridine-2,5-diamine

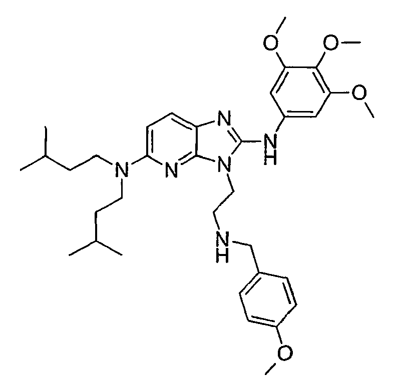

### Etape 1 : tert-butyl 2-[(6-chloro-3-nitropyridin-2-yl)amino]éthylcarbamate

A la 2,6-dichloro-3-nitropyridine (10,2 g, 1 éq) en solution dans le toluène (200 ml) sont successivement additionnés le carbonate de potassium (8,3 g, 1,2 éq) et le *tert*-butyl-*N*(2-aminoéthyl)carbamate (8 g, 1 éq). Après 4 heures d'agitation à une température voisine de 20° C, le mélange est concentré sous pression réduite à 40° C puis additionné d'eau (130 ml) et de dichlorométhane (250 ml). Après décantation et extractions, les phases organiques combinées sont lavées avec de la saumure, séchées sur Na₂SO₄ puis concentrées sous pression réduite à 40° C. La purification du solide obtenu, par chromatographie éclair sur gel de silice (éluant : heptane/acétate d'éthyle 7 :3), donne le composé attendu sous forme d'un solide jaune (12,5 g ; 79 % rendement).

SM/GL : MM calculée = 316,7 ; m/z = 317,1 (MH+)

RMN (¹H, 400 MHz, DMSO-*d₆*): *δ* 1,34 (s, 9H), 3,19 (dd, 2H), 3,56 (dd, 2H), 6,78 (d, 1H), 6,94 (t, 1H), 8,41 (d, 1H), 8,68 (t, 1H).

### Etape 2 : tert-butyl 2-({6-[bis(3-méthylbutyl)amino]-3-nitropyridin-2-yl}amino)éthyl carbamate

A une solution de *tert*-butyl 2-[(6-chloro-3-nitropyridin-2-yl)amino]éthylcarbamate (316 mg, 1 éq) dans l'acétonitrile (10 ml) sont successivement additionnés le carbonate de potassium (207 mg, 1,5 éq) et la diisoamylamine (188 mg, 1,2 éq). Le mélange est chauffé a reflux pendant 5 heures puis refroidi à température ambiante et concentré sous pression réduite à 40° C. Le résidu est repris dans le dichlorométhane (50 ml) et l'eau (15 ml). Après décantation et extractions, les phases organiques combinées sont lavées avec de la saumure, séchées sur Na₂SO₄ puis concentrées sous pression réduite à 40° C. La purification du résidu obtenu, par chromatographie éclair sur gel de silice (éluant : heptane/acétate d'éthyle 9 :1 à 7:3), donne le composé attendu (430 mg ; 98 % rendement).

SM/CL : MM calculée = 437,6 ; m/z = 438,3 (MH+)

RMN (¹H, 400 MHz, DMSO-*d₆*) : δ 0,91 (d, 12H), 1,35 (s, 9H), 1,45 (m, 4H), 1,60 (m, 2H), 3,17 (m, 2H), 3,40 (m, 2H), 3,56 (m, 4H), 6,10 (d, 1H), 6,93 (t, 1H), 8,04 (d, 1H), 8,83 (t, 1H).

### Etape 4 : tert-butyl 2-{5-[bis(3-méthylbutyl)amino]-2-[(3,4,5-triméthoxyphényl)amino]-3H-imidazo[4,5-b]pyridin-3-yl}éthylcarbamate

Dans un autoclave sont additionnés le *tert*-butyl 2-({6-[bis(3-méthylbutyl)amino]-3-nitropyridin-2-yl}amino)éthyl carbamate (400 mg, 1 éq) en solution dans un mélange d'acétate d'éthyle/ éthanol 2 :1 (60 ml), et le palladium sur charbon 10 % (40 mg). Après 5 heures d'agitation sous atmosphère d'hydrogène (3 bars) à une température d'environ 20° C, le mélange est filtré sur célite dans un ballon contenant le 3,4,5-triméthoxyphényl- isothiocyanate (248 mg, 1,2 éq). Le filtrat est concentré sous pression réduite à 40° C puis le résidu est dilué dans le tétrahydrofuranne (50 ml) et la résine *N*-cyclohexylcarbodiimide-*N*-méthyl-polystyrène (acquise auprès de Novabiochem; charge 1,9 mmol/g ; 1,92 g, 4 éq) est additionnée. Le mélange est chauffé à reflux pendant 8 heures puis refroidi à température ambiante et filtré sur fritté.

Le filtrat est concentré sous pression réduite à 40° C. La purification du résidu par chromatographie éclair sur gel de silice (éluant : heptane /acétate d'éthyle 9:1 à 6 :4) donne le composé attendu (324 mg ; 59 % rendement).

SM/CL : MM calculée = 598,8 ; m/z = 599,4 (MH+)

RMN (¹H, 400 MHz, DMSO-*d₆*) : δ 0,93 (d, 12H), 1,28 (s, 9H), 1,45 (m, 4H), 1,60 (m, 2H), 3,29 (m, 2H), 3,43 (m, 4H), 3,61 (s, 3H), 3,77 (s, 6H), 4,17 (t, 2H), 6,24 (d, 1H), 6,90 (t, 1H), 7,21 (s, 2H), 7,45 (d, 1H), 8,54 (s, 1H).

### Etape 5 : 3-(2-aminoéthyl)-N⁵,N⁵-bis(3-méthylbutyl)-N²-(3,4,5-triméthoxyphényl)-3H-imidazo[4,5-b]pyridine-2,5-diamine

A une solution de *tert*-butyl 2-{5-[bis(3-méthylbutyl)amino]-2-[(3,4,5-triméthoxyphényl) amino]-3*H*-imidazo[4,5-*b*]pyridin-3-yl}éthylcarbamate (300 mg) dans l'acétate d'éthyle (6 ml) est additionnée une solution d'acide chlorhydrique dans le dioxanne (4N, 3 ml). Après 4 heures d'agitation à une température voisine de 20° C, le mélange est filtré. Le solide obtenu est lavé à l'éther diéthylique. Le chlorhydrate ainsi obtenu est repris dans du dichlorométhane et de l'eau saturée en hydrogénocarbonate de sodium. Après décantation et extractions, les phases organiques réunies sont lavées avec de la saumure, séchées sur Na₂SO₄ puis concentrées sous pression réduite à 40° C pour donner le composé attendu sous forme de base libre (237 mg, 95 % rendement).

SM/CL : MM calculée = 498,7; m/z = 499,3 (MH+)

RMN (¹H, 400 MHz, DMSO-*d₆*) : δ 0,93 (d, 12H), 1,45 (m, 4H), 1,59 (m, 2H), 2,98 (t, 2H), 3,35 (t, 4H), 3,61 (s, 3H), 3,77 (s, 6H), 4,10 (t, 2H), 6,23 (d, 1H), 7,16 (s, 2H), 7,44 (d, 1H).

### Etape 6 : 3-{2-[(4-méthoxybenzyl)amino]éthyl}-N⁵,N⁵-bis(3-méthylbutyl)-N²-(3,4,5-triméthoxyphényl)-3H-imidazo[4,5-b]pyridine-2,5-diamine

Une solution de 3-(2-aminoéthyl)-*N*⁵,*N*⁵-bis(3-méthylbutyl)-*N*²-(3,4,5-triméthoxyphenyl)-3*H*-imidazo[4,5-*b*]pyridine-2,5-diamine (83 mg, 1 éq) et de *p*-anisaldéhyde (25 mg, 1 éq) dans le dichlorométhane (1,5 ml) est agitée 6 heures à une température voisine de 20° C. Le mélange est dilué avec du méthanol (2 ml) puis additionné de triacétoxyborohydrure de sodium (70 mg, 2 éq). Après 0,5 heure d'agitation à une température voisine de 20° C, le mélange est additionné de dichlorométhane (20 ml) et d'eau saturée en hydrogénocarbonate de sodium (10 ml). Après décantation et extractions, les phases organiques réunies sont lavées avec de la saumure, séchées sur Na₂SO₄ puis concentrées sous pression réduite à 40° C. La purification du résidu par chromatographie éclair sur gel de silice (éluant : heptane/acétate d'éthyle 1:1 à 2 :8) donne le composé attendu (70 mg, 68 % rendement).

SM/CL : MM calculée = 618,8 ; m/z = 619,4 (MH+)

RMN (¹H, 400 MHz, DMSO-*d₆*) : δ 0,92 (d, 12H), 1,44 (m, 4H), 1,57 (m, 2H), 2,89 (t, 2H), 3,44 (t, 4H), 3,60 (s, 3H), 3,70 (m, 8H), 4,19 (t, 2H), 6,23 (d, 1H), 6,80 (d, 2H), 7,06 (s, 2H), 7,18 (d, 2H), 7,45 (d, 1H), 9,90 (m, 1H).

### C. Préparation selon le schéma réactionnel C :

Les composés (7) pour lesquels s = 3 peuvent également être préparés selon le schéma C suivant :

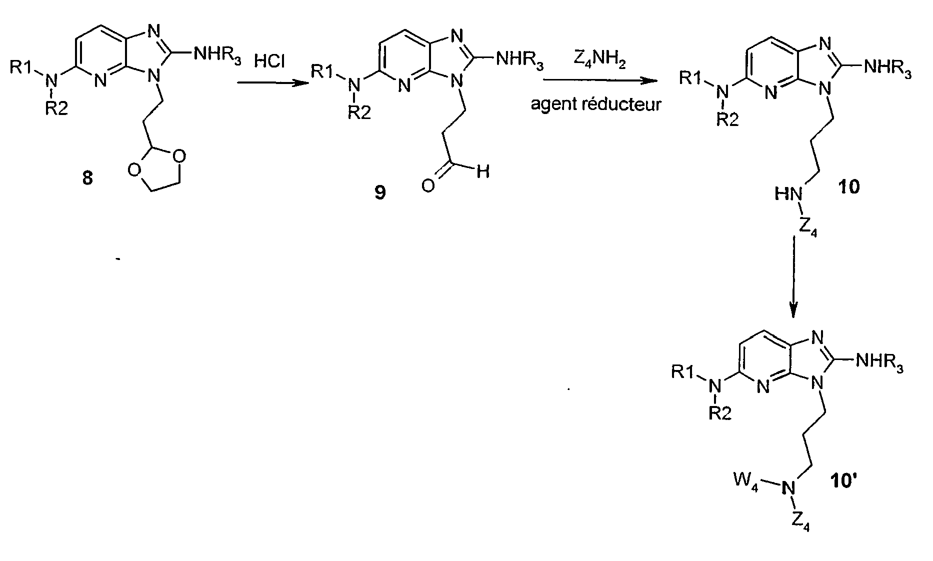

Comme décrit dans le schéma C, le dérivé (8) préparé selon le schéma réactionnel A peut être traité soit par un acide organique tel que le tosylate de pyridinium ou l'acide paratoluènesulfonique dans un solvant aprotique tel que l'acétone en présence d'eau, à une température de 20-70° C pendant 2 à 12 heures, soit par un acide inorganique tel que le chlorure d'hydrogène aqueux dans un solvant aprotique tel que le tétrahydrofuranne à une température de 0-20° C pendant 6 à 18 heures pour conduire au composé (9). L'aldéhyde (9) peut ensuite être traité par une amine dans un solvant protique ou aprotique tel que le dichlorométhane, le tétrahydrofuranne ou le méthanol pendant 1 à 18 heures à une température de 20° C. L'imine résultante est ensuite réduite *in situ* par un agent réducteur, de préférence le triacétoxyborohydrure de sodium ou le cyanoborohydrure de sodium, en présence ou non d'un acide tel que l'acide acétique, à une température de 20-50° C pendant une durée de 0,2 à 6 heures, pour conduire au composé (10). L'amine secondaire (10) peut éventuellement subir une seconde amination réductrice dans les mêmes conditions opératoires que celles décrites précédemment pour conduire à l'amine tertiaire (10').

Selon le schéma réactionnel B ou C, les composés suivants peuvent être préparés : 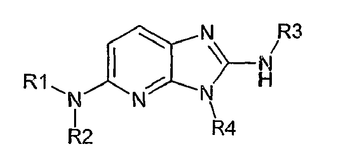 dans lesquels R₁R₂N représente l'un des radicaux ci-après : 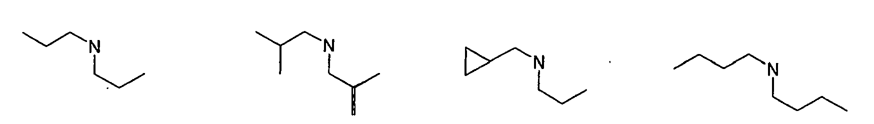 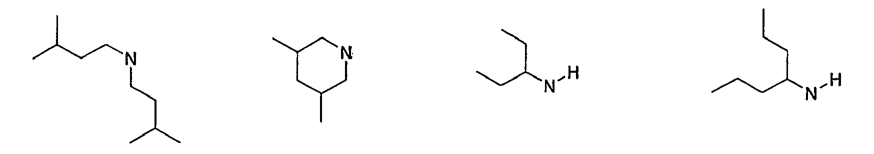 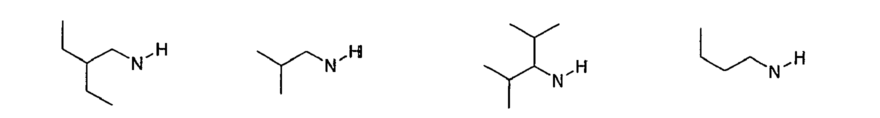 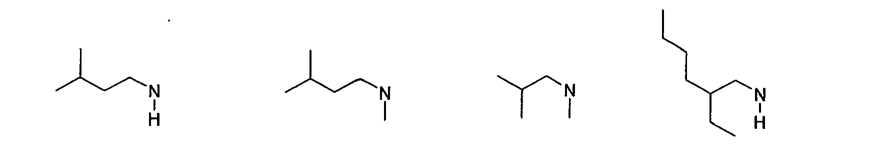 R₃ représente l'un des radicaux ci-après : 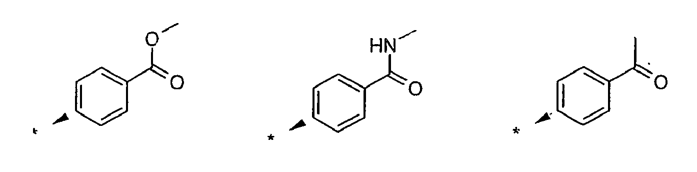 et R₄ représente l'un des radicaux ci-après :

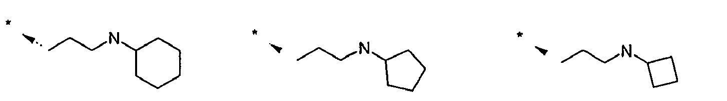

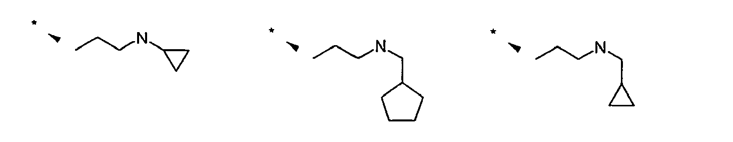

### D. Préparation selon le schéma réactionnel D :

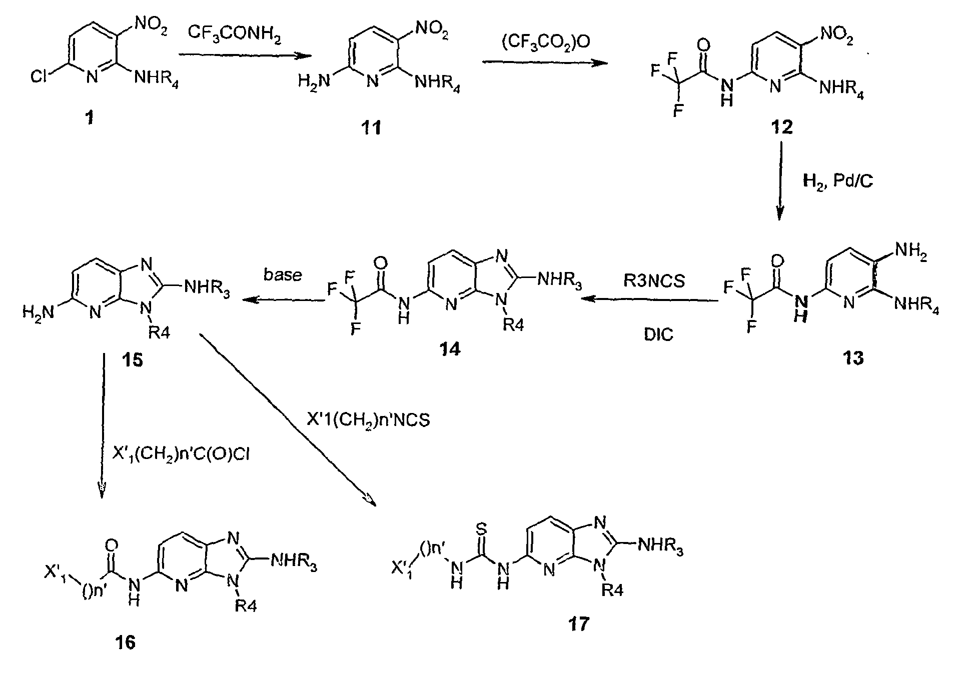

Comme décrit dans le schéma D, le dérivé chloré (1) préparé selon le schéma réactionnel A, peut être converti en aniline (11) par traitement avec du trifluoroacétamide, en présence d'une base inorganique tel que le carbonate de césium ou potassium et d'un catalyseur de transfert de phase tel que le bromure de tétrabutylammonium, dans un solvant aprotique polaire tel que le diméthylformamide, à une température de 80-110° C pendant 2-6 heures. L'aniline (11) peut-être protégée sous forme de trifluoroacétamide par traitement avec de l'anhydride trifluoroacétique en présence d'une amine tertiaire telle que la triéthylamine ou la pyridine dans un solvant aprotique tel que le dichlorométhane à une température de 0-20° C pendant 0,2-2 heures, pour conduire au composé (12). Le dérivé nitro (12) est réduit par hydrogénation catalytique en présence de palladium sur charbon 10 % dans un solvant inerte tel que le méthanol, éthanol, acétate d'éthyle ou un mélange de ces solvant à une température de 18-25° C, pendant 2 à 8 heures pour conduire à la dianiline (13). Le dérivé (13) est ensuite traité par un isothiocyanate en présence d'un agent de couplage supporté sur une résine ou non tel que le diisopropylcarbodiimide ou dicyclohexylcarbodiimide ou la résine *N*-cyclohexylcarbodiimide *N*-méthyl polystyrène dans un solvant inerte tel que le tétrahydrofuranne, chlorure de méthylène, ou chloroforme à une température de 20-70° C pendant 2 à 72 heures pour conduire au dérivé (14). Le trifluoroacétamide (14) est hydrolysé en présence de carbonate de potassium ou sodium dans un solvant protique polaire tel que le méthanol ou l'éthanol en présence d'eau, à une température de 50-80° C pendant 8-32 heures pour conduire à l'aniline (15). L'aniline (15) peut réagir avec un chlorure d'acide dans un solvant aprotique tel que le dichlorométhane ou tétrahydrofuranne en présence d'une base telle qu'une amine tertiaire supportée ou non sur une résine, telle que la triéthylamine ou la résine morpholino-méthylpolystyrène, à une température de 0-40° C pendant 0,3-2 heures pour conduire à l'amide (16). L'aniline (15) peut également réagir avec un isothiocyanate dans un solvant aprotique tel que le tétrahydrofuranne à une température de 20-70° C pendant 5-24 heures pour conduire à la thiourée (17).

### Exemple D1 : chlorhydrate de N-{3-(2-aminoéthyl)-2-[(3,4,5-triméthoxyphényl) amino]-3H-imidazo[4,5-b] pyridin-5-yl}-2-propylpentanamide

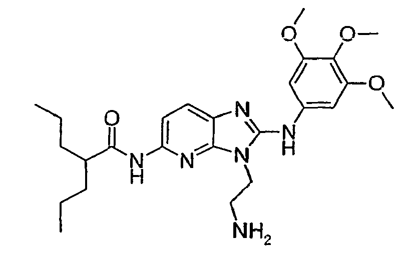

### Etape 1 : tert-butyl 2-[(6-amino-3-nitropyridin-2-yl)amino]éthylcarbamate

A une solution de *tert*-butyl 2-[(6-chloro-3-nitropyridin-2-yl)amino]éthylcarbamate préparé selon l'exemple B1, (2 g, 1 éq) dans le diméthylformamide (100 ml) sont successivement additionnés le carbonate de potassium (0,87 mg, 1 éq), le bromure de tétrabutylammonium (0,2 g, 0,1 éq) et le trifluoroacétamide (1,4 g, 2 éq). Le mélange est chauffé 4 heures à 100° C puis refroidi à température ambiante et filtré sur fritté. Le filtrat est concentré sous pression réduite à 40° C puis le résidu obtenu est additionné d'eau (40 ml) et de dichlorométhane (100 ml). Après décantation et extractions, les phases organiques combinées sont lavées avec de la saumure, séchées sur Na₂SO₄ puis concentrées sous pression réduite à 40° C. La purification du solide obtenu, par chromatographie éclair sur gel de silice (éluant : heptane/acétate d'éthyle 6 :4), donne le composé attendu (1,21 g ; 64 % rendement).

SM/CL : MM calculée = 297,3 ; m/z = 298,2 (MH+)

RMN (¹H, 400 MHz, DMSO-*d₆*) : δ 1,35 (s, 9H), 3.,17 (dd, 2H), 3,53 (dd, 2H), 5,90 (d, 1H), 6,93 (t, 1H), 7, 38 (m, 2H), 7,98 (d, 1H), 8,88 (t, 1H).

### Retape 2 : tert-butyl 2-({3-nitro-6-[(trifluoroacétyl)amino]pyridin-2-yl}amino)éthyl carbamate

A une solution refroidie à 0° C de *tert*-butyl 2-[(6-amino-3-nitropyridin-2-yl)amino] éthylcarbamate (840 mg, 1 éq) dans le dichlorométhane (25 ml) sont successivement additionnés la triéthylamine (0,59 ml) puis l'anhydride trifluoroacétique (0,56 ml). Après 1 heure d'agitation à 0° C, le mélange est additionné d'eau (10 ml). Après décantation et extractions, les phases organiques combinées sont lavées avec de la saumure, séchées sur Na₂SO₄ puis concentrées sous pression réduite à 40° C. Le solide jaune obtenu est recristallisé dans un mélange dichlorométhane / heptane et lavé à l'éther diéthylique (910 mg ; 81 % rendement).

RMN (¹H, 400 MHz, DMSO-*d₆*) : δ 1,31 (s, 9H), 3,22 (dd, 2H), 3,62 (dd, 2H), 6,86 (t, 1H), 7, 27 (d, 1H), 8,50 (d, 1H), 11,90 (s, 1H).

### Etape 3 : tert-butyl 2-({3-amino-6-[(trifluoroacétyl)amino]pyridin-2-yl}amino) éthyl carbamate

Dans un autoclave sont additionnés le *tert*-butyl 2-({3-nitro-6-[(trifluoroacétyl)amino]pyridin-2-yl}amino)éthylcarbamate (900 mg) en solution dans un mélange d'acétate d'éthyle/ éthanol 2 :1 (120 ml), et le palladium sur charbon 10 % (130 mg). Après 15 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu (820 mg ; 98 % rendement).

SM/CL : MM calculée = 363,3 ; m/z = 364,2 (MH+)

RMN (¹H, 400 MHz, DMSO-*d₆*) : δ 1,36 (s, 9H), 3,15 (dd, 2H), 3,37 (dd, 2H), 4,70 (m, 2H), 5,77 (t, 1H), 6,72 (d, 1H), 6,79 (t,1H), 6,88 (d, 1H), 10,81 (s, 1H).

### Etape 4 : tert-butyl 2-{5-[(trifluoroacétyl)amino]-2-[(3,4,5-triméthoxyphényl)amino]-3H-imidazo[4,5-b]pyridin-3-yl}éthylcarbamate

A une solution de *tert*-butyl 2-({3-amino-6-[(trifluoroacétyl)amino]pyridin-2-yl}amino)éthylcarbamate (800 mg, 1 éq) dans le tétrahydrofuranne (50 ml) sont successivement additionnés le 3,4,5-triméthoxyphényl isothiocyanate (600 mg, 1,2 éq) et la résine *N*-cyclohexylcarbodiimide-*N*-méthyl-polystyrène (acquise auprès de Novabiochem; charge 1,9 mmol/g; 4,6 g, 4 éq). Le mélange est chauffé à reflux pendant 24 heures puis refroidi à température ambiante et filtré sur fritté. Le filtrat est concentré sous pression réduite à 40° C. La purification du résidu par chromatographie éclair sur gel de silice (éludant : heptane /acétate d'éthyle 6 :4 à 3 :7) donne le composé attendu sous forme d'un solide crème (750 mg ; 62 % rendement).

SM/CL : MM calculée = 554,5 ; m/z = 555,2 (MH+)

RMN (¹H, 400 MHz, DMSO-*d₆*) : δ 1,24 (s, 9H), 3,32 (m, 2H), 3,63 (s, 3H), 3,79 (s, 6H), 4,30 (m, 2H), 6,95 (t, 1H), 7,31 (s, 2H), 7,59 (d, 1H), 7,72 (d, 1H), 8,93 (s, 1H), 11,7 (s, 1H).

### Etape 5 : tert-butyl 2-{5-amino-2-[(3,4,5-triméthoxyphényl)amino]-3H-imidazo[4,5-b] pyridin-3-yl} éthylcarbamate

Au *tert*-butyl 2-{5-[(trifluoroacétyl)amino]-2-[(3,4,5-triméthoxyphényl)amino]-3*H-*imidazo[4,5-*b*]pyridin-3-yl}éthylcarbamate (470 mg, 1 éq) dans le méthanol (32 ml) et l'eau (2 ml) est additionné le carbonate de potassium (1,17 g, 10 éq). Le mélange est chauffé au reflux pendant 26 heures puis refroidi à température ambiante et concentré sous pression réduite à 40° C. Le résidu est additionné d'eau (15 ml) et de dichlorométhane (50 ml). Après décantation et extractions, les phases organiques combinées sont lavées avec de la saumure, séchées sur Na₂SO₄ puis concentrées sous pression réduite à 40° C pour donner le composé attendu (379 mg ; 97 % rendement).

SM/CL : MM calculée = 458,5 ; m/z = 459,2 (MH+)

RMN (¹H, 400 MHz, DMSO-*d₆*) : δ 1,31 (s, 9H), 3,25 (m, 2H), 3,61 (s, 3H), 3,77 (s, 6H), 4,13 (t, 2H), 5,50 (m, 2H), 6,22 (d, 1H), 7,01 (t, 1H), 7,21 (s, 2H), 7,37 (d, 1H), 8,50 (s, 1H).

### Etape 6 : tert-butyl 2-{5-[(2-propylpentanoyl)amino]-2-[(3,4,5-triméthoxyphényl) amino]-3H-imidazo[4,5-b]pyridin-3-yl}éthylcarbamate

A une solution de *tert*-butyl 2-{5-amino-2-[(3,4,5-triméthoxyphényl)amino]-3*H-*imidazo[4,5-*b*] pyridin-3-yl}éthylcarbamate (37 mg, 1 éq) dans le dichlorométhane (2 ml) sont successivement additionnés la morpholino-méthylpolystyrène (acquise auprès de Novabiochem ; charge 3,64 mmol/g ; 33 mg, 1,5 éq) et le chlorure de 2-propylpentanoyl (15 mg, 1,2 éq). Le mélange est agité 1 heure à une température voisine de 20° C puis filtré sur fritté. Le filtrat est concentré sous pression réduite à 40° C. La purification du résidu par chromatographie éclair sur gel de silice (éluant : heptane / acétate d'éthyle 4 :6 à 3 :7) donne le composé attendu (34 mg ; 73 % rendement).

SM/CL : MM calculée = 584,7 ; m/z = 585,3 (MH+)

RMN (¹H, 400 MHz, DMSO-*d₆*): δ 0,86 (t, 6H),1,27 (s, 9H), 1,18-1,34 (m, 6H), 1,53 (m, 2H), 2,65 (m, 1H), 3,31 (m, 2H), 3,63 (s, 3H), 3,79 (s, 6H), 4,25 (t, 2H), 7,0 (t, 1H), 7,30 (s, 2H), 7,61 (d, 1H), 7,92 (d, 1H), 8,80 (s, 1H), 10,18 (s, 1H).

### Etape 7 : chlorhydrate de N-{3-(2-aminoéthyl)-2-[(3,4,5-triméthoxyphényl)amino]-3H-imidazo[4,5-b]pyridin-5-yl}-2-propylpentanamide

A une solution de *tert*-butyl 2-{5-[(2-propylpentanoyl)amino]-2-[(3,4,5-trimethoxyphenyl) amino]-3*H*-imidazo[4,5-*b*]pyridin-3-yl}ethylcarbamate (30 mg) dans l'acétate d'éthyle (1 ml) est additionnée une solution d'acide chlorhydrique dans le dioxanne (4N, 1 ml). Après 1 heure d'agitation à une température voisine de 20° C, le mélange est concentré sous pression réduite à 40° C. Le solide obtenu est lavé à l'éther éthylique et séché (29 mg, 97 % rendement).

SM/CL : MM calculée = 485,2 ; m/z = 484,6 (MH+)

RMN (¹H, 400 MHz, DMSO-*d₆*): *δ* 0,86 (t, 6H), 1,18-1,36 (m, 6H), 1,56 (m, 2H), 2,62 (m, 1H), 3,31 (m, 2H), 3,69 (s, 3H), 3,79 (s, 6H), 4,66 (t, 2H), 7,0 (m, 2H), 7,72 (d, 1H), 8,03 (d, 1H), 8,49 (m, 3H), 10,58 (s, 1H).

### Exemple D2 : N-{3-(2-aminoéthyl)-2-[(3,4,5-triméthoxyphényl)amino]-3H-imidazo [4,5-b] pyridin-5-yl}-N'-(sec-butyl)thiourée

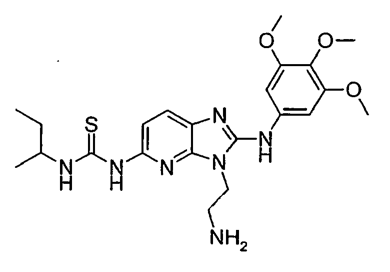

### Etape 1 : tert-butyl 2-{5-{[(sec-butylamino)carbonothioyl]amino}-2-[(3,4,5-triméthoxyphényl)amino]-3H-imidazo[4,5-b]pyridin-3-yl}éthylcarbamate

Une solution de *tert*-butyl 2-{5-amino-2-[(3,4,5-trimethoxyphenyl)amino]-3*H-*imidazo[4,5-*b*] pyridin-3-yl}ethylcarbamate préparé selon l'exemple D1 (37 mg, 1 éq) et de *sec*-butyl isothiocyanate (20 mg, 2 éq) dans le tétrahydrofuranne (3 ml) est chauffée au reflux pendant 17 heures puis refroidie à température ambiante et concentrée sous pression réduite à 40° C. La purification du résidu par chromatographie éclair sur gel de silice (éluant : heptane /acétate d'éthyle 4 :6 à 3 :7) donne le composé attendu (30 mg ; 65 % rendement).

SM/CL : MM calculée = 573,7 ; m/z = 574,2 (MH+)

RMN (¹H, 400 MHz, DMSO-*d₆*): δ 0,94 (t, 3H),1,23 (s, 9H), 1,29 (d, 3H), 1,68 (m, 1H), 1,75 (m, 1H), 3,31 (m, 2H), 3,62 (s, 3H), 3,78 (s, 6H), 4,25 (t, 2H), 4,30 (m, 1H), 6,90 (d, 1H), 6,95 (t, 1H), 7,27 (s, 2H), 7,67 (d, 1H), 8,82 (s, 1H), 10,37 (s, 1H), 11,04 (d, 1H).

### Etape 2 : chlorhydrate de N-{3-(2-aminoéthyl)-2-[(3,4,5-triméthoxyphényl)amino]-3H-imidazo[4,5-b] pyridin-5-yl}-N'-(sec-butyl)thiourée

A une solution de *tert*-butyl 2-{5-{[(*sec*-butylamino)carbonothioyl]amino}-2-[(3,4,5-triméthoxyphényl)amino]-3*H*-imidazo[4,5-*b*]pyridin-3-yl}éthylcarbamate (22 mg) dans l'acétate d'éthyle (1 ml) est additionnée une solution d'acide chlorhydrique dans le dioxanne (4N, 0,7 ml). Après 1 heure d'agitation à une température voisine de 20° C, le mélange est concentré sous pression réduite à 40° C. Le solide obtenu est lavé à l'éther éthylique et séché (20 mg, 91 % rendement).

SM/CL : MM calculée = 473,6 ; m/z = 474,2 (MH+)

RMN (¹H, 400 MHz, DMSO-*d₆*) : δ 0,94 (t, 3H), 1,27 (d, 3H), 1,69 (m, 2H), 3,31 (m, 2H), 3,67 (s, 3H), 3,79 (s, 6H), 4,32 (m, 1H), 4,68 (m, 2H), 7,10 (d, 1H), 7,18 (m, 2H), 7,78 (d, 1H), 8,53 (s, 3H), 10,50 (m, 1H), 10,60 (s, 1H).

Selon le schéma réactionnel D et selon la procédure décrite pour le chlorhydrate de *N*-{3-(2-aminoéthyl)-2-[(3,4,5-triméthoxyphényl)amino]-3*H*-imidazo[4,5-*b*] pyridin-5-yl}-2-propylpentanamide, les composés suivants peuvent être préparés : 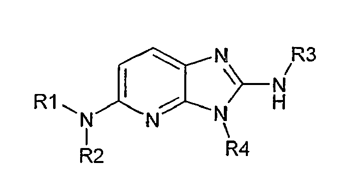 dans lesquels R₁R₂N représente l'un des radicaux ci-après : 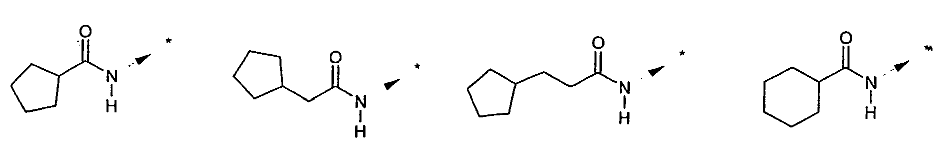 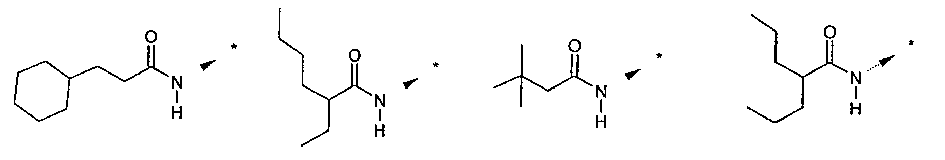 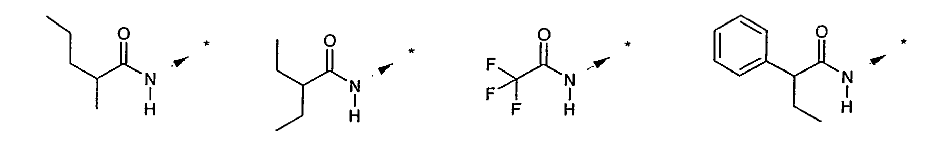 R₃ représente l'un des radicaux ci-après : 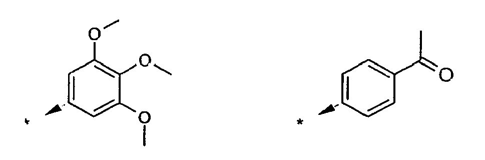 et R₄ représente l'un des radicaux ci-après :

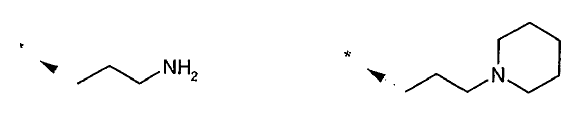

### E. Préparation selon le schéma réactionnel E :

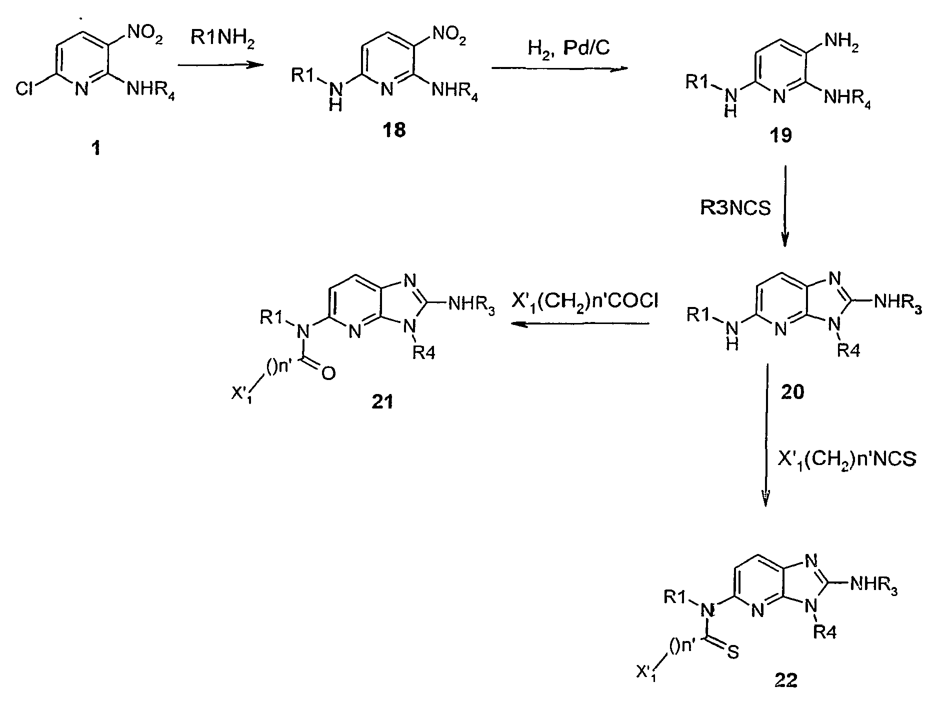

Comme décrit dans le schéma E, le dérivé chloré (1) préparé selon le schéma réactionnel A, peut réagir avec une amine primaire, en présence d'une base organique telle qu'une amine tertiaire ou une base inorganique tel que le carbonate de potassium ou césium, dans un solvant aprotique polaire tel que l'acétonitrile, le diméthylformamide ou l'HMPA à une température de 20-70° C pendant 2-18 heures pour conduire au composé (18). La fonction nitro du composé (18) est réduite par hydrogénation catalytique en présence de palladium sur charbon 10 % dans un solvant inerte tel que le méthanol, méthanol, acétate d'éthyle ou un mélange de ces solvants, à une température de 18-25° C, pendant 2 à 8 heures pour conduire à la dianiline (19). Le dérivé (19) est ensuite traité par un isothiocyanate en présence d'un agent de couplage supporté sur une résine ou non tel que le diisopropylcarbodiimide ou dicyclohexylcarbodiimide ou la résine *N*-cyclohexylcarbodiimide *N*-méthyl polystyrène dans un solvant inerte tel que le tétrahydrofuramie, chlorure de méthylène, ou chloroforme à une température de 20-70° C pendant 2 à 72 heures pour conduire au dérivé (20). Alternativement, le dérivé (19) peut être traité par un isothiocyanate dans un solvant inerte tel que le tétrahydrofuranne, chlorure de méthylène, chloroforme ou éthanol à une température de 20-80° C pendant 1-16 heures puis la thiourée résultante peut être traitée par de l'oxyde de mercure (II) jaune en présence d'une quantité catalytique de soufre dans un solvant polaire tel que le méthanol ou l'éthanol pendant 2 à 24 heures à une température de 20-80° C pour conduire à (20). Le dérivé (20) peut réagir avec un chlorure d'acide dans un solvant aprotique tel que le dichlorométhane ou tétrahydrofuranne en présence d'une base telle qu'une amine tertiaire supportée ou non sur une résine, telle que la triéthylamine ou la résine morpholino-méthylpolystyrène à une température de 0-40° C pendant 0,3-2 heures pour conduire à l'amide (21). L'aniline (20) peut également réagir avec un isothiocyanate dans un solvant aprotique tel que le tétrahydrofuranne à une température de 20-70° C pendant 5-48 heures pour conduire à la thiourée (22).

### Exemple E1 : Chlorhydrate de N-[2-[(4-acetylphényl)amino]-3-(3-pipéridin-1-ylpropyl)-3H-imidazo[4,5-b]pyridin-5-yl]-N-butylbutanamide

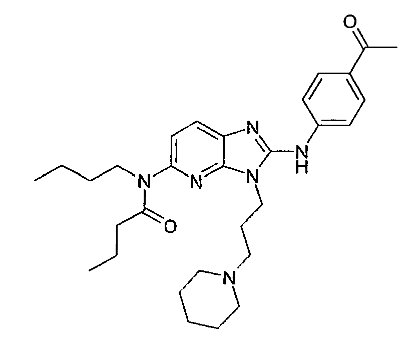

### Etape 1 : N⁶-butyl-3-nitro-N²-(3-pipéridin-1-ylpropyl)pyridine-2,6-diamine

A une solution de 6-chloro-3-nitro-*N*-(3-pipéridin-1-ylpropyl)pyridin-2-amine (1 g, 1 éq ; préparé selon l'exemple A2) dans l'acétonitrile (80 ml) sont successivement additionnés le carbonate de potassium (930 mg, 2 éq) et une solution de 1-butylamine (300 mg, 1,2 éq) dans l'acétonitrile (2 ml). Le mélange est chauffé a reflux pendant 15 heures puis refroidi à température ambiante et concentré sous pression réduite à 40° C. Le résidu est repris dans le dichlorométhane (200 ml) et l'eau (90 ml). Après décantation et extractions, les phases organiques combinées sont lavées avec de la saumure, séchées sur Na₂SO₄ puis concentrées sous pression réduite à 40° C. La purification du résidu obtenu, par chromatographie éclair sur gel de silice (éluant : heptane/acétate d'éthyle 9 :1 à acétate d'éthyle 100 %), donne le composé attendu (1,1 g; 98 % rendement).

SM/CL : MM calculée = 335,4 ; m/z = 336,4 (MH+)

RMN (¹H, 400 MHz, DMSO-*d₆*) : δ 0,89 (t, 3H), 1,34 (m, 4H), 1,48 (m, 6H), 1,72 (m, 2H), 2,29 (m, 6H), 3,33 (m, 2H), 3,53 (m, 2H), 5,90 (d, 1H), 7,91 (d, 1H), 8,06 (t, 1H), 9,12 (t, 1H).

### Etape 2 : 1-(4-{[5-(butylamino)-3-(3-pipéridin-1-ylpropyl)-3H-imidazo[4,5-b]pyridin-2-yl]amino}phényl)éthanone

Dans un autoclave sont additionnés le *N*⁶-butyl-3-nitro-*N*²-(3-pipéridin-1-ylpropyl)pyridine-2,6-diamine (500 mg) en solution dans un mélange d'acétate d'éthyle/ éthanol 3 :1 (10 ml), et le palladium sur charbon 10 % (50 mg). Après 3 heures d'agitation sous atmosphère d'hydrogène (3 bars) à une température d'environ 20° C, le mélange est filtré sur célite dans un ballon contenant une solution de 4-acétylphényl-isothiocyanate (270 mg, 1 éq) dans le tétrahydrofuranne (10 ml). Au filtrat ainsi obtenu est additionnée la résine *N*-cyclohexylcarbodiimide-*N*-méthylpolystyrène (acquise auprès de Novabiochem ; charge 1,9 mmol/g ; 2,63 g, 3 éq). Le mélange est chauffé à reflux pendant 15 heures, refroidi à température ambiante puis filtré sur fritté et le filtrat est concentré sous pression réduite à 40° C. La purification du résidu par chromatographie éclair sur gel de silice (éluant : dichlorométhane 100% à dichlorométhane/méthanol 9 :1) donne le composé attendu (230 mg ; 34 % rendement).

SM/CL : MM calculée = 448,6 ; m/z = 449,3 (MH+)

### Etape 3 : Chlorhydrate de N-[2-[(4-acétylphenyl)amino]-3-(3-piperidin-1-ylpropyl)-3H-imidazo[4,5-b]pyridin-5-yl]-N-butylbutanamide

A une solution de 1-(4-{[5-(butylamino)-3-(3-pipéridin-1-ylpropyl)-3*H* imidazo[4,5-*b*]pyridin-2-yl]amino}phényl)éthanone (54 mg) dans le dichlorométhane anhydre (1 ml) sont successivement additionnés de la résine morpholinométhyl (acquise auprès de Novabiochem, loading = 3,5 mmol/g ; 69 mg, 2 éq) et le chlorure de butyryl (17 mg). Après 30 minutes d'agitation à température ambiante, de la résine aminométhylpolystyrène résine est additionnée pour piéger l'excès de chlorure d'acide. Après 2 heures d'agitation à température ambiante, le mélange est filtré et concentré sous pression réduite à 40° C. Le sel de chlorhydrate correspondant est formé par addition d'une solution d'acide chlorhydrique 1N dans l'éther éthylique. Le précipité obtenu est filtré et séché pour donner le composé attendu (68 mg).

SM/CL : MM calculée = 518,7 ; m/z = 519,4 (MH+).

Selon le schéma réactionnel E et selon la procédure décrite pour le chlorhydrate de N-[2-[(4-acétylphényl)amino]-3-(3-piperidin-1-ylpropyl)-3*H*-imidazo[4,5-*b*]pyridin-5-yl]-*N*-butylbutanamide, les composés suivants peuvent être préparés : 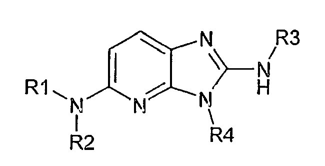 dans lesquels R₁R₂N représente l'un des radicaux ci-après : 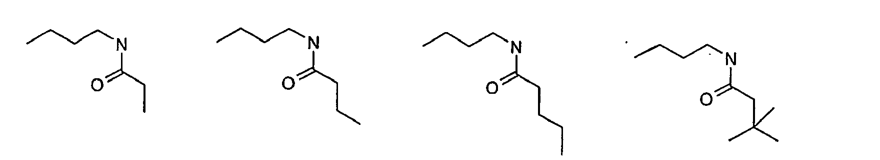 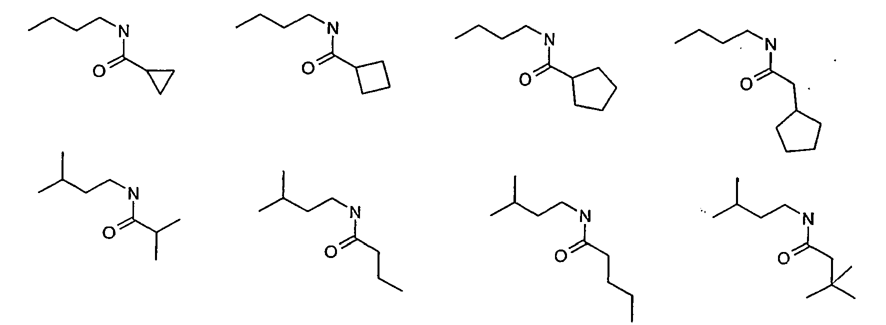 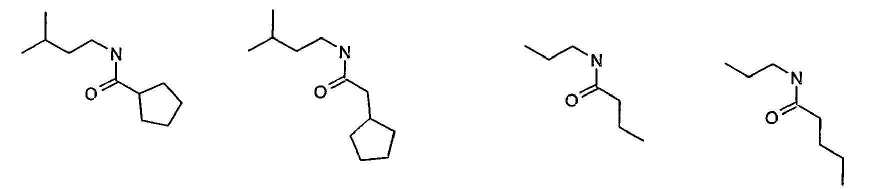 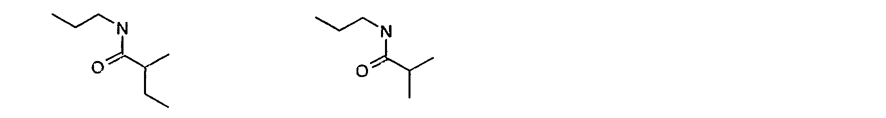 R₃ représente l'un des radicaux ci-après : 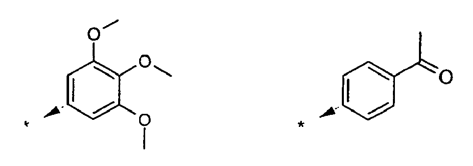 et R₄ représente l'un des radicaux ci-après :

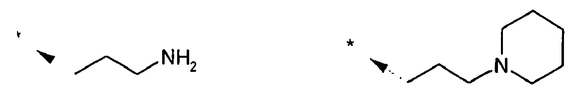

La présente invention a également pour objet un procédé de préparation d'un composé de formule (I) tel que défini ci-dessus, caractérisé en ce que l'on traite le composé de formule générale : 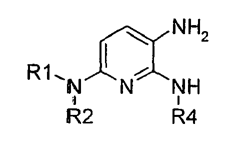 dans laquelle R₁, R₂, R₄ ont la signification indiquée ci-dessus, par un isothiocyanate de formule générale R₃N=C=S dans lequel R₃ a la signification ci-dessus, en présence d'un agent de couplage ou d'oxyde de mercure (II) jaune en présence de soufre, pendant une durée de 3 à 48 heures, dans un solvant protique ou aprotique, à une température de 50 à 80° C.

L'agent de couplage peut être supporté tel que la résine N-méthylcyclohexylcarbodiimide N-méthyl polystyrène ou non supporté tel que diisopropylcarbodiimide, diéthylcarbodiimide ou dicyclohexylcarbodiimide. On peut utiliser un solvant protique tel que le méthanol ou l'éthanol ou aprotique tel que le tétrahydrofuranne ou l'acétonitrile.

L'invention a également pour objet un composé de formule générale (II) 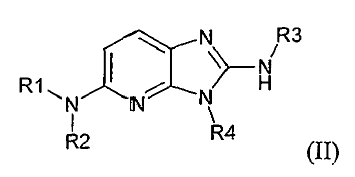 sous forme racémique, d'énantiomère ou toutes combinaisons de ces formes et dans laquelle :
R₁ et R₂ représentent, indépendamment, l'atome d'hydrogène, un radical (C₁-C₈)alkyle éventuellement substitué par hydroxy ; (C₂-C₆)alkénylealkényle, un bicycloalkyle ou un radical de formule -(CH₂)ₙ-X₁ ou -X-(CH₂)_{n'}-X'₁ ;
X représente -C(O)- ou -C(S)-NH- ;
X₁ représente un radical (C₁-C₆)alkoxy, (C₃-C₇)cycloalkyle, adamantyle, hétérocycloalkyle, aryle ou hétéroaryle,
   les radicaux (C₃-C₇)cycloalkyle, hétérocycloalkyle, aryle et hétéroaryle étant éventuellement substitués par un ou plusieurs substituants identiques ou différents choisis parmi : -(CH₂)ₙ₁-V₁-Y₁, halo , nitro et cyano ;
   V₁ représente -O-, -S- ou une liaison covalente ;
   Y₁ représente un radical (C₁-C₆)alkyle éventuellement substitué par un ou plusieurs radicaux halo identiques ou différents, ou aryle ;
   n et n' représentent un entier de 0 à 6 et n₁ un entier de 0 à 2 (étant entendu que lorsque n est égal à 0, alors X₁ ne représente pas le radical alkoxy) ;
X'₁ représente l'atome d'hydrogène ; un radical (C₁-C₆)alkyle éventuellement substitué par un ou plusieurs radicaux halo identiques ou différents ; (C₃-C₇)cycloalkyle ; ou aryle éventuellement substitué par un ou plusieurs substituants identiques ou différents choisi parmi : halo, nitro, cyano, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : hydroxy, (C₁-C₆)alkyle, (C₁-C₆)hydroxyalkyle, (C₁-C₆)alkoxy-carbonyle, -(CH₂)_{n"}-A, -C(O)-NV_{1'}Y₁', et hétérocycloalkyle ; ou bien R₁ et R₂ forment ensemble un radical de formule : 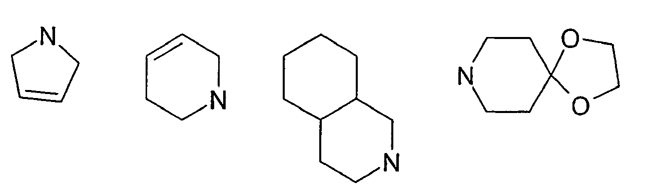 V₁' et Y₁' représentent, indépendamment, l'atome d'hydrogène ou un radical (C₁-C₆)alkyle;
A-représente un radical aryle éventuellement substitué par un ou plusieurs substituants identiques ou différents choisi parmi : halo, nitro, cyano, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
n" représente un entier de 0 à 2 ;
R₃ représente -(CH₂)ₚ-Z₃ ou -C(O)-Z'₃
Z₃ représente un radical (C₁-C₆)alkyle, (C₂-C₆)alkénylealkényle, (C₁-C₆)alkoxy, (C₁-C₆)alkoxy-carbonyle, (C₁-C₆)alkyl-amino-carbonyle, (C₃-C₇)cycloalkyle, hétérocycloalkyle, aryle ou hétéroaryle,
les radicaux (C₃-C₇) cycloalkyle et hétérocycloalkyle étant éventuellement substitués par un ou plusieurs radicaux (C₁-C₆)alkyle identiques ou différents,
le radical aryle étant éventuellement substitué par un ou plusieurs substituants identiques ou différents choisis parmi : halo, nitro, azido ou -(CH₂)_{p'}-V₃-Y₃ ;
V₃ représente -O-, -S-, -C(O)-, -C(O)-O-, -NH-C(O)-, -C(O)-NR'₃-, -NH-C(O)-NR'₃-, -NH-C(O)-NR'₃-O- (pour illustrer une préférence qui n'est pas exemplifiée) ou une liaison covalente ;
Y₃ représente l'atome d'hydrogène ou un radical (C₁-C₆)alkyle éventuellement substitué par un ou plusieurs radicaux halo identiques ou différents ;
   ou bien Z₃ représente un radical de formule 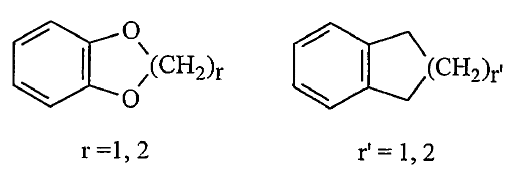
Z'₃ représente un radical aryle éventuellement substitué par un ou plusieurs substituants identiques ou différents choisis parmi : halo, nitro et -(CH₂)_{p"}-V'₃-Y'₃ ;
V'₃ représente -O-, -C(O)-, -C(O)-O-, -C(O)-NR'₃-, -NH-C(O)-, -NH-C(O)-NR'₃- ou une liaison covalente ;
Y'₃ représente l'atome d'hydrogène ou un radical (C₁-C₆)alkyle éventuellement substitué par un ou plusieurs radicaux halo identiques ou différents ;
R'₃ représente l'atome d'hydrogène, un radical (C₁-C₆)alkyle ou (C₁-C₆)alkoxy ;
p, p' et p" représentent, indépendamment, un entier de 0 à 4 ;
R₄ représente un radical de formule-(CH₂)ₛ-R'₄ ;
R'₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle, (C₂-C₆)alkenyle alkényle ; (C₃-C₇)cycloalkyle éventuellement substitué par un ou plusieurs substituants (C₁-C₆)alkyle identiques ou différents ; cyclohexène ; hétéroaryle, aryle éventuellement substitué par un ou plusieurs radicaux identiques ou différents choisis parmi : -(CH₂)_{s"}-V₄-Y₄, halo et nitro ;
   V₄ représente -O-, -S-, -NH-C(O)-, -NV₄'- ou une liaison covalente ;
   Y₄ représente un atome d'hydrogène ou un radical (C₁-C₆)alkyle éventuellement substitué par un ou plusieurs radicaux halo identiques ou différents ;
   V₄' représente un atome d'hydrogène ou un (C₁-C₆)alkyle ;
   s" représente un entier de 0 à 4 ;
   ou bien Z₄ représente un radical de fomule 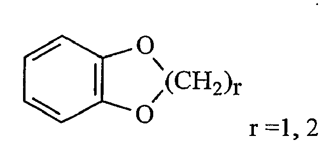
s et s' représentent, indépendamment, un entier de 0 à 6 ;
ou un sel pharmaceutiquement acceptable de ce dernier.

De préférence, l'invention concerne des composés de formule II telle que définie ci-dessus et dans laquelle R₁ et R₂ représentent, indépendamment, l'atome d'hydrogène, un radical (C₁-C₈)alkyle, un bicycloalkyle ou un radical de formule -(CH₂)ₙ-X₁ ou -X-(CH₂)_{n'}-X'₁ ;
X représente -C(O)- ou -C(S)-NH- ;
X₁ représente un radical (C₁-C₆)alkoxy, (C₃-C₇)cycloalkyle ou hétéroaryle ;
X'₁ représente l'atome d'hydrogène, un radical (C₁-C₆)alkyle éventuellement substitué par un ou plusieurs radicaux halo identiques ou différents, (C₃-C₇)cycloalkyle ou aryle ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : (C₁-C₆)alkyle, (C₁-C₆)alkoxy-carbonyle et -(CH₂)_{n"}-A ;
A représente un radical aryle éventuellement substitué par un ou plusieurs substituants identiques ou différents choisi parmi : halo et (C₁-C₆)alkyle ;
n" représente un entier de 0 à 3 ;
R₃ représente -(CH₂)ₚ-Z₃ ou -C(O)-Z'₃
Z₃ aryle éventuellement substitué par un ou plusieurs substituants identiques ou différents choisis parmi : halo, nitro et -(CH₂)_{p'}-V₃-Y₃;
V₃ représente -O-, -S-, -C(O)-, -C(O)-O-, -NH-C(O)-, -C(O)-NR'₃- ou une liaison covalente ;
Y₃ représente l'atome d'hydrogène ou un radical (C₁-C₆)alkyle éventuellement substitué par un ou plusieurs radicaux halo identiques ou différents ;
   ou bien Z₃ représente un radical de formule 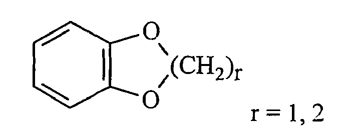
Z'₃ représente un radical aryle éventuellement substitué par un ou plusieurs substituants identiques ou différents choisis parmi : halo et -(CH₂)_{p"}-V'₃-Y'₃ ;
V'₃ représente -O- ou une liaison covalente ;
Y'₃ représente l'atome d'hydrogène ou un radical (C₁-C₆)alkyle ;
R'₃ représente l'atome d'hydrogène ;
p, p' et p" représentent, indépendamment, un entier de 0 à 4 ;
R₄ représente un radical de formule-(CH₂)ₛ-R'₄
R'₄ représente un hétérocycloalkyle contenant au moins un atome d'azote ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle ou aryle éventuellement substitué par un ou plusieurs radicaux identiques ou différents choisis parmi : -(CH₂)_{s"}-V₄-Y₄ ;
   V₄ représente -O- ;
   Y₄ représente un radical (C₁-C₆)alkyle éventuellement substitué par un ou plusieurs radicaux halo identiques ou différents ;
   s" représente un entier de 0 à 4 ;
s et s' représentent, indépendamment, un entier de 0 à 6 ; ou un sel pharmaceutiquement acceptable de ce dernier,
et plus particulièrement
le radical cycloalkyle est choisi parmi cyclopropyle et cyclohexyle ; et/ou
le radical bicycloalkyle est le bicylo[2,2,1]heptane, et/ou
le radical hétéroaryle est le radical furyle, et/ou
le radical aryle est le radical phényle, et/ou
l'hétérobicycloalkyle est le 7-aza-bicyclo[2,2,1]heptane, et/ou
l'hétérocycloalkyle est choisi parmi pipéridine et pipérazine.

De manière très préférentielle également, l'invention concerne des composés de formule II telle que définie ci-dessus et dans laquelle
R₁ et R₂ représentent, indépendamment, l'atome d'hydrogème, un radical (C₁-C₈)alkyle ou un radical de formule -(CH₂)ₙ-X₁ ;
X₁ représente un radical (C₃-C₇)cycloalkyle et plus particulièrement cyclopropyle ou cyclohexyle ;
ou bien R₁ et R₂ forment ensemble, avec l'atome d'azote auquel ils sont rattachés, un hétérocycloalkyle éventuellement substitué par un ou plusieurs substituants (C₁-C₆)alkyle identiques ou différents ; et/ou
R₃ représente -(CH₂)ₚ-Z₃
Z₃ représente un radical (C₁-C₆)alkoxy-carbonyle, (C₁-C₆)alkyl-amino-carbonyle, ou phényle éventuellement substitué par un ou plusieurs substituants identiques ou différents choisis parmi : nitro et -(CH₂)_{p'}-V₃-Y₃ ;
V₃ représente -O-, -C(O)-, -C(O)-O-, -NH-C(O)-, -C(O)-NR'₃-,- ou -NH-C(O)-NR'₃- ou -NH-C(O)-NR'₃-O- ;
Y₃ représente l'atome d'hydrogène ou un radical (C₁-C₆)alkyle éventuellement substitué par un ou plusieurs radicaux halo identiques ou différents ;
R'₃ représente l'atome d'hydrogène ;
p et p' représentent, indépendamment, un entier de 0 à 4 ; et/ou
R₄ représente un radical de formule-(CH₂)ₛ-R'₄
R'₄ représente un hétérocycloalkyle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle, (C₃-C₇)cycloalkyle ;
s et s' représentent, indépendamment, un entier de 0 à 6 ;
et plus particulièrement le cycloalkyle est choisi parmi cyclopropyle et cyclohexyle, et/ou l'hétérocycloalkyle est choisi parmi : pyrrolidine, pipéridine, morpholine, pipérazine ; ou un sel pharmaceutiquement acceptable de ce dernier.

Les composés 1 et II de la présente invention possèdent d'intéressantes propriétés pharmacologiques. C'est ainsi que l'on a découvert que les composés 1 de la présente invention possèdent une bonne affinité pour certains sous-types de récepteurs des mélanocortines, en particulier des récepteurs MC4.

Les composés de la présente invention peuvent ainsi être utilisés dans différentes applications thérapeutiques. Ils peuvent avantageusement être utilisés pour traiter les états pathologiques ou les maladies dans lesquels un ou plusieurs récepteurs des mélanocortines sont impliqués tels que les états inflammatoires, les désordres pondéraux (l'obésité, la cachexie, l'anorexie), les désordres de l'activité sexuelle (les troubles de l'érection), la douleur, mais également les troubles mentaux (l'anxiété, la dépression), les toxicomanies, les maladies de la peau (l'acné, les dermatoses, les mélanomes). On trouvera ci-après, dans la partie expérimentale, une illustration des propriétés pharmacologiques des composés de l'invention.

La présente demande a également pour objet des compositions pharmaceutiques contenant, à titre de principe actif, au moins un produit de formule I telle que définie ci-dessus, ainsi que les sels pharmaceutiquement acceptables dudit produit de formule I, en association avec un support pharmaceutiquement acceptable.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33,201-217.

La présente demande a également pour objet l'utilisation des composés selon la présente invention, pour la préparation d'un médicament pour le traitement des désordres pondéraux tels que l'obésité, la cachexie et plus particulièrement la cachexie des pathologies cancéreuses, cachexie du sida, cachexie de la personne âgée, cachexie cardiaque, cachexie rénale, cachexie de l'arthrite rhumatoïde, et l'anorexie, le traitement de la douleur et plus particulièrement la douleur neuropathique, le traitement des troubles mentaux tels que l'anxiété et la dépression, le traitement des désordres de l'activité sexuelle tels que les troubles de l'érection.

La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale :

Les composés selon l'invention obtenus selon les procédures des exemples A, B, C, D et E précédemment décrites, sont rassemblés dans le tableau ci-dessous.

Les composés sont caractérisés par leur temps de rétention (tr) et leur pic moléculaire déterminé par spectrométrie de masse (MH+).

Pour la spectrométrie de masse, un spectromètre de masse simple quadripôle (Micromass, modèle Platform) équipé d'une source *electrospray* est utilisé avec une résolution de 0,8 Da à 50 % de vallée. Un calibrage est effectué mensuellement entre les masses 80 et 1000 Da à l'aide d'un mélange calibrant d'iodure de sodium et d'iodure de rubidium en solution dans un mélange isopropanol / eau (1/1 Vol.)

Pour la chromatographie liquide, un système Waters incluant un dégazeur en ligne, une pompe quaternaire Waters 600, un injecteur plaque Gilson 233 et un détecteur UV Waters PAD 996, est utilisé.

Les conditions d'élution employées sont les suivantes :

| | | |
|---|---|---|
| Eluant | A | eau + 0,04 % acide trifluoroacétique |
| | B | acétonitrile |

| T (min) | A % | B % |
|---|---|---|
| 1 | 95 | 5 |
| 8,5 | 5 | 95 |
| 10,5 | 5 | 95 |
| 10,6 | 95 | 5 |
| 14,9 | 95 | 5 |
| 15,0 | 95 | 5 |

Débit : 1 ml/min
Infection : 10 µl
Colonne : Uptisphere ODS 3 µm 755*4,6 mm i.d

Ces exemples sont présentés pour illustrer les procédures ci-dessus et ne doivent en aucun cas être considérés comme une limite à la portée de l'invention.

| **Exemples** | **Structures moléculaires** | **[M+H]+** | **tr (min)** |
|---|---|---|---|
| 1 | 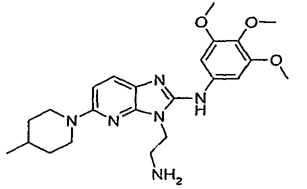 | 441,2 | 7,5 |
| 2 | 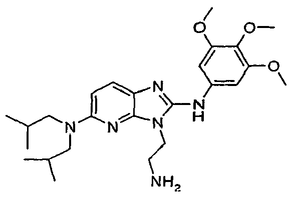 | 471,3 | 8,0 |
| 3 | 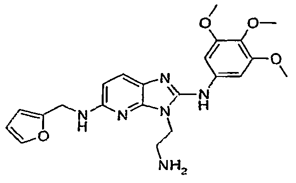 | 439,2 | 7,3 |
| 4 | 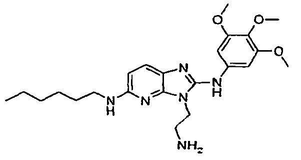 | 443,2 | 7,8 |
| 5 | 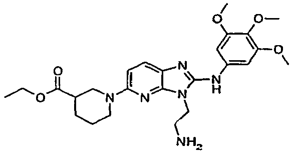 | 499,2 | 7,5 |
| 6 | 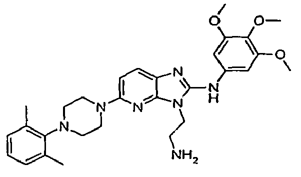 | 532,3 | 8,2 |
| 7 | 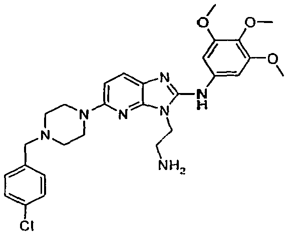 | 552,2 | 7,2 |
| 8 | 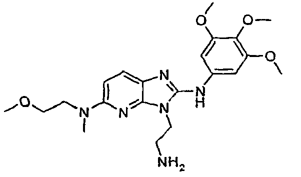 | 417,1 | 7,0 |
| 9 | 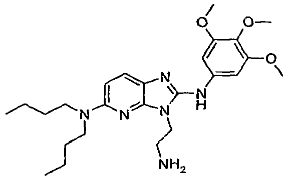 | 471,3 | 8,0 |
| 10 | 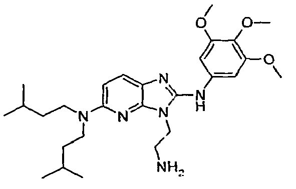 | 499,3 | 8,4 |
| 11 | 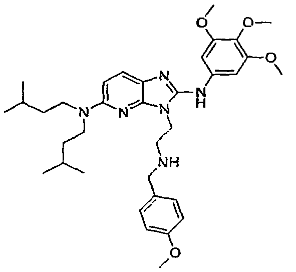 | 619,4 | 8,9 |
| 12 | 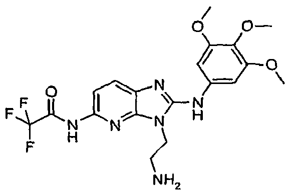 | 455,1 | 7,5 |
| 13 | 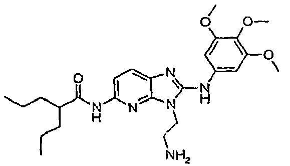 | 485,2 | 7,8 |
| 14 | 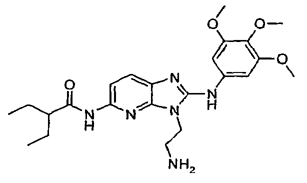 | 457,2 | 7,5 |
| 15 | 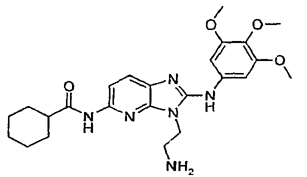 | 469,2 | 7,6 |
| 16 | 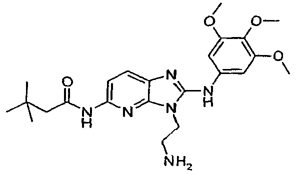 | 457,3 | 7,5 |
| 17 | 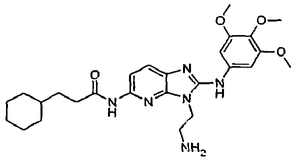 | 497,3 | 8,0 |
| 18 | 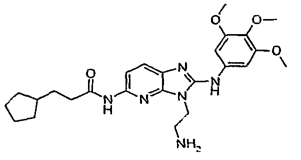 | 483,3 | 7,8 |
| 19 | 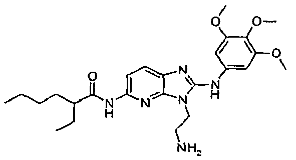 | 485,3 | 7,8 |
| 20 | 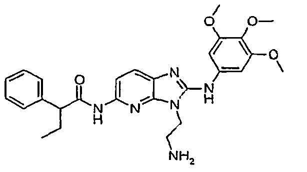 | 505,2 | 7,8 |
| 21 | 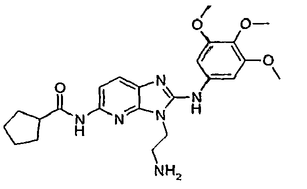 | 455,2 | 7,5 |
| 22 | 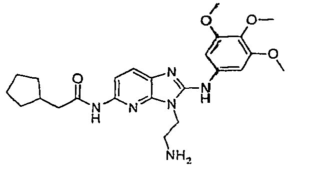 | 469,2 | 7,6 |
| 23 | 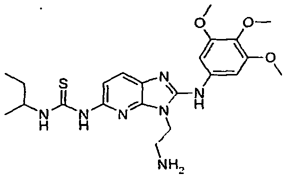 | 474,2 | 7,6 |
| 24 | 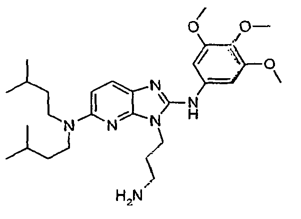 | 513,3 | 8,5 |
| 25 | 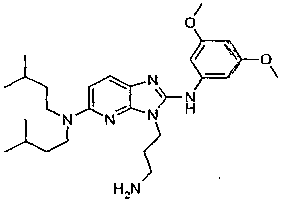 | 483,3 | 8,6 |
| 26 | 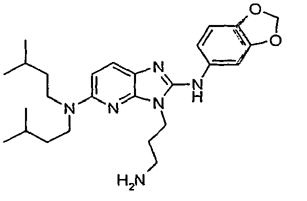 | 467,3 | 8,4 |
| 27 | 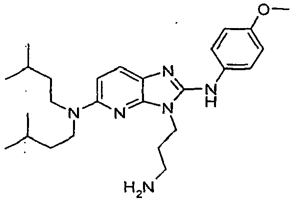 | 453,3 | 8,5 |
| 28 | 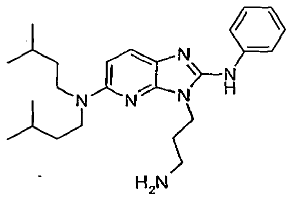 | 423,3 | 8,5 |
| 29 | 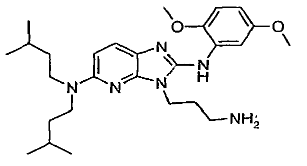 | 483,3 | 8,6 |
| 30 | 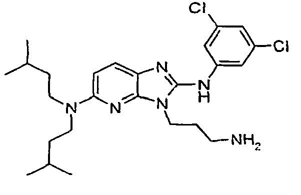 | 491,2 | 9,4 |
| 31 | 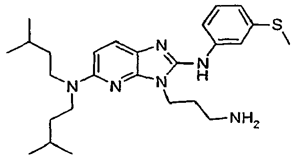 | 469,3 | 8,7 |
| 32 | 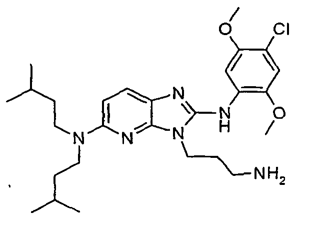 | 517,3 | 8,7 |
| 33 | 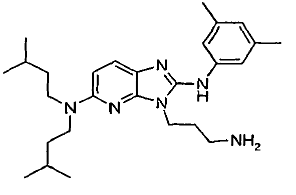 | 453,3 | 8,5 |
| 34 | 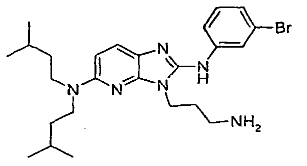 | 501,2 | 8,8 |
| 35 | 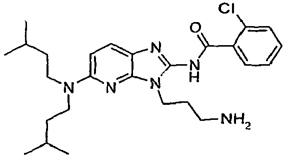 | 485,2 | 10,2 |
| 36 | 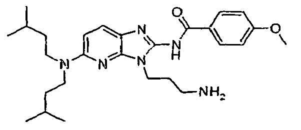 | 465,3 | 10,5 |
| 37 | 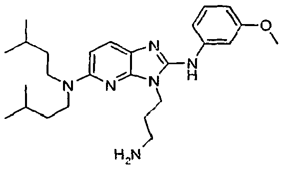 | 453,2 | 8,5 |
| 38 | 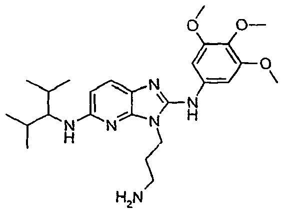 | 471,3 | 7,3 |
| 39 | 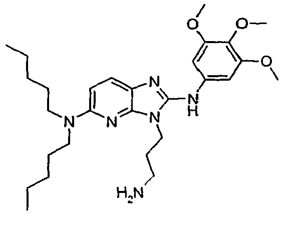 | 513,3 | 7,8 |
| 40 | 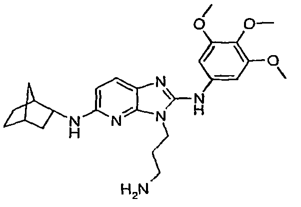 | 467,3 | 7,2 |
| 41 | 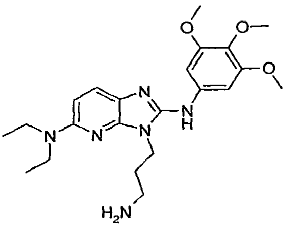 | 429,2 | 7,0 |
| 42 | 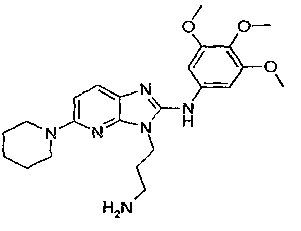 | 441,2 | 7,0 |
| 43 | 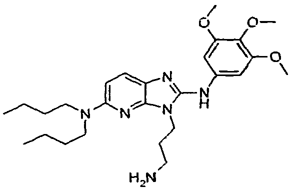 | 485,3 | 7,5 |
| 44 | 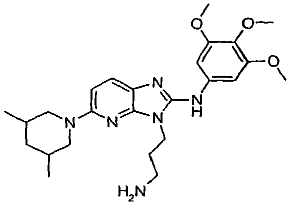 | 469,3 | 7,3 |
| 45 | 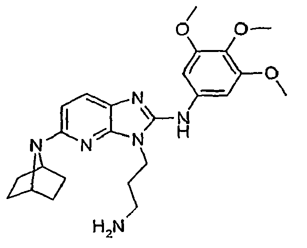 | 453,2 | 7,0 |
| 46 | 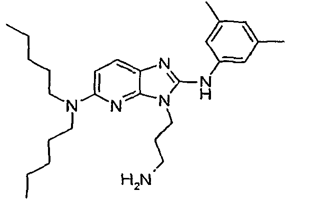 | 451,3 | 8,0 |
| 47 | 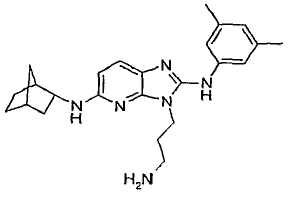 | 405,2 | 7,4 |
| 48 | 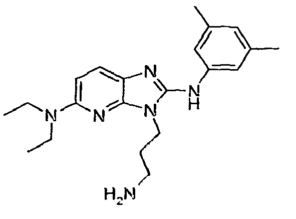 | 367,2 | 7,2 |
| 49 | 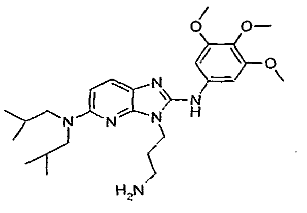 | 485,2 | 7,5 |
| 50 | 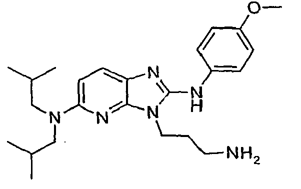 | 425,3 | 8,2 |
| 51 | 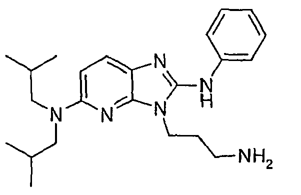 | 395,3 | 8,0 |
| 52 | 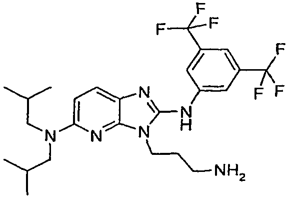 | 531,2 | 9,6 |
| 53 | 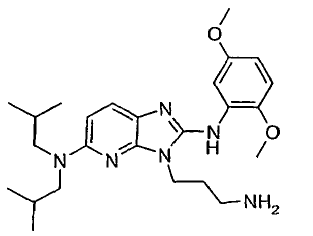 | 455,3 | 8,2 |
| 54 | 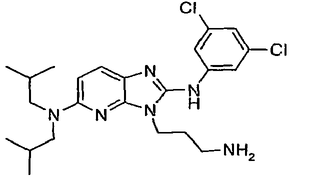 | 463,2 | 8,8 |
| 55 | 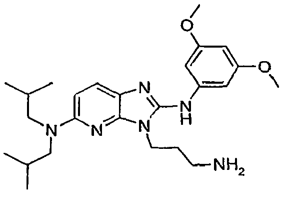 | 455,3 | 8,2 |
| 56 | 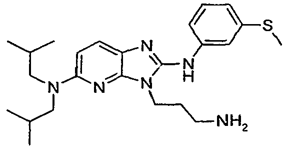 | 441,3 | 8,2 |
| 57 | 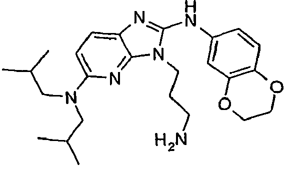 | 453,3 | 8,1 |
| 58 | 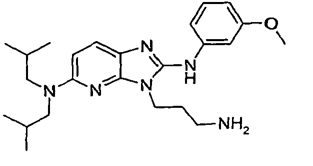 | 425,3 | 8,1 |
| 59 | 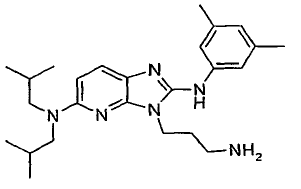 | 423,3 | 8,3 |
| 60 | 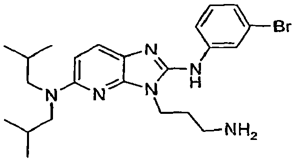 | 473,2 | 8,3 |
| 61 | 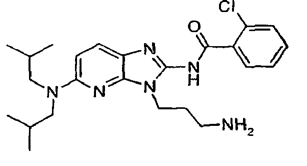 | 457,2 | 9,4 |
| 62 | 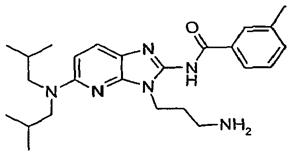 | 437,2 | 9,8 |
| 63 | 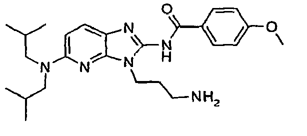 | 453,3 | 9,6 |
| 64 | 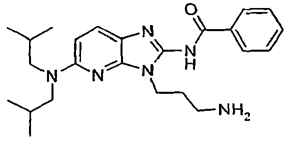 | 423,2 | 9,6 |
| 65 | 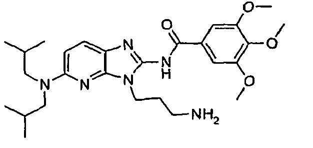 | 513,3 | 9,6 |
| 66 | 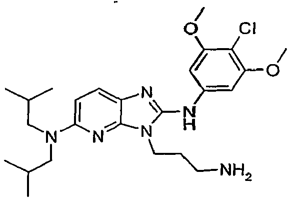 | 489,2 | 8,4 |
| 67 | 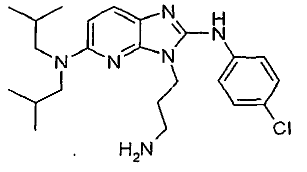 | 429,2 | 8,2 |
| 68 | 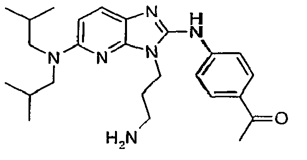 | 437,3 | 8,1 |
| 69 | 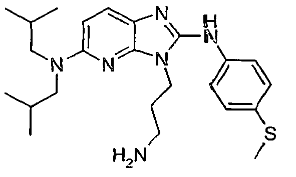 | 441,2 | 8,2 |
| 70 | 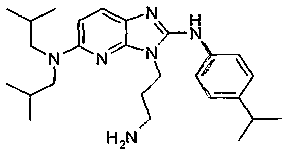 | 437,2 | 8,4 |
| 71 | 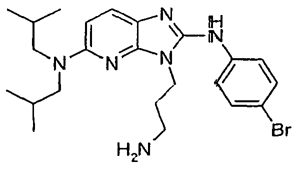 | 473,1 | 8,3 |
| 72 | 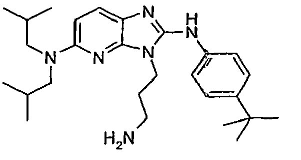 | 451,2 | 8,5 |
| 73 | 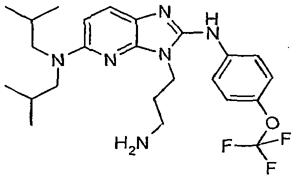 | 479,2 | 8,4 |
| 74 | 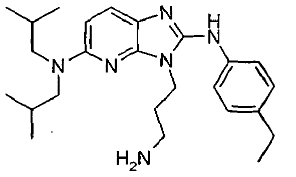 | 423,3 | 8,3 |
| 75 | 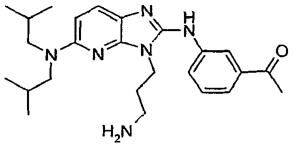 | 437,2 | 8,0 |
| 76 | 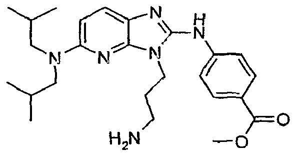 | 453,2 | 8,2 |
| 77 | 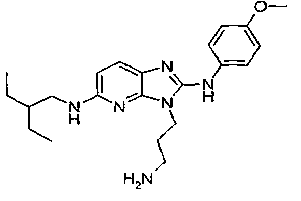 | 397,1 | 7,8 |
| 78 | 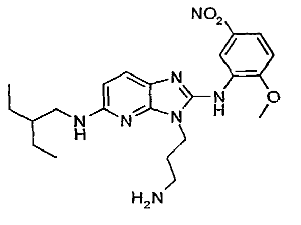 | 442,1 | 7,9 |
| 79 | 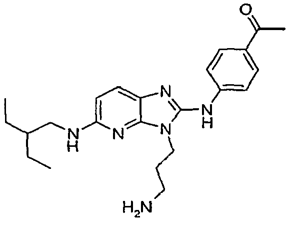 | 409,0 | 7,8 |
| 80 | 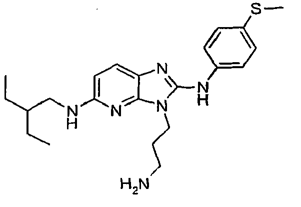 | 413,0 | 7,9 |
| 81 | 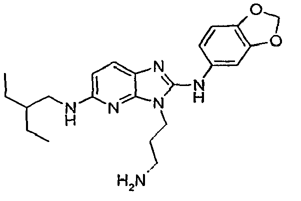 | 411,0 | 7,8 |
| 82 | 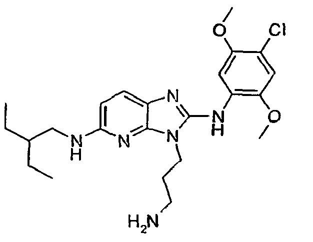 | 461,0 | 7,9 |
| 83 | 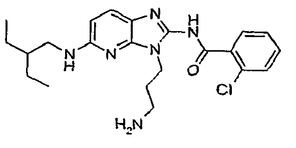 | 429,0 | 8,7 |
| 84 | 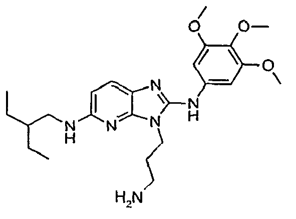 | 457,1 | 7,8 |
| 85 | 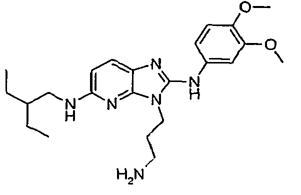 | 427,1 | 7,7 |
| 86 | 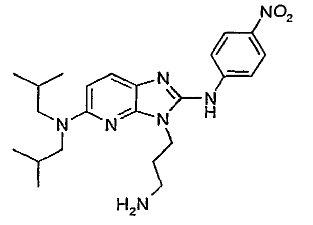 | 440,0 | 8,7 |
| 87 | 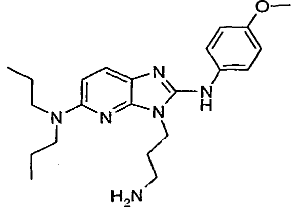 | 397,1 | 7,8 |
| 88 | 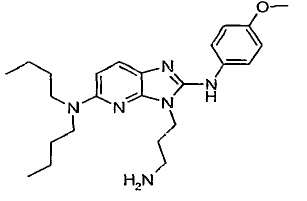 | 425,1 | 8,2 |
| 89 | 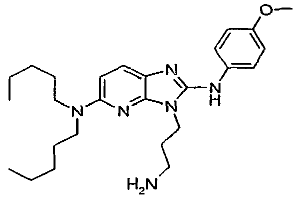 | 453,1 | 8,5 |
| 90 | 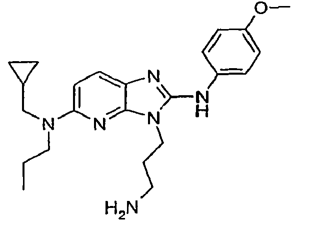 | 409,2 | 7,8 |
| 91 | 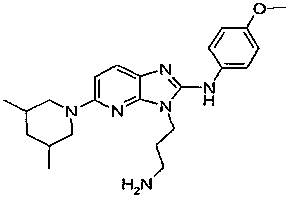 | 409,2 | 7,8 |
| 92 | 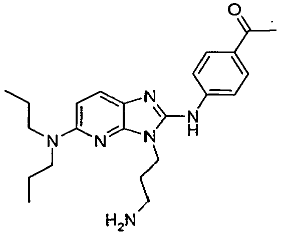 | 409,0 | 7,8 |
| 93 | 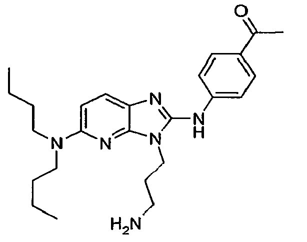 | 437,0 | 8,1 |
| 94 | 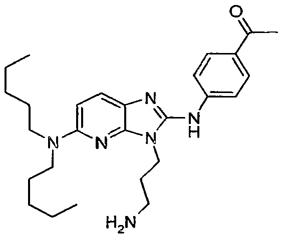 | 465,2 | 8,5 |
| 95 | 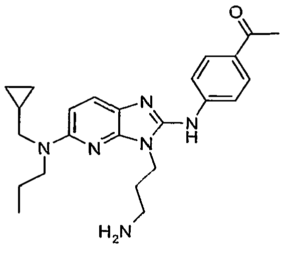 | 421,0 | 7,8 |
| 96 | 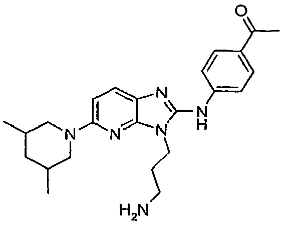 | 421,2 | 7,8 |
| 97 | 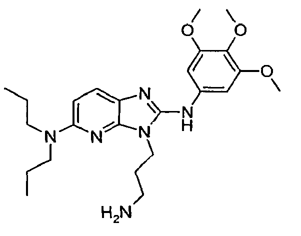 | 457,1 | 7,8 |
| 98 | 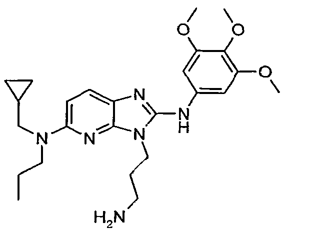 | 469,1 | 7,8 |
| 99 | 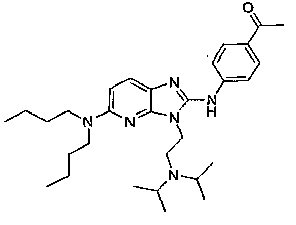 | 507,3 | 9,1 |
| 100 | 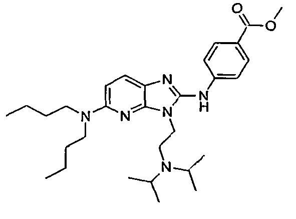 | 523,3 | 9,3 |
| 101 | 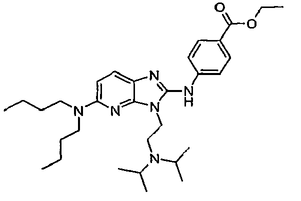 | 537,3 | 9,5 |
| 102 | 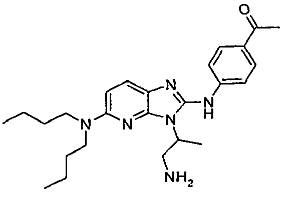 | 437,2 | 8,4 |
| 103 | 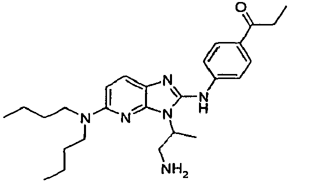 | 451,3 | 8,6 |
| 104 | 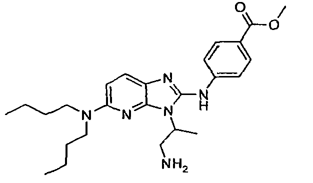 | 453,3 | 8,6 |
| 105 | 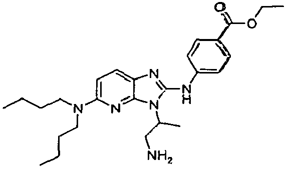 | 467,3 | 8,8 |
| 106 | 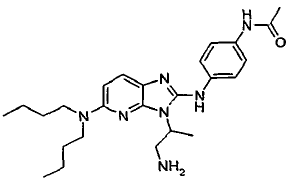 | 452,3 | 8,0 |
| 107 | 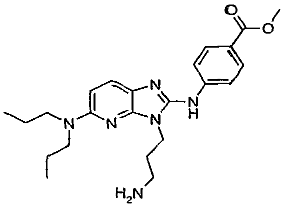 | 425,2 | 7,9 |
| 108 | 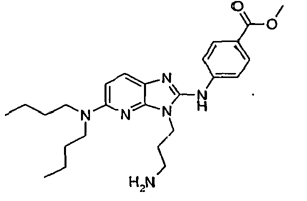 | 453,3 | 8,2 |
| 109 | 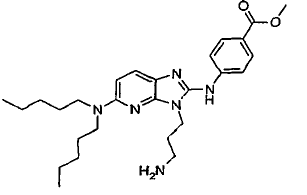 | 481,3 | 8,7 |
| 110 | 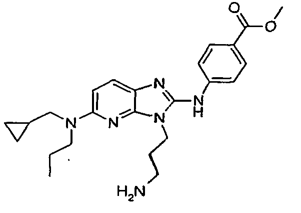 | 437,2 | 7,9 |
| 111 | 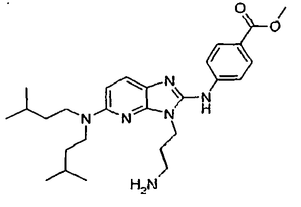 | 481,3 | 8,6 |
| 112 | 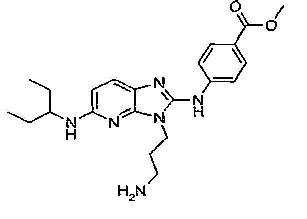 | 411,2 | 7,7 |
| 113 | 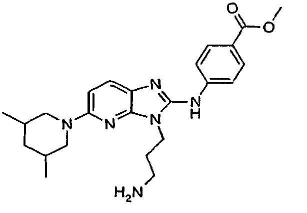 | 437,2 | 8,0 |
| 114 | 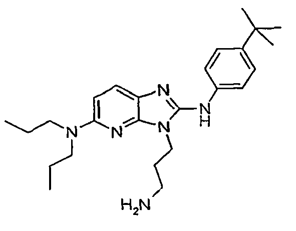 | 423,2 | 8,3 |
| 115 | 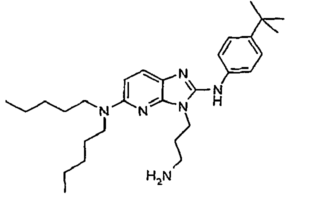 | 479,3 | 9,0 |
| 116 | 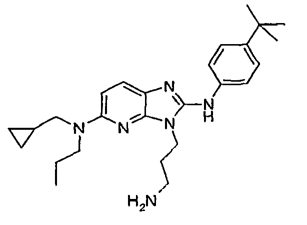 | 435,2 | 8,3 |
| 117 | 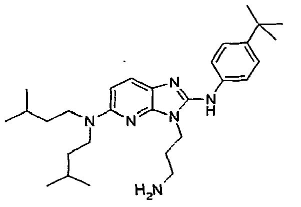 | 479,3 | 8,9 |
| 118 | 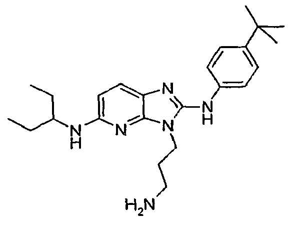 | 409,2 | 8,1 |
| 119 | 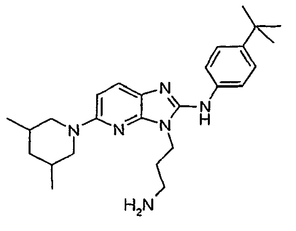 | 435,2 | 8,3 |
| 120 | 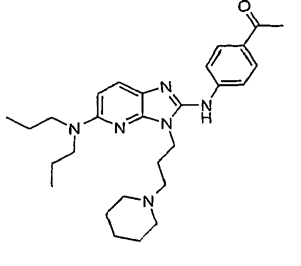 | 477,2 | 8,1 |
| 121 | 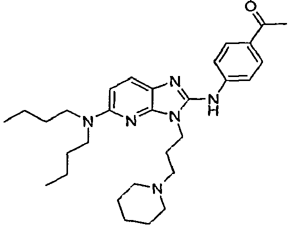 | 505,2 | 8,5 |
| 122 | 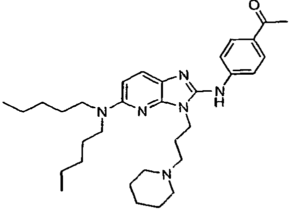 | 533,3 | 9,0 |
| 123 | 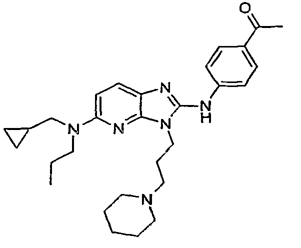 | 489,2 | 8,1 |
| 124 | 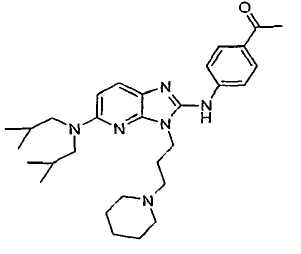 | 505,3 | 8,4 |
| 125 | 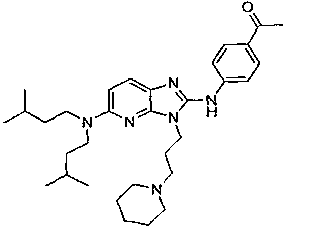 | 533,3 | 8,9 |
| 126 | 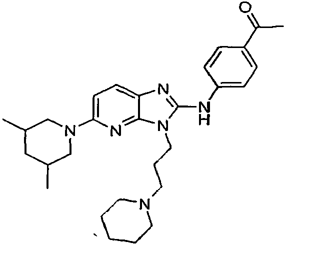 | 489,2 | 8,2 |
| 127 | 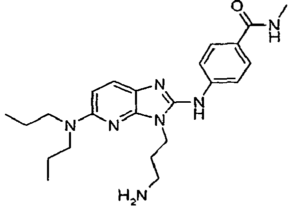 | 424,3 | 7,6 |
| 128 | 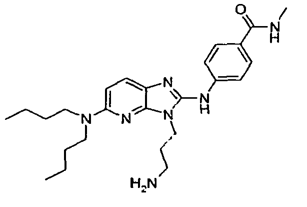 | 452,3 | 7,9 |
| 129 | 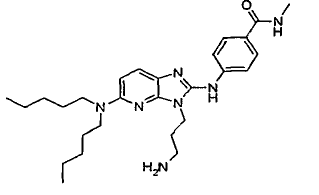 | 480,4 | 8,3 |
| 130 | 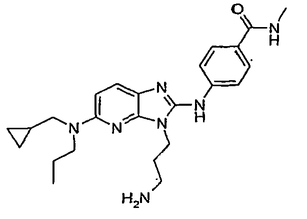 | 436,3 | 7,6 |
| 131 | 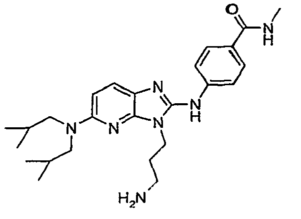 | 452,3 | 7,9 |
| 132 | 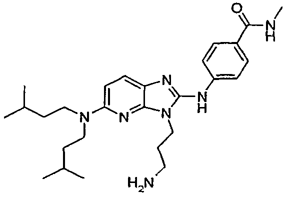 | 480,4 | 8,2 |
| 133 | 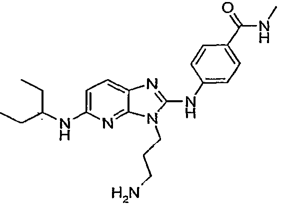 | 410,3 | 7,4 |
| 134 | 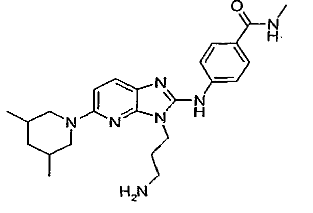 | 436,3 | 7,6 |
| 135 | 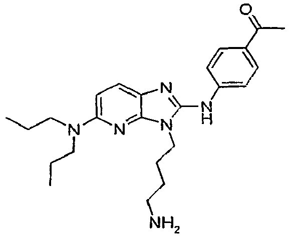 | 423,1 | 7,8 |
| 136 | 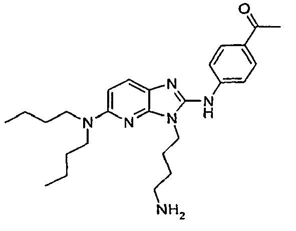 | 451,1 | 8,2 |
| 137 | 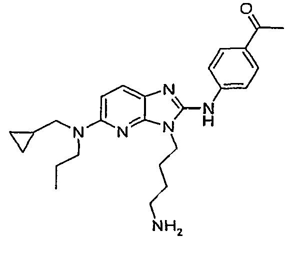 | 435,1 | 7,8 |
| 138 | 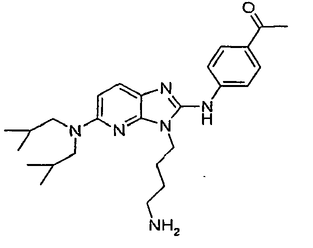 | 451,2 | 8,1 |
| 139 | 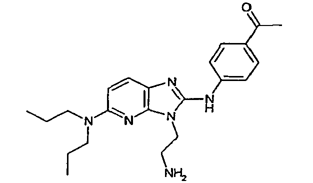 | 395,0 | 7,9 |
| 140 | 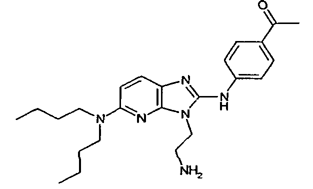 | 423,0 | 8,3 |
| 141 | 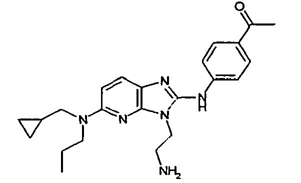 | 407,0 | 7,9 |
| 142 | 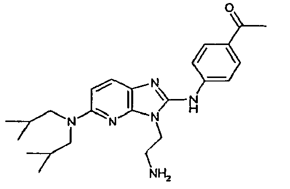 | 423,0 | 8,3 |
| 143 | 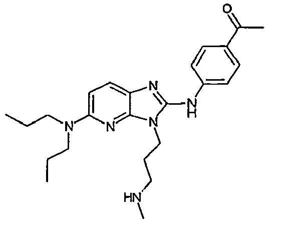 | 423,2 | 7,9 |
| 144 | 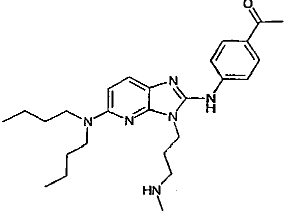 | 451,2 | 8,3 |
| 145 | 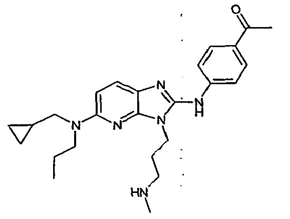 | 435,2 | 7,9 |
| 146 | 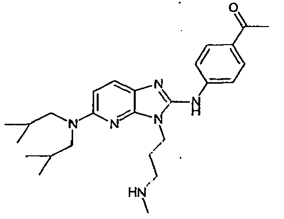 | 451,3 | 8,2 |
| 147 | 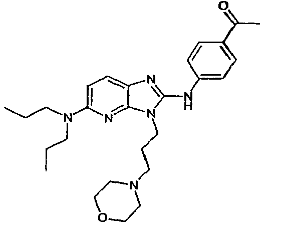 | 479,2 | 8,0 |
| 148 | 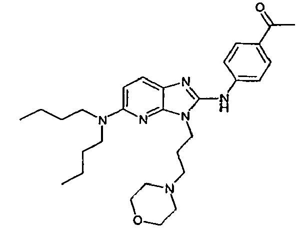 | 507,3 | 8,4 |
| 149 | 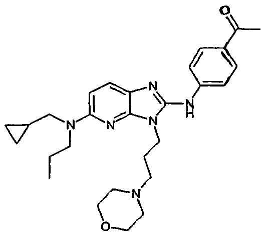 | 491,3 | 8,0 |
| 150 | 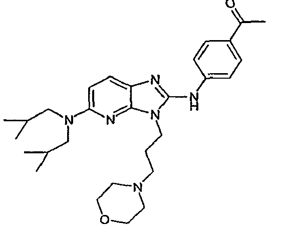 | 507,3 | 8,4 |
| 151 | 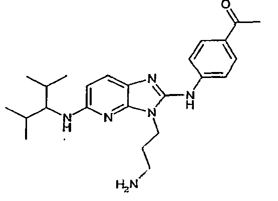 | 423,3 | 7,3 |
| 152 | 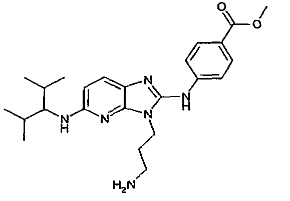 | 439,3 | 7,4 |
| 153 | 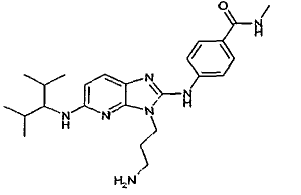 | 438,3 | 7,2 |
| 154 | 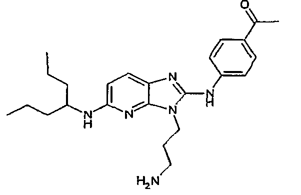 | 423,2 | 7,4 |
| 155 | 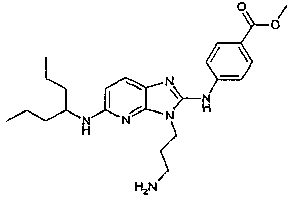 | 439,3 | 7,5 |
| 156 | 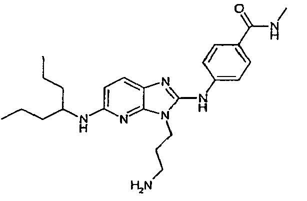 | 438,3 | 7,2 |
| 157 | 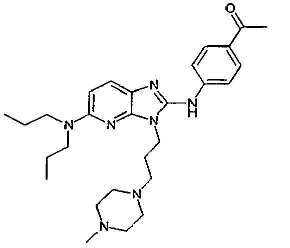 | 492,4 | 7,3 |
| 158 | 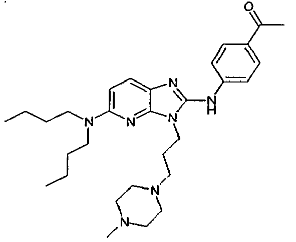 | 520,4 | 7,6 |
| 159 | 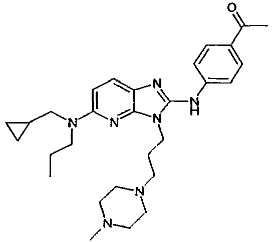 | 504,4 | 7,3 |
| 160 | 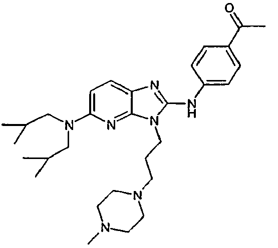 | 520,4 | 7,6 |
| 161 | 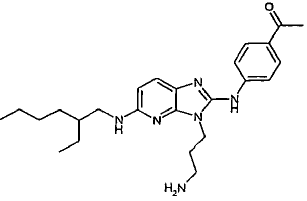 | 437,3 | 8,3 |
| 162 | 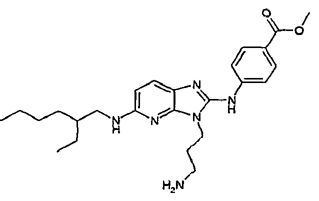 | 453,3 | 8,4 |
| 163 | 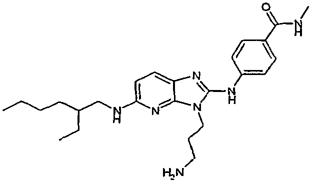 | 452,3 | 8,0 |
| 164 | 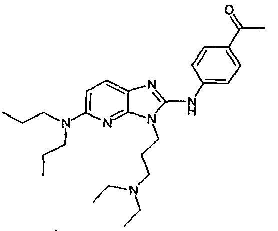 | 465,3 | 8,0 |
| 165 | 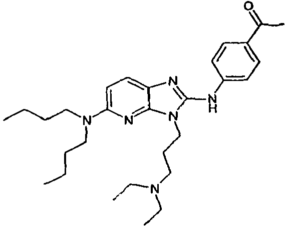 | 493,4 | 8,5 |
| 166 | 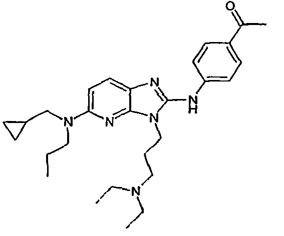 | 477,3 | 8,1 |
| 167 | 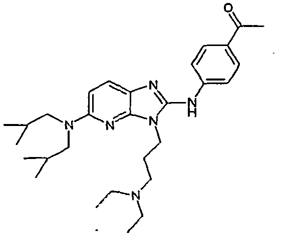 | 493,4 | 8,4 |
| 168 | 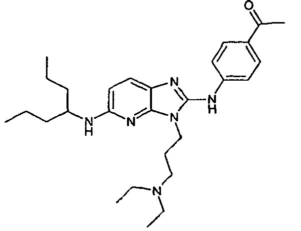 | 479,4 | 8,2 |
| 169 | 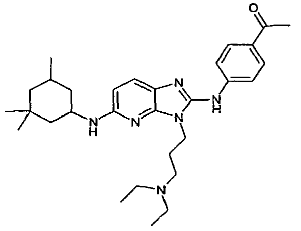 | 505,4 | 8,4 |
| 170 | 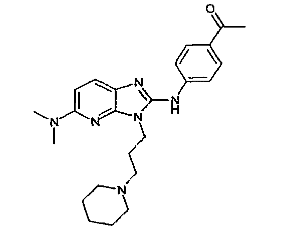 | 421,3 | 7,4 |
| 171 | 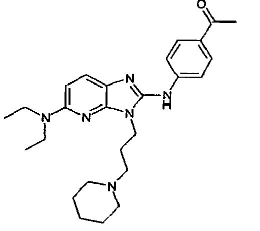 | 449,4 | 7,6 |
| 172 | 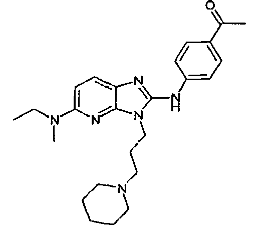 | 435,3 | 7,5 |
| 173 | 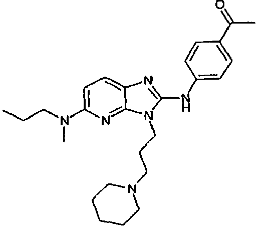 | 449,4 | 7,7 |
| 174 | 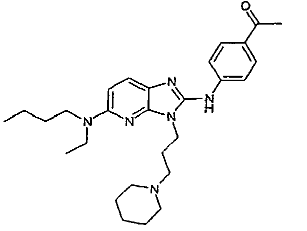 | 477,4 | 8,0 |
| 175 | 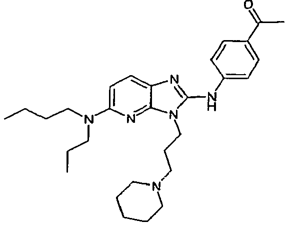 | 491,4 | 8,3 |
| 176 | 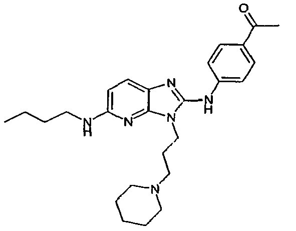 | 449,4 | 7,6 |
| 177 | 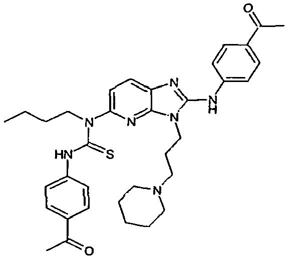 | 626,4 | 8,8 |
| 178 | 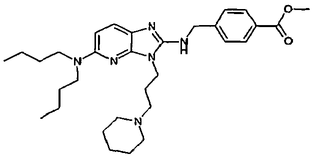 | 535,4 | 8,4 |
| 179 | 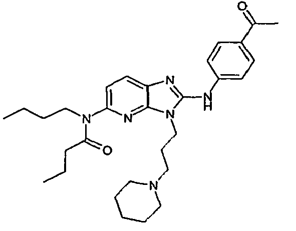 | 519,4 | 8,8 |
| 180 | 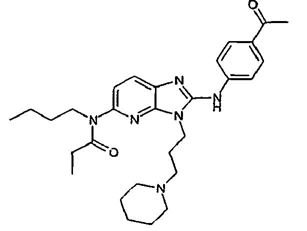 | 505,3 | 8,6 |
| 181 | 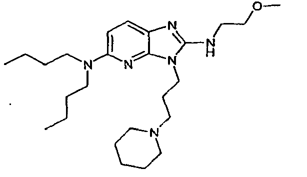 | 445,4 | 8,4 |
| 182 | 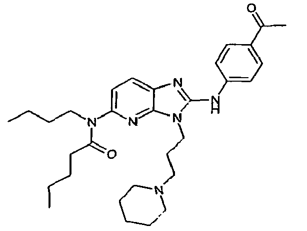 | 533,3 | 8,9 |
| 183 | 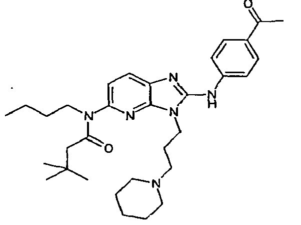 | 547,4 | 9,1 |
| 184 | 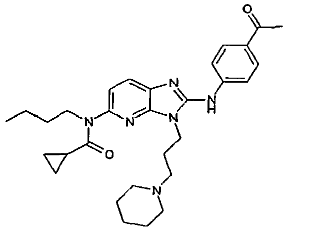 | 517,3 | 8,5 |
| 185 | 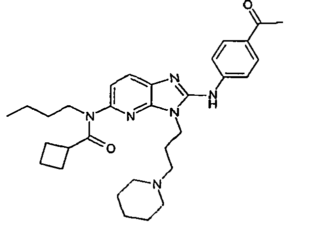 | 531,3 | 8,7 |
| 186 | 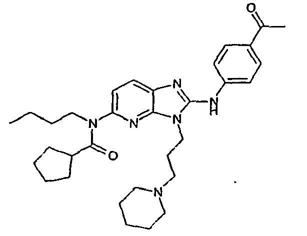 | 545,3 | 8,9 |
| 187 | 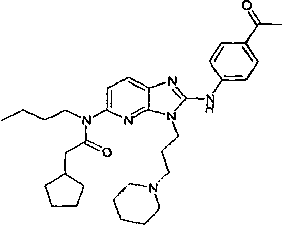 | 559,4 | 9,1 |
| 188 | 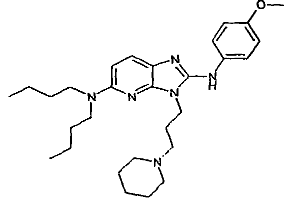 | 493,4 | 8,7 |
| 189 | 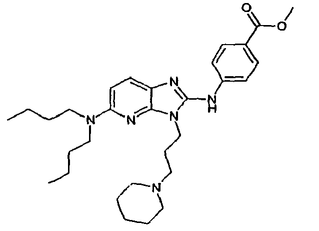 | 521,4 | 8,8 |
| 190 | 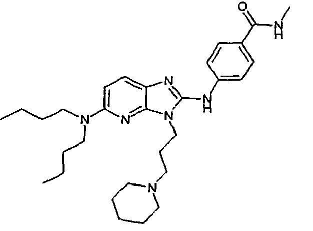 | 520,4 | 8,4 |
| 191 | 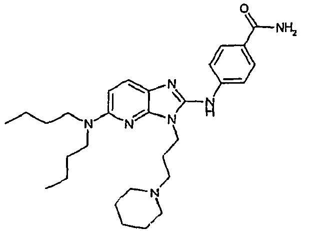 | 506,4 | 8,4 |
| 192 | 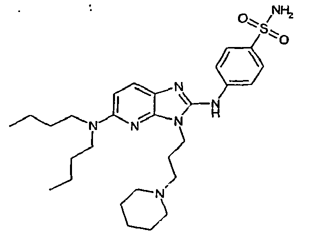 | 542,3 | 8,6 |
| 193 | 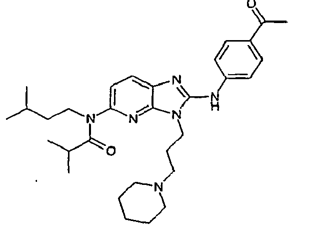 | 533,3 | 8,9 |
| 194 | 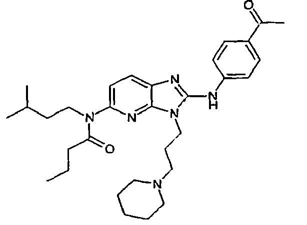 | 533,3 | 8,9 |
| 195 | 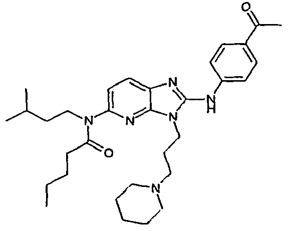 | 547,3 | 9,1 |
| 196 | 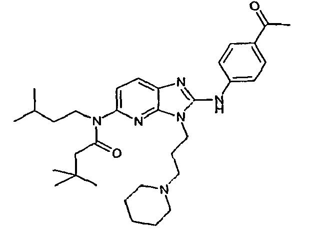 | 561,4 | 9,4 |
| 197 | 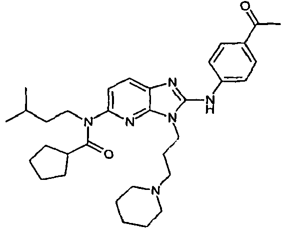 | 559,4 | 9,2 |
| 198 | 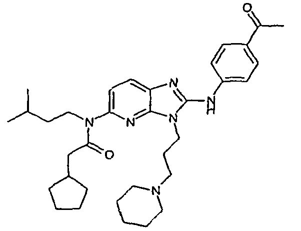 | 573,4 | 9,4 |
| 199 | 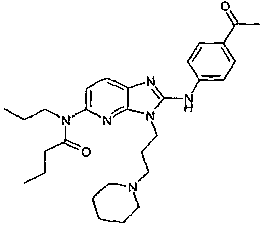 | 505,3 | 8,4 |
| 200 | 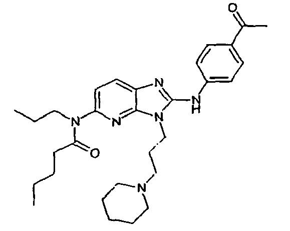 | 519,3 | 8,7 |
| 201 | 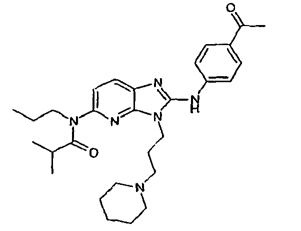 | 505,3 | 8,4 |
| 202 | 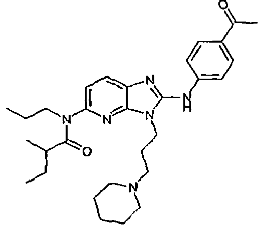 | 519,3 | 8,6 |
| 203 | 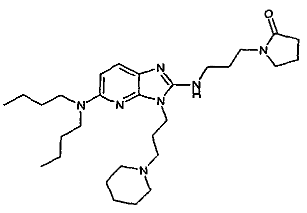 | 512,4 | 8,6 |
| 204 | 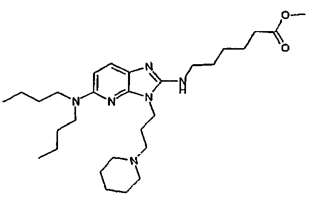 | 515,4 | 8,8 |
| 205 | 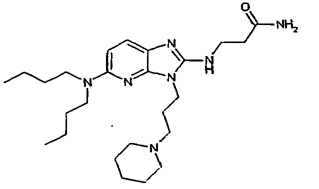 | 458,3 | 8,4 |
| 206 | 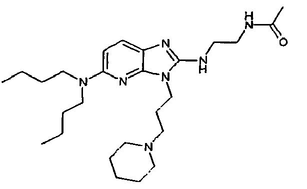 | 472,4 | 8,5 |
| 207 | 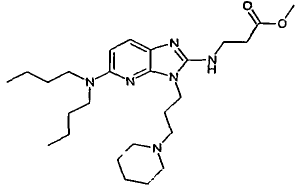 | 473,4 | 8,2 |
| 208 | 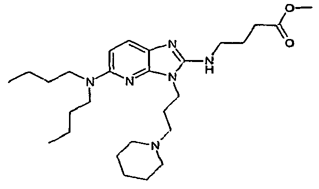 | 487,4 | 8,3 |
| 209 | 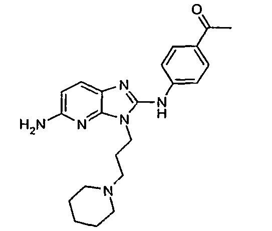 | 393,2 | 7,2 |
| 210 | 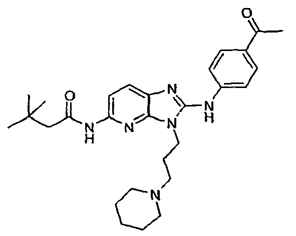 | 491,6 | 8,1 |
| 211 | 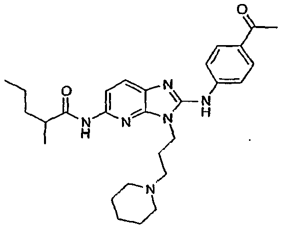 | 491,6 | 8,1 |

### Etude pharmacologique

L'affinité des composés de la présente invention pour les différents sous-types de récepteurs des mélanocortines a été mesurée selon les procédures analogues à celles décrites ci-après pour les récepteurs MC4.

### Etude de l'affinité des composés pour les récepteurs MC4 des mélanocortines :

L'affinité des composés de invention pour les récepteurs MC4 est déterminée par la mesure de l'inhibition de la liaison de la [¹²⁵I]-[Nle⁴, D-Phe⁷]-α-MSH à des préparations membranaines de cellules CHO-K1 transfectées.

Les cellules CHO-K1 exprimant de façon stable les récepteurs MC4 humains sont cultivées dans un milieu RPMI 1640 contenant 10 % de sérum foetal de veau, 2 mM de glutamine, 100 U/ml de pénicilline, 0,1 mg/ml de streptomycine et 0,5 mg/ml de G418. Les cellules sont collectées avec 0,5 mM d'EDTA et centrifugées à 500 g pendant 5 min à 4° C. Le culot est re-suspendu dans un milieu salin avec tampon phosphate (PBS) et centrifugé à 500 g pendant 5 min à 4° C. Le culot est re-suspendu dans un milieu tampon Tris 50 mM à pH 7,4 et centrifugé à 500 g pendant 5 min à 4° C. Les cellules sont lysées par sonication et centrifugées à 39 000 g pendant 10 min à 4° C. Le culot est re-suspendu dans le milieu tampon Tris 50 mM à pH 7,4 et centrifugé à 50 000 g pendant 10 min à 4° C. Les membranes obtenues dans ce dernier culot sont stockées à -80° C.

La mesure de l'inhibition compétitive de la liaison de la [¹²⁵I]-[Nle⁴, D-Phe⁷]-α-MSH sur les récepteurs MC4 est effectuée en duplicats à l'aide de plaques en polypropylène de 96 puits. Les membranes cellulaires (50 µg de protéines/puits) sont incubées avec la [¹²⁵I]-[Nle⁴, D-Phe⁷]-α-MSH (0,5 nM) pendant 90 min à 37° C dans un milieu tampon Tris-HCl 50 mM, pH 7,4, comprenant 0,2 % d'albumine bovine de sérum (BSA), 5 mM de MgCl₂, et 0,1 mg/ml de bacitracine.

La [¹²⁵I]-[Nle⁴, D-Phe⁷]-α-MSH liée est séparée de la [¹²⁵I]-[Nle⁴, D-Phe⁷]-α-MSH libre par filtration à travers des plaques de filtres en fibre de verre GF/C (Unifilter, Packard) pré-imprégné avec 0,1 % de polyéthylènimine (P.E.I.), en utilisant un Filtermate 196 (Paclcard). Les filtres sont lavés avec du tampon Tris-HCl 50 mM, pH 7,4 à 0-4° C et la radioactivité présente est déterminée à l'aide d'un compteur (Packard Top Count).

La liaison spécifique est obtenue en soustrayant la liaison non spécifique (déterminée en présence de 0,1 µM de Nle⁴, D-Phe⁷-α-MSH) de la liaison totale. Les données sont analysées par régression non-linéaire assistée par ordinateur (MDL) et les valeurs des constantes d'inhibition (Ki) sont déterminées.

L'activité agoniste ou antagoniste des récepteurs MC4 des composés de la présente invention a été déterminée en mesurant la production d'AMP cyclique par les cellules CHO-K1 transfectées par le récepteur MC4.

### Mesure de la production d'AMP cyclique intracellulaire via les récepteurs MC4 :

Les cellules CHO-K1 exprimant les récepteurs MC4 des mélanocortines sont cultivées dans des plaques à 384 puits dans un milieu RPMI 1640 avec 10 % de sérum foetal de veau et 0,5 mg/ml de G418. Les cellules sont lavées 2 fois avec 50 µl de milieu RPMI comprenant 0,2 % BSA et 0,5 mM de 3-isobutyl-1-méthylxanthine (IBMX).

Pour mesurer l'effet agoniste d'un composé, les cellules sont incubées pendant 5 min à 37° C en présence de 0,5 mM d'IBMX, puis la stimulation de la production d'AMP cyclique est obtenue en ajoutant le composé à des concentrations comprises entre 1 pM et 10 µM en duplicats pendant 20 min à 37° C. L'effet antagoniste d'un composé est mesuré par l'addition simultanée de Nle⁴, D-Phe⁷-α-MSH à des concentrations comprises entre 1 pM et 10 µM, en présence du composé à tester, à des concentrations comprises entre 0,1 nM et 10 µM en duplicats pendant 20 min à 37° C.

Le milieu réactionnel est éliminé et 80 µl de tampon de lyse sont ajoutés. Le taux d'AMP cyclique intracellulaire est mesuré par un test de compétition avec de l'AMP cyclique fluorescent (CatchPoint, Molecular Devices).

## Revendications

1. Composé de formule générale (I) 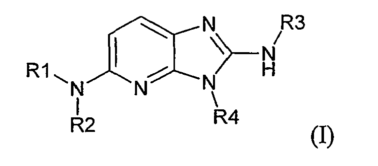 sous forme racémique, d'énantiomère ou toutes combinaisons de ces formes et dans laquelle :
R₁ et R₂ représentent, indépendamment, l'atome d'hydrogène ; un radical (C₁-C₈)alkyle éventuellement substitué par hydroxy; (C₂-C₆)alkényle ; un bicycloalkyle ; ou un radical de formule -(CH₂)ₙ-X₁ ou -X-(CH₂)ₙ-X'₁ ;
X représente -C(O)- ou -C(S)-NH- ;
X₁ représente un radical (C₁-C₆)alkoxy, (C₃-C₇)cycloalkyle, adamantyle, hétérocycloalkyle, aryle ou hétéroaryle,
les radicaux (C₃-C₇)cycloalkyle, hétérocycloalkyle, aryle et hétéroaryle étant éventuellement substitués par un ou plusieurs substituants identiques ou différents choisis parmi : -(CH₂)ₙ)-V₁-Y₁, halo , nitro et cyano ;
V₁ représente -O-, -S- ou une liaison covalente ;
Y₁ représente un radical (C₁-C₆)alkyle éventuellement substitué par un ou plusieurs radicaux halo identiques ou différents, ou aryle ;
n et n^{'} représentent un entier de 0 à 6 et n₁ un entier de 0 à 2 (étant entendu que lorsque n est égal à 0, alors X₁ ne représente pas le radical alkoxy) ;
X'₁ représente l'atome d'hydrogène, un radical (C₁-C₆)alkyle éventuellement substitué par un ou plusieurs radicaux halo identiques ou différents, (C₃-C₇)cycloalkyle ; ou aryle éventuellement substitué par un ou plusieurs substituants identiques ou différents choisi parmi : halo, nitro, cyano, (C₁-C₆)alkyl-carbonyle, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, -(CH₂)_{n"}-A, -C(O)-NV₁'Y₁', et hétérocycloalkyle ; ou bien R₁ et R₂ forment ensemble un radical de formule : 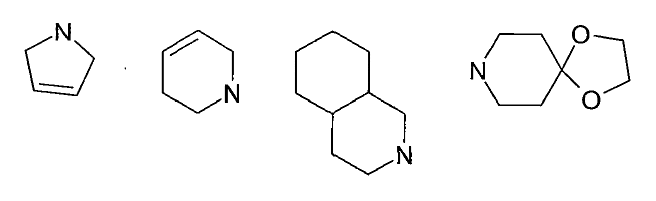
V₁'et Y₁' représentent, indépendamment, l'atome d'hydrogène ou un (C₁-C₆)alkyle ;
A représente un radical aryle éventuellement substitué par un ou plusieurs substituants identiques ou différents choisi parmi : halo, nitro, cyano, (C₁-C₆)alkyle éventuellement substitué par un ou plusieurs radicaux halo identiques ou différents, et (C₁-C₆)alkoxy éventuellement substitué par un ou plusieurs radicaux halo identiques ou différents ;
n^{"} représente un entier de 0 à 2 ;
R₃ représente -Z₃, -C(R_{Z3})(R'_{Z3})-Z₃, -C(R_{Z3})(R'_{Z3})-(CH₂)ₚ-Z₃ ou -C(O)-Z'₃ ;
R_{Z3} et R'_{Z3} représentent, indépendamment, l'atome d'hydrogène ou un radical (C₁-C₆)alkyle ;
Z₃ représente Z₃ₐ, Z_{3b}, Z_{3c}, Z_{3d}, ou Z₃ₑ ;
Z₃ₐ représente un radical (C₁-C₆)alkyle ou (C₂-C₆)alkényle ;
Z_{3b} représente un radical (C₁-C₆)alkoxy, (C₁-C₆)alkylthio, (C₁-C₆)alkylamino ou di((C₁-C₆)alkyl)amino ;
Z_{3c} représente un radical aryle ou hétéroaryle ;
les radicaux aryle et hétéroaryle étant éventuellement substitués par un ou plusieurs substituants identiques ou différents choisis parmi : halo, cyano, nitro, azido, oxy ou -(CH₂)_{p'}-V₃-Y₃;
V₃ représente -O-, -S-, -C(O)-, -C(O)-O-, -O(CO)-, -SO₂-, -SO₂NH-, -NR'₃-SO₂-, -NR'₃-, -NR'₃-C(O)-, -C(O)-NR'₃-, -NH-C(O)-NR'₃- ou une liaison covalente ;
Y₃ représente l'atome d'hydrogène ou un radical (C₁-C₆)alkyle éventuellement substitué par un ou plusieurs radicaux halo identiques ou différents ; un radical aryle éventuellement substitué par un ou plusieurs substituants identiques ou différents choisis parmi : halo, nitro, (C₁-C₆)alkyle et (C₁-C₆)alkoxy ; ou un radical aryl-(C₁-C₆)alkyle éventuellement substitué par un ou plusieurs substituants identiques ou différents choisis parmi : halo, nitro, (C₁-C₆)alkyle et (C₁-C₆)alkoxy ;
Z_{3d} représente un radical (C₁-C₆)alkoxy-carbonyle, amino-carbonyle, (C₁-C₆)alkylamino-carbonyle, di((C₁-C₆)alkyl)amino-carbonyle ;
Z₃ₑ représente un radical (C₁-C₆)alkyl-C(O)-NH-, (C₃-C₇)cycloalkyle, hétérocycloalkyle ou un radical de formule 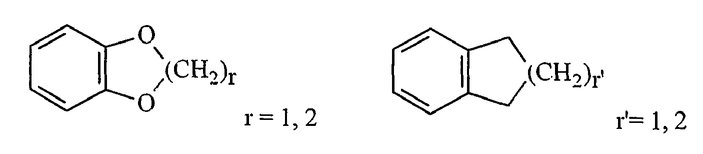 les radicaux (C₃-C₇) cycloalkyle et hétérocycloalkyle étant éventuellement substitués par un ou plusieurs radicaux oxy ou (C₁-C₆)alkyle identiques ou différents,
Z'₃ représente un radical aryle éventuellement substitué par un ou plusieurs substituants identiques ou différents choisis parmi : halo, nitro et -(CH₂)_{p"}-V'₃-Y'₃ ;
V'₃ représente -O-, -C(O)-, -C(O)-O-, -C(O)-NR'₃-, -NR'₃-C(O)-, -NH-C(O)-NR'₃ ou une liaison covalente ;
Y'₃ représente l'atome d'hydrogène ou un radical (C₁-C₆)alkyle éventuellement substitué par un ou plusieurs radicaux halo identiques ou différents ;
R'₃ représente l'atome d'hydrogène, un radical (C₁-C₆)alkyle ou (C₁-C₆)alkoxy ;
p, p^{'} et p^{"} représentent, indépendamment, un entier de 0 à 6 ;
R₄ représente un radical de formule-(CH₂)ₛ-R'₄
R'₄ représente un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle ; aryle éventuellement substitué par un ou plusieurs radicaux identiques ou différents choisis parmi : -(CH₂)_{s"}-V₄-Y₄,, halo et nitro ;
V₄ représente -O-, -S-, -NH-C(O)-, -NV₄'- ou une liaison covalente ;
Y₄ représente un atome d'hydrogène ou un radical (C₁-C₆)alkyle éventuellement substitué par un ou plusieurs radicaux halo identiques ou différents ;
V₄' représente un atome d'hydrogène ou un (C₁-C₆)alkyle ;
s^{"} représente un entier de 0 à 4 ;
ou bien Z₄ représente un radical de formule 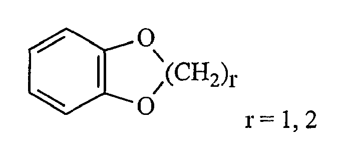
s et s^{'} représentent, indépendamment, un entier de 0 à 6 ;
ou un sel pharmaceutiquement acceptable de ce dernier.

2. Composé selon la revendication 1, **caractérisé en ce que**
R₁ et R₂ représentent, indépendamment, l'atome d'hydrogène, un radical (C₁-C₈)alkyle, un bicycloalkyle ou un radical de formule -(CH₂)ₙ-X₁ ou -X-(CH₂)_{n'}-X'₁ ;
X représente -C(O)- ou -C(S)-NH- ;
X₁ représente un radical (C₁-C₆)alkoxy, (C₃-C₇)cycloalkyle éventuellement substitué par un (C₁-C₆)alkyle, ou hétéroaryle ;
X'₁ représente l'atome d'hydrogène, un radical (C₁-C₆)alkyle éventuellement substitué par un ou plusieurs radicaux halo identiques ou différents, (C₃-C₇)cycloalkyle ou aryle éventuellement substitué par un (C₁-C₆)alkyl-carbonyle ;
ou bien R₁ et R₂ forment ensemble, avec l'atome d'azote auquel ils sont rattachés, un hétérobicycloalkyle ou un hétérocycloalkyle éventuellement substitué par un ou plusieurs substituants identiques ou différents choisis parmi : (C₁-C₆)alkyle, (C₁-C₆)alkoxy-carbonyle et -(CH₂)_{n"}-A ;
A représente un radical aryle éventuellement substitué par un ou plusieurs substituants identiques ou différents choisi parmi : halo et (C₁-C₆)alkyle ;
n^{"} représente un entier de 0 à 1 ;
R₄ représente un radical de formule-(CH₂)ₛ-R'₄
R'₄ représente un hétérocycloalkyle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène, (C₁-C₈)alkyle ou aryle éventuellement substitué par un ou plusieurs radicaux identiques ou différents choisis parmi : -(CH₂)_{s"}-V₄-Y₄ ;
V₄ représente -O- ;
Y₄ représente un radical (C₁-C₆)alkyle éventuellement substitué par un ou plusieurs radicaux halo identiques ou différents ;
s^{"} représente un entier de 0 à 4 ;
s et s^{'} représentent, indépendamment, un entier de 1 à 4 ;
ou un sel pharmaceutiquement acceptable de ce dernier.

3. Composé selon la revendication 2, **caractérisé en ce qu'**il comporte au moins une des caractéristiques suivantes :
- le radical cycloalkyle est choisi parmi cyclopropyle, cyclobutyle et cyclohexyle ;
- le radical bicycloalkyle est le bicylo[2,2,1]heptane ;
- l'hétérobicycloalkyle est le 7-aza-bicyclo[2,2,1]heptane ;
- le radical aryle est le radical phényle ;
- le radical hétéroaryle est le radical furyle ;
- l'hétérocycloalkyle est choisi parmi pipéridine, morpholine et pipérazine ;
ou un sel pharmaceutiquement acceptable de ce dernier.

4. Composé selon l'une des revendications 1 à 2, **caractérisé en ce que**
R₁ et R₂ représentent, indépendamment, l'atome d'hydrogène, un radical (C₁-C₈)alkyle ou un radical de formule -(CH₂)ₙ-X₁ ou -X-(CH₂)_{n'}-X'₁ ;
X représente -C(O)- ;
X₁ représente un radical (C₃-C₇)cycloalkyle ;
X'₁ représente l'atome d'hydrogène ou un radical (C₃-C₇)cycloalkyle ;
n représente 0 ou 1 ; n' représente un entier de 0 à 5 ;
ou bien R₁ et R₂ forment ensemble, avec l'atome d'azote auquel ils sont rattachés, un hétérocycloalkyle éventuellement substitué par un ou plusieurs substituants (C₁-C₆)alkyle identiques ou différents ; ou un sel pharmaceutiquement acceptable de ce dernier.

5. Composé selon la revendication 4, **caractérisé en ce que** le radical (C₃-C₇)cycloalkyle que représentent X₁ et X'₁ est choisi parmi cyclopropyle, cyclobutyle et cyclohexyle ; et l'hétérocycloalkyle que forment ensemble R₁ et R₂, est le cycle pipéridine ; ou un sel pharmaceutiquement acceptable de ce dernier.

6. Composé selon l'une des revendications 1 à 2 et 4 à 5, **caractérisé en ce que**
R₄ représente un radical de formule-(CH₂)ₛ-R'₄
R'₄ représente un hétérocycloalkyle contenant au moins un atome d'azote et éventuellement substitué par (C₁-C₆)alkyle ; ou un radical de formule -NW₄W'₄
W₄ représente l'atome d'hydrogène ou (C₁-C₈)alkyle ;
W'₄ représente un radical de formule -(CH₂)_{s'}-Z₄ ;
Z₄ représente l'atome d'hydrogène ou (C₁-C₈)alkyle ;
s et s^{'} représentent, indépendamment, un entier de 2 à 4 ;
ou un sel pharmaceutiquement acceptable de ce dernier.

7. Composé selon la revendication 6, **caractérisé en ce que** l'hétérocycloalkyle que représente R'₄ est choisi parmi : pipéridine et morpholine ; ou un sel pharmaceutiquement acceptable de ce dernier.

8. Composé selon l'une des revendications précédentes, **caractérisé en ce que** R₃ représente -C(O)-Z'₃
Z'₃ représente un radical aryle éventuellement substitué par un ou plusieurs substituants identiques ou différents choisis parmi halo et -(CH₂)_{p"}-V'₃-Y'₃ ;
V'₃ représente -O- ou une liaison covalente ;
Y'₃ représente l'atome d'hydrogène ou un radical (C₁-C₆)alkyle éventuellement substitué par un ou plusieurs radicaux halo identiques ou différents ;
p^{"} représente un entier de 0 à 2 ;
ou un sel pharmaceutiquement acceptable de ce dernier.

9. Composé selon l'une des revendications précédentes, **caractérisé en ce que** R₃ représente -Z₃, -C(R_{Z3})(R'_{Z3})-Z₃ ou -C(R_{Z3})(R'_{Z3})-(CH₂)ₚ-Z₃; ou un sel pharmaceutiquement acceptable de ce dernier.

10. Composé selon la revendication 9, **caractérisé en ce que** R₃ représente -Z₃ et Z₃ représente Z_{3b}, Z_{3c} ou Z₃ₑ ; ou un sel pharmaceutiquement acceptable de ce dernier.

11. Composé selon la revendication 10, **caractérisé en ce que** Z₃ représente Z_{3c} et Z_{3c} représentent un radical aryle ; ou un sel pharmaceutiquement acceptable de ce dernier.

12. Composé selon la revendication 11, **caractérisé en ce que** Z_{3c} représente un radical phényle substitué par un ou plusieurs substituants identiques ou différents choisis parmi : halo, nitro ou -(CH₂)_{p'}-V₃-Y₃ ;
V₃ représente -O-, -S-, -C(O)-, -C(O)-O-, -SO₂NH-, -NR'₃-C(O)-, -C(O)-NR'₃- ou une liaison covalente ;
R'₃ représente l'atome d'hydrogène ;
Y₃ représente l'atome d'hydrogène ou un radical (C₁-C₆)alkyle éventuellement substitué par un ou plusieurs radicaux halo identiques ou différents ; ou un sel pharmaceutiquement acceptable de ce dernier.

13. Composé selon la revendication 11, **caractérisé en ce que** Z_{3c} représente un radical phényle substitué par un ou plusieurs substituants identiques ou différents de formule -(CH₂)_{p'}-V₃-Y₃;
V₃ représente -C(O)-, -C(O)-O- ou -C(O)-NR'₃- ;
R'₃ représente l'atome d'hydrogène ;
Y₃ représente l'atome d'hydrogène ou un radical (C₁-C₆)alkyle ; ou un sel pharmaceutiquement acceptable de ce dernier.

14. Composé selon la revendication 9, **caractérisé en ce que** R₃ représente -C(R_{Z3})(R'_{Z3})-Z₃ et Z₃ représente Z_{3d} ou Z₃ₑ ; ou un sel pharmaceutiquement acceptable de ce dernier.

15. Composé selon la revendication 9, **caractérisé en ce que** R₃ représente -C(R_{Z3})(R'_{Z3})-(CH₂)ₚ-Z₃ et Z₃ représente Z_{3c}, Z_{3d} ou Z₃ₑ ; ou un sel pharmaceutiquement acceptable de ce dernier.

16. Composé selon la revendication 15, **caractérisé en ce que** Z₃ représente Z_{3d} ou Z₃ₑ ;
Z_{3d} représente un radical (C₁-C₆)alkoxy-carbonyle ou amino-carbonyle ;
Z₃ₑ représente un radical (C₁-C₆)alkyl-C(O)-NH-, hétérocycloalkyle étant éventuellement substitué par un radical oxy, ou un radical de formule 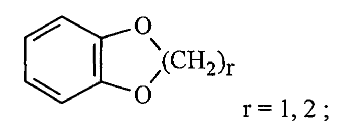
ou un sel pharmaceutiquement acceptable de ce dernier.

17. Procédé de préparation d'un composé de formule (I) selon l'une des revendications précédentes **caractérisé en ce que** l'on traite le composé de formule générale : 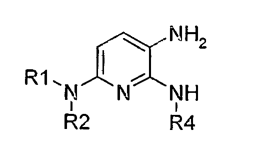 dans laquelle R₁, R₂, R₄ ont la signification indiquée dans la revendication 1, par un isothiocyanate de formule générale R₃N=C=S dans lequel R₃ a la signification indiquée dans la revendication 1, en présence d'un agent de couplage ou d'oxyde de mercure (II) jaune en présence de soufre, pendant une durée de 3 à 48 heures, dans un solvant protique ou aprotique, à une température de 50 à 80° C.

18. Composition pharmaceutique contenant, à titre de principe actif, au moins un composé selon l'une des revendications 1 à 16, en association avec un support pharmaceutiquement acceptable.

19. Utilisation d'un composé selon l'une des revendications 1 à 16, pour la préparation d'un médicament pour le traitement des désordres pondéraux, des troubles mentaux, de la douleur, des désordres de l'activité sexuelle.

20. Utilisation d'un composé selon la revendication 19, pour la préparation d'un médicament pour le traitement des désordres pondéraux tels que l'obésité, la cachexie et plus particulièrement la cachexie des pathologies cancéreuses, cachexie du sida, cachexie de la personne âgée, cachexie cardiaque, cachexie rénale, cachexie de l'arthrite rhumatoïde, et l'anorexie.

21. Utilisation d'un composé selon la revendication 19, pour la préparation d'un médicament pour le traitement des troubles mentaux tels que l'anxiété et la dépression.

22. Utilisation d'un composé selon la revendication 19, pour la préparation d'un médicament pour le traitement de la douleur et plus particulièrement la douleur neuropathique.

## Claims

1. Compound of general formula (I) 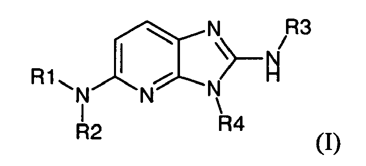 in racemic, or enantiomeric form or any combinations of these forms and in which:
R₁ and R₂ represent, independently, the hydrogen atom; a (C₁-C₈)alkyl radical optionally substituted by hydroxy; (C₂-C₆)alkenyl; a bicycloalkyl; or a radical of formula -(CH₂)ₙ-X₁ or -X-(CH₂)_{n'}-X'₁;
X represents -C(O)- or -C(S)-NH-;
X₁ represents a (C₁-C₆)alkoxy, (C₃-C₇)cycloalkyl, adamantyl, heterocycloalkyl, aryl or heteroaryl radical,
the (C₃-C₇)cycloalkyl, heterocycloalkyl, aryl and heteroaryl radicals being optionally substituted by one or more identical or different substituents chosen from: -(CH₂)ₙ₁-V₁-Y₁, halo, nitro and cyano;
V₁ represents -O-, -S- or a covalent bond;
Y₁ represents a (C₁-C₆)alkyl radical optionally substituted by one or more identical or different halo radicals, or aryl;
n and n' represent an integer from 0 to 6 and n₁ an integer from 0 to 2 (it being understood that when n is equal to 0, then X₁ does not represent the alkoxy radical);
X'₁ represents the hydrogen atom, a (C₁-C₆)alkyl radical optionally substituted by one or more identical or different halo radicals, (C₃-C₇)cycloalkyl; or aryl optionally substituted by one or more identical or different substituents chosen from: halo, nitro, cyano, (C₁-C₆)alkyl-carbonyl, (C₁-C₆)alkyl optionally substituted by one or more identical or different halo radicals, and (C₁-C₆)alkoxy optionally substituted by one or more identical or different halo radicals;
or R₁ and R₂ form together, with the nitrogen atom to which they are attached, a heterobicycloalkyl or a heterocycloalkyl optionally substituted by one or more identical or different substituents chosen from: hydroxy, (C₁-C₆)alkyl optionally substituted by hydroxy, (C₁-C₆)alkoxy-carbonyl, -(CH₂)_{n"}-A, -C(O)-NV₁'Y₁', and heterocycloalkyl; or R₁ and R₂ form together a radical of formula: 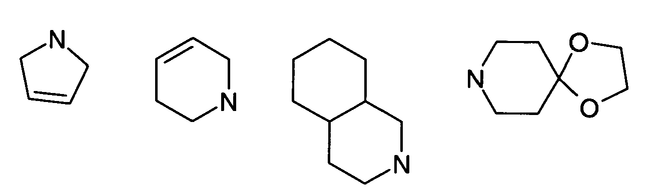
V₁' and Y₁' represent, independently, the hydrogen atom or a (C₁-C₆)alkyl;
A represents an aryl radical optionally substituted by one or more identical or different substituents chosen from: halo, nitro, cyano, (C₁-C₆)alkyl optionally substituted by one or more identical or different halo radicals, and (C₁-C₆)alkoxy optionally substituted by one or more identical or different halo radicals;
n^{"} represents an integer from 0 to 2;
R₃ represents -Z₃, -C(R_{Z3})(R'_{Z3})-Z₃, -C(R_{Z3})(R'_{Z3})-(CH₂)ₚ-Z₃ or -C(O)-Z'₃;
R_{Z3} and R'_{Z3} represent, independently, the hydrogen atom or a (C₁-C₆)alkyl radical;
Z₃ represents Z₃ₐ, Z_{3b}, Z_{3c}, Z_{3d}, or Z₃ₑ;
Z₃ₐ represents a (C₁-C₆)alkyl or (C₂-C₆)alkenyl radical;
Z_{3b} represents a (C₁-C₆)alkoxy, (C₁-C₆)alkylthio, (C₁-C₆)alkylamino or di((C₁-C₆)alkyl)amino radical;
Z_{3c} represents an aryl or heteroaryl radical;
the aryl and heteroaryl radicals being optionally substituted by one or more identical or different substituents chosen from: halo, cyano, nitro, azido, oxy or -(CH₂)_{p'}-V₃-Y_{3;}
V₃ represents -O-, -S-, -C(O)-, -C(O)-O-, -O(CO)-, -SO₂-, -SO₂NH-, -NR'₃-SO₂-, -NR'₃-, -NR'₃-C(O)-, -C(O)-NR'₃-, -NH-C(O)-NR'₃- or a covalent bond;
Y₃ represents the hydrogen atom or a (C₁-C₆)alkyl radical optionally substituted by one or more identical or different halo radicals; an aryl radical optionally substituted by one or more identical or different substituents chosen from: halo, nitro, (C₁-C₆)alkyl and (C₁-C₆)alkoxy; or an aryl-(C₁-C₆)alkyl radical optionally substituted by one or more identical or different substituents chosen from: halo, nitro, (C₁-C₆)alkyl and (C₁-C₆)alkoxy;
Z_{3d} represents a (C₁-C₆)alkoxy-carbonyl, amino-carbonyl, (C₁-C₆)alkylaminocarbonyl, di((C₁-C₆)alkyl)amino-carbonyl radical;
Z₃ₑ represents a (C₁-C₆)alkyl-C(O)-NH-, (C₃-C₇)cycloalkyl, heterocycloalkyl radical or a radical of formula 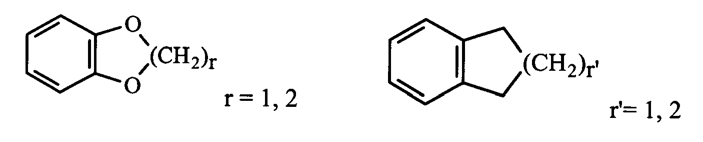 the (C₃-C₇) cycloalkyl and heterocycloalkyl radicals being optionally substituted by one or more identical or different oxy or (C₁-C₆)alkyl radicals,
Z'₃ represents an aryl radical optionally substituted by one or more identical or different substituents chosen from: halo, nitro and -(CH₂)_{p"}-V'₃-Y'_{3;}
V'₃ represents -O-, -C(O)-, -C(O)-O-, -C(O)-NR'₃-, -NR'₃-C(O)-, -NH-C(O)-NR'₃ or a covalent bond;
Y'₃ represents the hydrogen atom or a (C₁-C₆)alkyl radical optionally substituted by one or more identical or different halo radicals;
R'₃ represents the hydrogen atom, a (C₁-C₆)alkyl or (C₁-C₆)alkoxy radical;
p, p^{'} and p^{"} represent, independently, an integer from 0 to 6;
R₄ represents a radical of formula -(CH₂)ₛ-R'₄
R'₄ represents a heterocycloalkyl containing at least one nitrogen atom and optionally substituted by (C₁-C₆)alkyl or aralkyl; a heteroaryl containing at least one nitrogen atom and optionally substituted by (C₁-C₆)alkyl; or a radical of formula -NW₄W'₄
W₄ represents the hydrogen atom or (C₁-C₈)alkyl;
W'₄ represents a radical of formula -(CH₂)_{s'}-Z₄;
Z₄ represents the hydrogen atom, (C₁-C₈)alkyl; (C₂-C₆)alkenyl; (C₃-C₇)cycloalkyl optionally substituted by one or more identical or different (C₁-C₆)alkyl substituents; cyclohexene; heteroaryl; aryl optionally substituted by one or more identical or different radicals chosen from: -(CH₂)_{s"}-V₄-Y₄, halo and nitro;
V₄ represents -O-, -S-, -NH-C(O)-, -NV₄'- or a covalent bond;
Y₄ represents a hydrogen atom or a (C₁-C₆)alkyl radical optionally substituted by one or more identical or different halo radicals;
V₄' represents a hydrogen atom or a (C₁-C₆)alkyl;
s^{"} represents an integer from 0 to 4;
or Z₄ represents a radical of formula 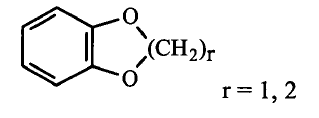
s and s' represent, independently, an integer from 0 to 6;
or a pharmaceutically acceptable salt thereof.

2. Compound according to claim 1, **characterized in that**
R₁ and R₂ represent, independently, the hydrogen atom, a (C₁-C₈)alkyl, a bicycloalkyl radical or a radical of formula -(CH₂)ₙ-X₁ or -X-(CH₂)_{n'}-X'₁;
X represents -C(O)- or -C(S)-NH-;
X₁ represents a (C₁-C₆)alkoxy, (C₃-C₇)cycloalkyl radical optionally substituted by a (C₁-C₆)alkyl, or heteroaryl;
X'₁ represents the hydrogen atom, a (C₁-C₆)alkyl radical optionally substituted by one or more identical or different halo radicals, (C₃-C₇)cycloalkyl or aryl optionally substituted by a (C₁-C₆)alkyl-carbonyl;
or R₁ and R₂ form together, with the nitrogen atom to which they are attached, a heterobicycloalkyl or a heterocycloalkyl optionally substituted by one or more identical or different substituents chosen from: (C₁-C₆)alkyl, (C₁-C₆)alkoxy-carbonyl and -(CH₂)_{n"}-A;
A represents an aryl radical optionally substituted by one or more identical or different substituents chosen from: halo and (C₁-C₆)alkyl;
n^{"} represents an integer from 0 to 1;
R₄ represents a radical of formula-(CH₂)ₛ-R'₄
R'₄ represents a heterocycloalkyl containing at least one nitrogen atom and optionally substituted by (C₁-C₆)alkyl; or a radical of formula -NW₄W'₄
W₄ represents the hydrogen atom or (C₁-C₈)alkyl;
W'₄ represents a radical of formula -(CH₂)_{s'}-Z₄;
Z₄ represents the hydrogen atom, (C₁-C₈)alkyl or aryl optionally substituted by one or more identical or different radicals chosen from: -(CH₂)_{s"}-V₄-Y₄;
V₄ represents -O-;
Y₄ represents a (C₁-C₆)alkyl radical optionally substituted by one or more identical or different halo radicals;
s^{"} represents an integer from 0 to 4;
s and s^{'} represent, independently, an integer from 1 to 4;
or a pharmaceutically acceptable salt thereof.

3. Compound according to claim 2, **characterized in that** it comprises at least one of the following characteristics:
- the cycloalkyl radical is chosen from cyclopropyl, cyclobutyl and cyclohexyl;
- the bicycloalkyl radical is bicyclo[2,2,1]heptane;
- the heterobicycloalkyl is 7-aza-bicyclo[2,2,1]heptane;
- the aryl radical is the phenyl radical;
- the heteroaryl radical is the furyl radical;
- the heterocycloalkyl is chosen from piperidine, morpholine and piperazine; or a pharmaceutically acceptable salt thereof.

4. Compound according to one of claims 1 to 2, **characterized in that**
R₁ and R₂ represent, independently, the hydrogen atom, a (C₁-C₈)alkyl radical or a radical of formula -(CH₂)ₙ-X₁ or -X-(CH₂)_{n'}-X'₁;
X represents -C(O)-;
X₁ represents a (C₃-C₇)cycloalkyl radical;
X'₁ represents the hydrogen atom or a (C₃-C₇)cycloalkyl radical;
n represents 0 or 1; n' represents an integer from 0 to 5;
or R₁ and R₂ form together, with the nitrogen atom to which they are attached, a heterocycloalkyl optionally substituted by one or more identical or different (C₁-C₆)alkyl substituents; or a pharmaceutically acceptable salt thereof

5. Compound according to claim 4, **characterized in that** the (C₃-C₇)cycloalkyl radical represented by X₁ and X'₁ is chosen from cyclopropyl, cyclobutyl and cyclohexyl; and the heterocycloalkyl that together form R₁ and R₂, is the piperidine ring; or a pharmaceutically acceptable salt thereof.

6. Compound according to one of claims 1 to 2 and 4 to 5, **characterized in that**
R₄ represents a radical of formula-(CH₂)ₛ-R'₄
R'₄ represents a heterocycloalkyl containing at least one nitrogen atom and optionally substituted by (C₁-C₆)alkyl; or a radical of formula -NW₄W'₄
W₄ represents the hydrogen atom or (C₁-C₈)alkyl;
W'₄ represents a radical of formula -(CH₂)_{s'}-Z₄;
Z₄ represents the hydrogen atom or (C₁-C₈)alkyl;
s and s^{'} represent, independently, an integer from 2 to 4;
or a pharmaceutically acceptable salt thereof

7. Compound according to claim 6, **characterized in that** the heterocycloalkyl represented by R'₄ is chosen from: piperidine and morpholine; or a pharmaceutically acceptable salt thereof

8. Compound according to one of the preceding claims, **characterized in that** R₃ represents -C(O)-Z'₃
Z'₃ represents an aryl radical optionally substituted by one or more identical or different substituents chosen from halo and -(CH₂)_{p"}-V'₃-Y'_{3;}
V'₃ represents -O- or a covalent bond;
Y'₃ represents the hydrogen atom or a (C₁-C₆)alkyl radical optionally substituted by one or more identical or different halo radicals;
p^{"} represents an integer from 0 to 2;
or a pharmaceutically acceptable salt thereof.

9. Compound according to one of the preceding claims, **characterized in that** R₃ represents -Z₃, -C(R_{Z3})(R'_{Z3})-Z₃ or -C(R_{Z3})(R'_{Z3})-(CH₂)ₚ-Z₃; or a pharmaceutically acceptable salt thereof

10. Compound according to claim 9, **characterized in that** R₃ represents -Z₃ and Z₃ represents Z_{3b}, Z_{3c} or Z₃ₑ; or a pharmaceutically acceptable salt thereof.

11. Compound according to claim 10, **characterized in that** Z₃ represents Z_{3c} and Z_{3c} represents an aryl radical; or a pharmaceutically acceptable salt thereof.

12. Compound according to claim 11, **characterized in that** Z_{3c} represents a phenyl radical substituted by one or more identical or different substituents chosen from: halo, nitro or -(CH₂)_{p'}-V₃-Y_{3;}
V₃ represents -O-, -S-, -C(O)-, -C(O)-O-, -SO₂NH-, -NR'₃-C(O)-, -C(O)-NR'₃- or a covalent bond;
R'₃ represents the hydrogen atom;
Y₃ represents the hydrogen atom or a (C₁-C₆)alkyl radical optionally substituted by one or more identical or different halo radicals; or a pharmaceutically acceptable salt thereof.

13. Compound according to claim 11, **characterized in that** Z_{3c} represents a phenyl radical substituted by one or more identical or different substituents of formula -(CH₂)_{p'}-V₃-Y_{3;}
V₃ represents -C(O)-, -C(O)-O- or -C(O)-NR'₃-;
R'₃ represents the hydrogen atom;
Y₃ represents the hydrogen atom or a (C₁-C₆)alkyl radical; or a pharmaceutically acceptable salt thereof.

14. Compound according to claim 9, **characterized in that** R₃ represents -C(R_{Z3})(R'_{Z3})-Z₃ and Z₃ represents Z_{3d} or Z₃ₑ; or a pharmaceutically acceptable salt thereof.

15. Compound according to claim 9, **characterized in that** R₃ represents -C(R_{Z3})(R'_{Z3})-(CH₂)ₚ-Z₃ and Z₃ represents Z_{3c}, Z_{3d} or Z₃ₑ; or a pharmaceutically acceptable salt thereof.

16. Compound according to claim 15, **characterized in that** Z₃ represents Z_{3d} or Z₃ₑ;
Z_{3d} represents a (C₁-C₆)alkoxy-carbonyl or amino-carbonyl radical;
Z₃ₑ represents a (C₁-C₆)alkyl-C(O)-NH-, heterocycloalkyl radical being optionally substituted by an oxy radical, or a radical of formula 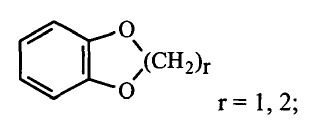
or a pharmaceutically acceptable salt thereof.

17. Process for the preparation of a compound of formula (I) according to one of the preceding claims **characterized in that** the compound of general formula: 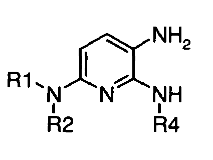 in which R₁, R₂, R₄ have the meaning indicated in claim 1, is treated with an isothiocyanate of general formula R₃N=C=S in which R₃ has the meaning indicated in claim 1, in the presence of a coupling agent or of yellow mercury (II) oxide in the presence of sulphur, for a duration of 3 to 48 hours, in a protic or aprotic solvent, at a temperature of 50 to 80°C.

18. Pharmaceutical composition containing, as active ingredient, at least one compound according to one of claims 1 to 16, in combination with a pharmaceutically acceptable support.

19. Use of a compound according to one of claims 1 to 16, for the preparation of a medicament for the treatment of weight disorders, mental disorders, pain, sexual activity disorders.

20. Use of a compound according to claim 19, for the preparation of a medicament for the treatment of weight disorders such as obesity, cachexia and more particularly cancer cachexia, AIDS cachexia, old age cachexia, cardiac cachexia, renal cachexia, rheumatoid arthritis cachexia, and anorexia.

21. Use of a compound according to claim 19, for the preparation of a medicament for the treatment of mental disorders such as anxiety and depression.

22. Use of a compound according to claim 19, for the preparation of a medicament for the treatment of pain and more particularly neuropathic pain.

## Patentansprüche

1. Verbindung der allgemeinen Formel (I) 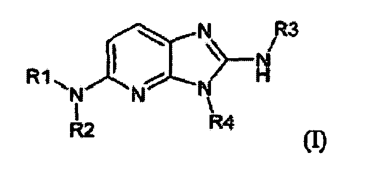 in racemischer Form, in enantiomerer Form oder in allen Kombinationen dieser Formen, bei der:
R₁ und R₂ unabhängig voneinander darstellen: ein Wasserstoffatom; einen (C₁-C₈)-Alkylrest, gegebenenfalls substituiert mit Hydroxy; (C₂-C₆)-Alkenyl; ein Bicycloalkyl; oder einen Rest der Formel -(CH₂)ₙ-X₁ oder -X-(CH₂)_{n'}-X'1;
X -C(O)- oder -C(S)-NH darstellt;
X₁ einen Rest (C₁-C₆)-Alkoxy, (C₃-C₇)-Cycloalkyl, Adamantyl, Heterocycloalkyl, Aryl oder Heteroaryl darstellt,
wobei die Reste (C₃-C₇)-Cycloalkyl, Heterocycloalkyl, Aryl und Heteroaryl gegebenenfalls mit einem oder mehreren gleichen oder verschiedenen Substituenten substituiert sind, ausgewählt aus: -(CH₂)ₙ₁-V₁-Y₁, Halogen, Nitro und Cyano;
V₁ -O-, -S- oder eine kovalente Bindung darstellt;
Y₁ einen (C₁-C₆)-Alkylrest, gegebenenfalls substituiert mit einem oder mehreren gleichen oder verschiedenen Halogenresten, oder Aryl darstellt;
n und n' eine ganze Zahl von 0 bis 6 und n₁ eine ganze Zahl von 0 bis 2 darstellen (wobei gilt, dass, wenn n gleich 0 ist, dann X₁ nicht den Alkoxyrest darstellt);
X'₁ darstellt: ein Wasserstoffatom, einen (C₁-C₆)-Alkylrest, der gegebenenfalls mit einem oder mehreren gleichen oder verschiedenen Halogenresten substituiert ist, (C₃-C₇)-Cycloalkyl; oder Aryl, gegebenenfalls substituiert mit einem oder mehreren gleichen oder verschiedenen Substituenten, ausgewählt aus: Halogen, Nitro, Cyano, (C₁-C₆)-Alkylcarbonyl, (C₁-C₆)-Alkyl, gegebenenfalls substituiert mit einem oder mehreren gleichen oder verschiedenen Halogenresten, und (C₁-C₆)-Alkoxy, gegebenenfalls substituiert mit einem oder mehreren gleichen oder verschiedenen Halogenresten;
oder R₁ und R₂ zusammen mit dem Stickstoffatom, an das sie gebunden sind, ein Heterobicycloalkyl oder ein Heterocycloalkyl bilden, das gegebenenfalls mit einem oder mehreren gleichen oder verschiedenen Substituenten substituiert ist, ausgewählt aus: Hydroxy, (C₁-C₆)-Alkyl, gegebenenfalls substituiert mit Hydroxy, (C₁-C₆)-Alkoxycarbonyl, -(CH₂)ₙ-A, -C(O)-NV₁'Y₁' und Heterocycloalkyl; oder R₁ und R₂ zusammen einen Rest der Formel bilden: 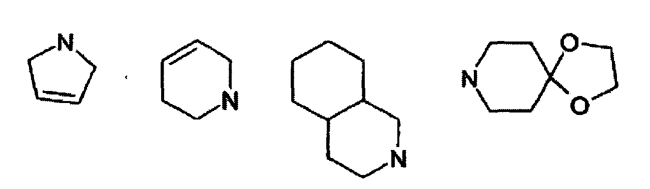 V₁' und Y₁' unabhängig voneinander ein Wasserstoffatom oder ein (C₁-C₆)-Alkyl darstellen;
A darstellt: einen Arylrest, gegebenenfalls substituiert mit einem oder mehreren gleichen oder verschiedenen Substituenten, ausgewählt aus: Halogen, Nitro, Cyano, (C₁-C₆)-Alkyl, gegebenenfalls substituiert mit einem oder mehreren gleichen oder verschiedenen Halogenresten, und (C₁-C₆)-Alkoxy, gegebenenfalls substituiert mit einem oder mehreren gleichen oder verschiedenen Halogenresten;
n" eine ganze Zahl von 0 bis 2 darstellt;
R₃ -Z₃, -C(R_{Z3})(R'_{Z3})-Z₃, -C(R_{Z3})(R'_{Z3})-(CH₂)ₚ-Z₃ oder -C(O)-Z'₃ darstellt;
R_{Z3} und R'_{Z3} unabhängig voneinander ein Wasserstoff oder einen (C₁-C₆)-Alkylrest darstellen;
Z₃ Z₃ₐ, Z_{3b}, Z_{3c}, Z_{3d} oder Z₃ₑ darstellt;
Z₃ₐ einen Rest (C₁-C₆)-Alkyl oder (C₂-C₆)-Alkenyl darstellt;
Z_{3b} einen Rest (C₁-C₆)-Alkoxy, (C₁-C₆)-Alkylthio, (C₁-C₆)-Alkylamino oder Di((C₁-C₆)-alkyl)amino darstellt;
Z_{3c} einen Aryl- oder Heteroarylrest darstellt;
wobei die Reste Aryl und Heteroaryl gegebenenfalls mit einem oder mehreren gleichen oder verschiedenen Substituenten substituiert sind, ausgewählt aus: Halogen, Cyano, Nitro, Azido, Oxy oder-(CH₂)_{p'}-V₃-Y₃;
V₃ -O-, -S-, -C(O)-, -C(O)-O-, -O(CO)-, -SO₂-, -SO₂NH-, -NR'₃-SO₂-, -NR'₃-, -NR-₃-C(O)-, -C(O)-NR'₃-, -NH-C(O)-NR'₃- oder eine kovalente Bindung darstellt;
Y₃ darstellt: ein Wasserstoffatom oder einen Rest (C₁-C₆)-Alkyl, gegebenenfalls substituiert mit einem oder mehreren gleichen oder verschiedenen Halogenresten; einen Arylrest, gegebenenfalls substituiert mit einem oder mehreren gleichen oder verschiedenen Substituenten, ausgewählt aus: Halogen, Nitro, (C₁-C₆)-Alkyl und (C₁-C₆)-Alkoxy; oder einen Rest Aryl-(C₁-C₆)-Alkyl, gegebenenfalls substituiert mit einem oder mehreren gleichen oder verschiedenen Substituenten, ausgewählt aus: Halogen, Nitro, (C₁-C₆)-Alkyl und (C₁-C₆)-Alkoxy;
Z_{3d} darstellt einen Rest (C₁-C₆)-Alkoxycarbonyl, Aminocarbonyl, (C₁-C₆)-Alkylaminocarbonyl, Di((C₁-C₆)-alkyl)aminocarbonyl;
Z₃ₑ einen Rest (C₁-C₆)-Alkyl-C(O)-NH-, (C₃-C₇)-Cycloalkyl, Heterocycloalkyl oder einen Rest der Formel darstellt: 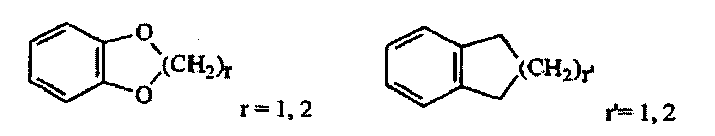 wobei die Reste (C₃-C₇)-Cycloalkyl und Heterocycloalkyl gegebenenfalls mit einem oder mehreren gleichen oder verschiedenen Resten Oxy oder (C₁-C₆)-Alkyl substituiert sind;
Z'₃ einen Arylrest darstellt, gegebenenfalls substituiert mit einem oder mehreren gleichen oder verschiedenen Substituenten, ausgewählt aus Halogen, Nitro und -(CH₂)_{p"}-V'₃-Y'₃;
V'₃ -O-, -C(O)-, -C(O)-O-, -C(O)-NR'₃-, -NR'₃-C(O)-, -NH-C(O)-NR'₃- oder eine kovalente Bindung darstellt;
Y'₃ ein Wasserstoffatom oder einen (C₁-C₆)-Alkylrest darstellt, gegebenenfalls substituiert mit einem oder mehreren gleichen oder verschiedenen Halogenresten;
R'₃ ein Wasserstoffatom, einen Rest (C₁-C₆)-Alkyl oder (C₁-C₆)-Alkoxy darstellt;
p, p' und p" unabhängig voneinander eine ganze Zahl von 0 bis 6 darstellen;
R₄ einen Rest der Formel -(CH₂)ₛ-R'₄ darstellt;
R'₄ darstellt: ein Heterocycloalkyl, das mindestens ein Stickstoffatom enthält und gegebenenfalls mit (C₁-C₆)-Alkyl oder Aralkyl substituiert ist; ein Heteroaryl, das mindestens ein Stickstoffatom enthält und gegebenenfalls mit (C₁-C₆)-Alkyl substituiert ist; oder einen Rest der Formel -NW₄W'₄
W₄ ein Wasserstoffatom oder (C₁-C₈)-Alkyl darstellt;
W'₄ einen Rest der Formel -(CH)₂)_{s'}-Z₄ darstellt;
Z₄ darstellt: ein Wasserstoffatom, (C₁-C₈)-Alkyl; (C₂-C₆)-Alkenyl; (C₃-C₇)-Cycloalkyl, gegebenenfalls substituiert mit einem oder mehreren gleichen oder verschiedenen (C₁-C₆)-Alkylsubstituenten; Cyclohexen, Heteroaryl; Aryl, gegebenenfalls substituiert mit einem oder mehreren gleichen oder verschiedenen Resten, ausgewählt aus -(CH₂)_{s"}-V₄-Y₄, Halogen und Nitro;
V₄ -O-, -S-, -NH-C(O)-, -NV₄'- oder eine kovalente Bindung darstellt;
Y₄ ein Wasserstoffatom oder einen (C₁-C₆)-Alkylrest darstellt, gegebenenfalls substituiert mit einem oder mehreren gleichen oder verschiedenen Halogenresten;
V₄' ein Wasserstoffatom oder ein (C₁-C₆)-Alkyl darstellt;
s" eine ganze Zahl von 0 bis 4 darstellt;
oder Z₄ einen Rest der Formel darstellt: 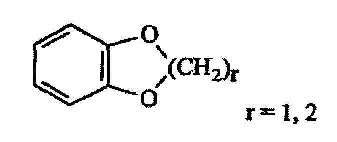 s und s' unabhängig voneinander eine ganze Zahl von 0 bis 6 darstellen;
oder ein pharmazeutisch annehmbares Salz dieser Verbindung.

2. Verbindung nach Anspruch 1, **dadurch gekennzeichnet, dass**
R₁ und R₂ unabhängig voneinander ein Wasserstoffatom, einen Rest (C₁-C₈)-Alkyl, ein Bicycloalkyl oder einen Rest der Formel -(CH₂)ₙ-X₁ oder -X-(CH₂)_{n'}-X'₁ darstellen;
X -C(O)- oder -C(S)-NH- darstellt;
X₁ darstellt: einen Rest (C₁-C₆)-Alkoxy, (C₃-C₇)-Cycloalkyl, gegebenenfalls substituiert mit einem (C₁-C₆)-Alkyl, oder Heteroaryl;
X'₁ darstellt: ein Wasserstoffatom, einen Rest (C₁-C₆)-Alkyl, gegebenenfalls substituiert mit einem oder mehreren gleichen oder verschiedenen Halogenresten, (C₃-C₇)-Cycloalkyl oder Aryl, gegebenenfalls substituiert mit einem (C₁-C₆)-Alkylcarbonyl;
oder R₁ und R₂ zusammen mit dem Stickstoffatom, an das sie gebunden sind, ein Heterobicycloalkyl oder ein Heterocycloalkyl bilden, gegebenenfalls substituiert mit einem oder mehreren gleichen oder verschiedenen Substituenten, ausgewählt aus: (C₁-C₆)-Alkyl, (C₁-C₆)-Alkoxycarbonyl und -(CH₂)_{n"}-A;
A einen Arylrest darstellt, gegebenenfalls substituiert mit einem oder mehreren gleichen oder verschiedenen Substituenten, ausgewählt aus Halogen und (C₁-C₆)-Alkyl;
n" eine ganze Zahl von 0 bis 1 darstellt;
R₄ einen Rest der Formel -(CH₂)ₛ-R'₄ darstellt;
R'₄ darstellt: ein Heterocycloalkyl, das mindestens ein Stickstoffatom enthält und gegebenenfalls substituiert ist mit (C₁-C₆)-Alkyl; oder einen Rest der Formel -NW₄W'₄;
W₄ ein Wasserstoffatom oder (C₁-C₈)-Alkyl darstellt;
W'₄ einen Rest der Formel -(CH₂)_{s'}-Z₄ darstellt;
Z₄ ein Wasserstoffatom, (C₁-C₈)-Alkyl oder Aryl darstellt, gegebenenfalls substituiert mit einem oder mehreren gleichen oder verschiedenen Resten, ausgewählt aus: -(CH₂)_{s"}-V₄-Y₄;
V₄ -O- darstellt;
Y₄ einen Rest (C₁-C₆)-Alkyl darstellt, gegebenenfalls substituiert mit einem oder mehreren gleichen oder verschiedenen Halogenresten;
s" eine ganze Zahl von 0 bis 4 darstellt;
s und s' unabhängig voneinander eine ganze Zahl von 1 bis 4 darstellen;
oder ein pharmazeutisch annehmbares Salz dieser Verbindung.

3. Verbindung nach Anspruch 2, **dadurch gekennzeichnet, dass** sie mindestens eines der folgenden Merkmale aufweist:
- der Cycloalkylrest ist ausgewählt aus Cyclopropyl, Cyclobutyl und Cyclohexyl;
- der Bicycloalkylrest ist Bicyclo[2,2,1]heptan;
- das Heterobicycloalkyl ist 7-Aza-bicyclo[2,2,1]heptan;
- der Arylrest ist der Phenylrest;
- der Heteroarylrest ist der Furylrest;
- das Heterocycloalkyl ist ausgewählt aus Piperidin, Morpholin und Piperazin;
oder ein pharmazeutisch annehmbares Salz dieser Verbindung.

4. Verbindung nach einem der Ansprüche 1 bis 2, **dadurch gekennzeichnet, dass**
R₁ und R₂ unabhängig voneinander ein Wasserstoffatom, einen Rest (C₁-C₈)-Alkyl oder einen Rest der Formel -(CH₂)ₙ-X₁ oder -X-(CH₂)ₙ-X'₁ darstellen;
X -C(O)- darstellt;
X₁ einen Rest (C₃-C₇)-Cycloalkyl darstellt;
X'₁ ein Wasserstoffatom oder einen Rest (C₃-C₇)-Cycloalkyl darstellt;
n 0 oder 1 darstellt; n' eine ganze Zahl von 0 bis 5 darstellt;
oder R₁ und R₂ zusammen mit dem Stickstoffatom, an das sie gebunden sind, ein Heterocycloalkyl bilden, gegebenenfalls substituiert mit einem oder mehreren gleichen oder verschiedenen (C₁-C₆)-Alkylsubstituenten; oder ein pharmazeutisch annehmbares Salz dieser Verbindung.

5. Verbindung nach Anspruch 4, **dadurch gekennzeichnet, dass** der Rest (C₃-C₇)-Cycloalkyl, den X₁ und X'₁ darstellen, ausgewählt ist aus Cyclopropyl, Cyclobutyl und Cyclohexyl; und dass das Heterocycloalkyl, das R₁ und R₂ zusammen bilden, der Piperidinring ist; oder ein pharmazeutisch annehmbares Salz dieser Verbindung.

6. Verbindung nach einem der Ansprüche 1 bis 2 und 4 bis 5, **dadurch gekennzeichnet, dass** R₄ einen Rest der Formel -(CH₂)ₛ-R'₄ darstellt;
R'₄ darstellt: ein Heterocycloalkyl, das mindestens ein Stickstoffatom enthält und gegebenenfalls mit (C₁-C₆)-Alkyl substituiert ist; oder einen Rest der Formel -NW₄W'₄;
W₄ ein Wasserstoffatom oder (C₁-C₈)-Alkyl darstellt;
W'₄ einen Rest der Formel -(CH₂)_{s'}-Z₄ darstellt;
Z₄ ein Wasserstoffatom oder (C₁-C₈)-Alkyl darstellt;
s und s' unabhängig voneinander eine ganze Zahl von 2 bis 4 darstellen;
oder ein pharmazeutisch annehmbares Salz dieser Verbindung.

7. Verbindung nach Anspruch 6, **dadurch gekennzeichnet, dass** das Heterocycloalkyl, das R'₄ darstellt, ausgewählt ist aus: Piperidin und Morpholin; oder ein pharmazeutisch annehmbares Salz dieser Verbindung.

8. Verbindung nach einem der vorhergehenden Ansprüche, **dadurch gekennzeichnet, dass** R₃ -C(O)-Z'₃ darstellt;
Z'₃ einen Arlyrest darstellt, gegebenenfalls substituiert mit einem oder mehreren gleichen oder verschiedenen Substituenten, ausgewählt aus Halogen und -(CH₂)_{p"}-V'₃-Y'₃;
V'₃ -O- oder eine kovalente Bindung darstellt;
Y'₃ ein Wasserstoffatom oder einen Rest (C₁-C₆)-Alkyl darstellt, gegebenenfalls substituiert mit einem oder mehreren gleichen oder verschiedenen Halogenresten;
p" eine ganze Zahl von 0 bis 2 darstellt;
oder ein pharmazeutisch annehmbares Salz dieser Verbindung.

9. Verbindung nach einem der vorhergehenden Ansprüche, **dadurch gekennzeichnet, dass** R₃ darstellt: -Z₃, -C(R_{Z3})(R'_{Z3})-Z₃ oder -C(R_{Z3})(R'_{Z3})-(CH₂)ₚ-Z₃; oder ein pharmazeutisch annehmbares Salz dieser Verbindung.

10. Verbindung nach Anspruch 9, **dadurch gekennzeichnet, dass** R₃ -Z₃ darstellt und Z₃ Z_{3b}, Z_{3c} oder Z₃ₑ darstellt; oder ein pharmazeutisch annehmbares Salz dieser Verbindung.

11. Verbindung nach Anspruch 10, **dadurch gekennzeichnet, dass** Z₃ Z_{3c} darstellt und Z_{3c} einen Arylrest darstellt; oder ein pharmazeutisch annehmbares Salz dieser Verbindung.

12. Verbindung nach Anspruch 11, **dadurch gekennzeichnet, dass** Z_{3c} einen Phenylrest darstellt, substituiert mit einem oder mehreren gleichen oder verschiedenen Substituenten, ausgewählt aus Halogen, Nitro oder -(CH₂)_{p'}-V₃-Y₃;
V₃ -O-, -S-, -C(O)-, -C(O)-O-, -SO₂NH-, -NR'₃-C(O)-, -C(O)-NR'₃- oder eine kovalente Bindung darstellt;
R'₃ ein Wasserstoffatom darstellt;
Y₃ ein Wasserstoffatom oder einen Rest (C₁-C₆)-Alkyl darstellt, gegebenenfalls substituiert mit einem oder mehreren gleichen oder verschiedenen Halogenresten; oder ein pharmazeutisch annehmbares Salz dieser Verbindung.

13. Verbindung nach Anspruch 11, **dadurch gekennzeichnet, dass** Z_{3c} einen Phenylrest darstellt, substituiert mit einem oder mehreren gleichen oder verschiedenen Substituenten der Formel -(CH₂)_{p'}-V₃-Y₃;
V₃ -C(O)-, -C(O)-O- oder -C(O)-NR'₃- darstellt;
R'₃ ein Wasserstoffatom darstellt;
Y₃ ein Wasserstoffatom oder einen Rest (C₁-C₆)-Alkyl darstellt; oder ein pharmazeutisch annehmbares Salz dieser Verbindung.

14. Verbindung nach Anspruch 9, **dadurch gekennzeichnet, dass** R₃ -C(R_{Z3})(R'_{Z3})-Z₃ darstellt und Z₃ Z_{3d} oder Z₃ₑ darstellt; oder ein pharmazeutisch annehmbares Salz dieser Verbindung.

15. Verbindung nach Anspruch 9, **dadurch gekennzeichnet, dass** R₃ -C(R_{Z3})(R'_{Z3})-(CH₂)ₚ-Z₃ darstellt und Z₃ Z_{3c}, Z_{3d} oder Z₃ₑ darstellt; oder ein pharmazeutisch annehmbares Salz dieser Verbindung.

16. Verbindung nach Anspruch 15, **dadurch gekennzeichnet, dass** Z₃ Z_{3d} oder Z₃ₑ darstellt;
Z_{3d} einen Rest (C₁-C₆)-Alkoxycarbonyl oder Aminocarbonyl darstellt;
Z₃ₑ darstellt: einen Rest (C₁-C₆)-Alkyl-C(O)-NH-, wobei Heterocycloalkyl gegebenenfalls mit einem Oxyrest substituiert ist, oder einen Rest der Formel: 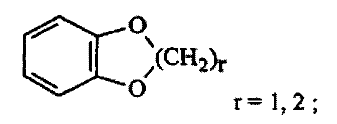 oder ein pharmazeutisch annehmbares Salz dieser Verbindung.

17. Verfahren zur Herstellung einer Verbindung der Formel (I) nach einem der vorhergehenden Ansprüche, **dadurch gekennzeichnet, dass** man die Verbindung der allgemeinen Formel: 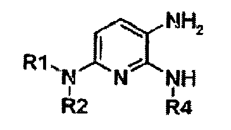 in der R₁, R₂, R₄ die in Anspruch 1 angegebene Bedeutung haben, mit einem Isothiocyanat der allgemeinen Formel R₃N=C=S, in der R₃ die in Anspruch 1 angegebene Bedeutung hat, in Gegenwart eines Kopplungsmittels oder von gelbem Quecksilber(II)-Oxid in Gegenwart von Schwefel während einer Dauer von 3 bis 48 Stunden in einem protischen oder aprotischen Lösungsmittel bei einer Temperatur von 50 bis 80°C behandelt.

18. Pharmazeutische Zusammensetzung, die als Wirkstoff mindestens eine Verbindung nach einem der Ansprüche 1 bis 16 in Kombination mit einem pharmazeutisch annehmbaren Träger enthält.

19. Verwendung einer Verbindung nach einem der Ansprüche 1 bis 16 für die Herstellung eines Medikaments für die Behandlung von Gewichtsstörungen, geistigen Störungen, Schmerz, Störungen der sexuellen Aktivität.

20. Verwendung einer Verbindung nach Anspruch 19 für die Herstellung eines Medikaments für die Behandlung von Gewichtsstörungen, wie Adipositas, Kachexie und insbesondere Kachexie von Krebserkrankungen, Aids-Kachexie, Kachexie älterer Menschen, Herzkachexie, Nierenkachexie, Kachexie der rheumatoiden Arthritis und Anorexie.

21. Verwendung einer Verbindung nach Anspruch 19 für die Herstellung eins Medikaments für die Behandlung von geistigen Störungen wie Angst und Depression.

22. Verwendung einer Verbindung nach Anspruch 19 für die Herstellung eines Medikaments für die Behandlung von Schmerz und insbesondere neuropathischem Schmerz.
